# EUROPEAN PATENT APPLICATION

(11) **EP 2 518 071 A1**
(43) Date of publication of application: **31.10.2012**
(21) Application number: 11382125.0
(22) Date of filing: 29.04.2011
(51) Int. Cl.: C07D 473/34, C07D 487/04, A61K 31/4985, A61K 31/437, A61P 35/00

(54) **Imidazopyridine derivatives as PI3K inhibitors**

(71) Applicant: Almirall, S.A., 08022 Barcelona (ES)
(72) Inventor: Carrascal Riera, Marta, 08980, Sant Feliu de Llobregat (ES); Gracia Ferrer, Jordi, 08980, Sant Feliu de Llobregat (ES); Matassa, Victor Giulio, 08980, Sant Feliu de Llobregat (ES); Terricabras Belart, Emma, 08980, Sant Feliu de Llobregat (ES)
(74) Representative: Elzaburu Marquez, Alberto

(57) **Abstract**

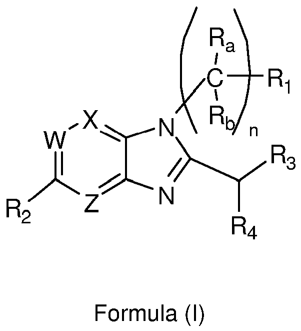

New imidazopyridine derivatives having the chemical structure of formula (I) are disclosed; as well as process for their preparation, pharmaceutical compositions comprising them and their use in therapy as inhibitors of Phosphoinositide 3-Kinases (PI3Ks)

## Description

When cells are activated by extracellular stimuli, intracellular signalling cascades involving the regulation of second messengers are initiated that eventually produce a response of the cell to the stimuli. Phosphoinositide 3-Kinases (PI3Ks) are among the enzymes involved in early signalling events to a plethora of different types of stimuli. PI3Ks phosphorylate the 3-hydroxyl group of the inositol ring of phosphatidylinositol (Ptdlns), Ptdlns-4-phosphate (Ptdlns4P), and Ptdlns-4,5-bisphosphate (Ptdlns(4,5)P2). The resulting 3-phosphoinositides mediate correct localization and subsequent activation of a number of downstream effector proteins that bind to the lipids via specific lipid binding sequences such as the pleckstrin homology (PH) domain *(*Vanhaesebroeck B, 2010, Nat Rev Mol Cell Biol 5:11381-6*).*

The PI3K family is divided into 3 different classes (Pl3K class I, class II, and class III), depending on substrate preference and structural features.

The best characterized is the PI3K class I with the preferential substrate Ptdlns-(4,5)P2. It englobes 4 different isoforms which originally were further subdivided into class IA (p110a, p110b, p110d), binding to a p85 type of regulatory subunit, and class IB (p110g) which is regulated by p101 and p87 subunits. Whereas p110a (PI3Ka or PI3Ka) and p110b (PI3Kb or PI3Kβ) isoforms are expressed ubiquitously, p110g (PI3Kg or PI3Kγ) and especially p110d (PI3Kd or PI3Kδ) have a more restricted expression pattern and seem to play a major role in leukocytes *(*Kok K, Trends Biochem Science 34:115-127, 2009*)*.

Both, PI3Kd and PI3Kg are involved in activation of immune cells by a large variety of different stimuli. Pharmacological inhibition or genetic deficiency in active p110d has been shown to inhibit T cell proliferation and cytokine production in response to different stimuli such as anti-CD3, anti-CD3/CD28, superantigen or antigen in vitro (Ji H, Blood 2007; Okkenhaug K, Science 2002; Garcon F, 2009; Soond DR, Blood 2010; Herman SEM, Blood June 3, 2010; William O, Chemistry & Biology 17, 2010) and to suppress concanavalin A and anti-CD3 induced cytokine production as well as antigen-dependent tissue retention in vivo (Soond DR, Blood 2010; Jarmin SJ, JCI 2008). In addition, B cell function is critically dependent on functional PI3Kd activity as demonstrated by suppressed B cell proliferation and cytokine release in vitro in response to anti-IgM (Bilancio A, Blood 107, 2006), toll like receptor agonists such as LPS and oligodeoxynucleotides (Dil N, Mol Immunol 46, 2009) or impaired ability to stimulate antigen-specific T cells (Al-Alwan M, JI 2007) in the absence of functional p110d or pharmacological inhibition. In vivo, PI3Kg deficient mice display partially suppressed antibody production upon immunization (Garcon F, 2009; Durand CA, JI 2009). Further studies have demonstrated an important role of PI3Kd in inhibition of T cell apoptosis and in TH17 differentiation (Haylock-Jacobs S, J. Autoimmun 2010).

In addition, mast cell degranulation was reduced in cells from mice with inactivated PI3Kd or by pharmacological inhibition of PI3Kd (Ali K, Nature 431:1007-1011, 2004; Ali K, Journal of Immunology 180:2538-2544, 2008) and basophil activation via the FcE receptor is suppressed by pharmacological inhibition of PI3Kd (Lannutti BJ, Blood Oct. 2010).

In terms of neutrophil function, PI3Kd inhibition inhibits migration of mouse neutrophils to fMLP in an under-agarose migration assay by inhibiting cell polarization and directional movement (Sadhu C, JI 170, 2003) and mouse PI3Kd deficient or inhibitor treated neutrophils show slightly (25%) reduced in vitro chemotaxis to LTB4, whereas in vivo accumulation in the lung in response to LPS was reduced by more than 80%, indicating an important role of PI3Kd in endothelial cells for mediating PMN transendothelial migration (Puri KD, Blood 103, 2004). Furthermore, TNF induced neutrophil infiltration to an air pouch in mice and elastase release is partially inhibited by a PI3Kd selective inhibitor (Sadhu C, Biochem Biophys Res Comm 308, 2003). In addition, TNF mediated priming of oxidative burst by human neutrophils depends on PI3Kd activity (Condliffe AM, Blood 106, 2005).

In contrast to the dominant role of PI3Kd in lymphocyte activation, PI3Kg seems to affect primarily chemotaxis of different immune cells induced by various mediators and chemokines (Martin AL, JI 180, 2008; Thomas MS, J Leukoc Biol 84, 2008; Jarmin SJ, JCI 2008; Matthew T, Immunology 126, 2008), as well as degranulation and oxidative burst of innate immuce cells induced by GPCR mediated stimuli such as fMLP, IL-8 or C5a (Condliffe AM, Blood 106, 2005; Yum HK, JI 167, 2001; Pinho V, JI 179, 2007

The above mentioned findings suggest that selective PI3Kd or dual PI3Kd/PI3Kg pharmacological inhibition represents a promising approach for treating a variety of diseases such as respiratory diseases (asthma, chronic obstructive pulmonary disease (COPD), cystic fibrosis, idiopathic pulmonary fibrosis, sarcoidosis), allergic diseases (allergic rhinitis), inflammatory or autoimmune diseases (rheumatoid arthritis, multiple sclerosis, amyotrophic lateral sclerosis, Crohn's disease, ulcerative colitis, systemic lupus erythematosis, myastenia gravias, acute disseminated encephalomyelitis, idiopathic thromocytopenic purpura, Sjoegren's syndrome, autoimmune hemolytic anemia, type I diabetes, psoriasis, acrodermatitis, angiodermatitis, atopic dermatitis, contact dermatitis, eczema, acne, chronic urticaria, blistering diseases including but not limited to bullous pemphigoid, scleroderma, dermatomyositis, etc.), cardiovascular diseases; viral infection; metabolism/endocrine function disorders; neurological disorders and pain (such as pain associated with rheumatoid arthritis or osteoarthritis, back pain, general inflammatory pain, inflammatory neuropathic pain, trigeminal neuralgia or central pain) as well as in bone marrow and organ transplant rejection; myelo-dysplastic syndrome; myeloproliferative disorders (MPDs); cancer and hematologic malignancies, leukemia, lymphomas and solid tumors (such as pancreatic cancer; bladder cancer; colorectal cancer; breast cancer; prostate cancer; renal cancer; hepatocellular cancer; lung cancer; ovarian cancer; cervical cancer; gastric cancer; esophageal cancer; head and neck cancer; non-small cell lung cancer and small-cell lung cancer; melanoma; neuroendocrine cancers; central nervious system cancers; brain tumors; bone cancer; soft tissue sarcoma; chronic lymphocytic leukemia, B-cell acute lymphoblastic leukemia, T-cell acute lymphoblastic leukaemia, non-hodgkins lymphoma, B-cell lymphoma, acute myeloid leukaemia; cutaneous T cell lymphoma, premalignant and malignant skin conditions including but not limited to basal cell carcinoma (BCC), squamous cell carcinoma (SCC) or actinic keratosis (AK)).

There is substantial experimental evidence supporting this view. In rodent models of allergic lung inflammation, genetic or pharmacolocical inactivation of PI3Kd or dual PI3Kd/g dual inhibition reduces cell influx, mucus production, cytokine production and airway hyperreactivity (Nashed et a. 2007, Eur J Immunol 37:416; Lee et al. 2006, FASEB J 20:455 & Lee KS et al. 2006, J Allergy Clin Immunol 118:403; Doukas J, JPET 2009;328:758; Par SJ, ERJ 2010). Moreover, LPS induced lung neutrophil infiltration is blocked by PI3Kd inhibition (Puri KD, Blood 2004;103:3448) and inflammation in response to LPS or tobacco smoke exposure is suppressed by a dual PI3Kd/g inhibitor (Doukas J, JPET 2009;328:758). Moreover, PI3Kd seems to be involved in the reduction of responsiveness to corticosteroid treatment associated with oxidative stress and chronic obstructive pulmonary disease (COPD). This notion is based on the findings that tobacco smoke induced inflammation remains responsive to treatment with budesonide, whereas wild type or PI3Kg deficient mice develop resistance to corticosteroid treatment (Marwick JA, JRCCM 179:542-548, 2009). Similar results were obtained with a PI3Kd selective inhibitor (To Y, AJRCCM 182:897-904, 2010). In addition, in vitro induction of corticosteroid resistance by oxidative stress is prevented by PI3Kd inhibition (To Y, AJRCCM 2010). In COPD patients, lung macrophages display increased expression of PI3Kd and phosphorylation of its downstream effector Akt and non-selective PI3K or PI3Kd- selective inhibition restored the impaired inhibitory efficacy of dexamethasone in PBMC from COPD patients (To Y, AJRCCM 182:897-904, 2010; Marwick JA, JACI 125:1146-53, 2010).

Furthermore, PI3Kd inhibition was effective in a model of contact hypersensitivity (Soond DR, Blood Jan 2010). In a model of experimental autoimmune encephalomyelitis, PI3Kd deficiency or pharmacological inhibition of PI3Kd attenuated T cell activation and function and reduced T cell numbers in the CNS, suggesting a therapeutic benefit of PI3Kd inhibitor in multiple sclerosis and other Th17-mediated autoimmune diseases (Haylock-Jacobs S, J. Autoimmun 2010). In line with that, genetic deficiency or pharmacological inhibition of PI3Kd diminished joint erosion in a mouse model of inflammatory arthritis (Randis TM, Eur J Immunol 38, 2008). Concerning metabolic diseases, PI3Kd overexpression seems to contribute to excessive vascular contraction and PI3Kd inhibition normalized vascular contractive responses in a mouse model of type I diabetes, suggesting a therapeutic potential of PI3Kd blockade to treat vascular dysfunction in diabetic patients (Pinho JF, Br. J. Pharmacol 161, 2010).

There is also substantial experimental evidence supporting that genetic of pharmacolocical inactivation of PI3Kd or dual PI3Kd/g dual inhibition is effective in the treatment of cancers including but not restricted to leukemias, such as chronic lymphocytic leukemia, B-cell acute lymphoblastic leukemia, T-cell acute lymphoblastic leukaemia, non-hodgkins lymphoma, B-cell lymphoma, acute myeloid leukaemia, myelo-dysplastic syndrome or myelo-proliferative diseases. In this aspect, the selective PI3Kd inhibitor CAL-1 01 demonstrated anti-proliferative properties on different tumor cells in vitro and efficacy in cancer patients with a dysregulated PI3Kd activity, such as chronic lymphocytic leukemia (Hermann SE, Blood 116:2078-88, 2010; Lannutti BJ, Blood Oct. 2010).

Conditions in which targeting of the PI3K pathway or modulation of the PI3 Kinases, particularly PI3Kd or PI3Kd/g, are contemplated to be therapeutically useful for the treatment or prevention of diseases including: respiratory diseases (asthma, chronic obstructive pulmonary disease (COPD), cystic fibrosis, idiopathic pulmonary fibrosis, sarcoidosis), allergic diseases (allergic rhinitis), inflammatory or autoimmune-mediated diseases (rheumatoid arthritis, multiple sclerosis, amyotrophic lateral sclerosis, Crohn's disease, ulcerative colitis, systemic lupus erythematosis, myastenia gravias, acute disseminated encephalomyelitis, idiopathic thromocytopenic purpura, Sjoegren's syndrome, autoimmune hemolytic anemia, type I diabetes, psoriasis, acrodermatitis, angiodermatitis, atopic dermatitis, contact dermatitis, eczema, acne, chronic urticaria, scleroderma, dermatomyositis and blistering diseases including but not limited to bullous pemphigoid), cardiovascular diseases; viral infection; metabolism/endocrine function disorders; neurological disorders and pain (such as pain associated with rheumatoid arthritis or osteoarthritis, back pain, general inflammatory pain, inflammatory neuropathic pain, trigeminal neuralgia or central pain) as well as in bone marrow and organ transplant rejection; myelo-dysplastic syndrome; myeloproliferative disorders (MPDs); cancer and hematologic malignancies, leukemia, lymphomas and solid tumors (such as pancreatic cancer; bladder cancer; colorectal cancer; breast cancer; prostate cancer; renal cancer; hepatocellular cancer; lung cancer; ovarian cancer; cervical cancer; gastric cancer; esophageal cancer; head and neck cancer; non-small cell lung cancer and small-cell lung cancer; melanoma; neuroendocrine cancers; central nervious system cancers; brain tumors; bone cancer; soft tissue sarcoma; chronic lymphocytic leukemia, B-cell acute lymphoblastic leukemia, T-cell acute lymphoblastic leukaemia, non-hodgkins lymphoma, B-cell lymphoma, acute myeloid leukaemia; cutaneous T cell lymphoma, premalignant and malignant skin conditions including but not limited to basal cell carcinoma (BCC), squamous cell carcinoma (SCC) or actinic keratosis (AK)).

In view of the numerous conditions that are contemplated to benefit by treatment involving modulation of the PI3K pathway or modulation of the PI3 Kinases it is immediately apparent that new compounds that modulate PI3K pathways and use of these compounds should provide substantial therapeutic benefits to a wide variety of patients.

Provided herein are novel imidazopyridine derivatives for use in the treatment of conditions in which targeting of the PI3K pathway or inhibition of PI3 Kinases can be therapeutically useful.

The compounds described in the present invention are potent PI3K inhibitors, particularly PI3Kd or dual PK3Kd/g inhibitors. This property makes them useful for the treatment or prevention of pathological conditions or diseases such as respiratory diseases (asthma, chronic obstructive pulmonary disease (COPD), cystic fibrosis, idiopathic pulmonary fibrosis, sarcoidosis), allergic diseases (allergic rhinitis), inflammatory or autoimmune diseases (rheumatoid arthritis, multiple sclerosis, amyotrophic lateral sclerosis, Crohn's disease, ulcerative colitis, systemic lupus erythematosis, myastenia gravias, acute disseminated encephalomyelitis, idiopathic thromocytopenic purpura, Sjoegren's syndrome, autoimmune hemolytic anemia, type I diabetes, psoriasis, acrodermatitis, angiodermatitis, atopic dermatitis, contact dermatitis, eczema, acne, chronic urticaria, scleroderma, dermatomyositis and blistering diseases including but not limited to bullous pemphigoid), cardiovascular diseases; viral infection; metabolism/endocrine function disorders; neurological disorders and pain (such as pain associated with rheumatoid arthritis or osteoarthritis, back pain, general inflammatory pain, inflammatory neuropathic pain, trigeminal neuralgia or central pain) as well as in bone marrow and organ transplant rejection; myelo-dysplastic syndrome; myeloproliferative disorders (such as polycythemia vera, essential thrombocythemia or mielofibrosis); cancer and hematologic malignancies, leukemia, lymphomas and solid tumors (such as pancreatic cancer; bladder cancer; colorectal cancer; breast cancer; prostate cancer; renal cancer; hepatocellular cancer; lung cancer; ovarian cancer; cervical cancer; gastric cancer; esophageal cancer; head and neck cancer; non-small cell lung cancer and small-cell lung cancer; melanoma; neuroendocrine cancers; central nervious system cancers; brain tumors; bone cancer; soft tissue sarcoma; chronic lymphocytic leukemia, B-cell acute lymphoblastic leukemia, T-cell acute lymphoblastic leukaemia, non-hodgkins lymphoma, B-cell lymphoma, acute myeloid leukaemia; cutaneous T cell lymphoma, premalignant and malignant skin conditions including but not limited to basal cell carcinoma (BCC), squamous cell carcinoma (SCC) or actinic keratosis (AK)).

The compounds described in the present invention are particularly useful for the treatment or prevention of pathological conditions or diseases such as neoplastic diseases (e.g. leukemia, lymphomas, solid tumors); transplant rejection, bone marrow transplant applications (e.g., graft- versus-host disease); autoimmune diseases (e.g. rheumatoid arthritis, multiple sclerosis, amyotrophic lateral sclerosis, Crohn's disease, ulcerative colitis, systemic lupus erythematosis, autoimmune hemolytic anemia, type I diabetes); respiratory inflammation diseases (e.g. asthma, chronic obstructive pulmonary disease, cystic fibrosis, idiopathic pulmonary fibrosis, sarcoidosis); skin inflammatory diseases (e.g., atopic dermatitis, contact dermatitis, eczema or psoriasis); premalignant and malignant skin conditions (e.g. basal cell carcinoma (BCC), squamous cell carcinoma (SCC) or actinic keratosis (AK)); neurological disorders and pain (such as pain associated with rheumatoid arthritis or osteoarthritis, back pain, general inflammatory pain, inflammatory neuropathic pain, trigeminal neuralgia or central pain)

The compounds described in the present invention are particularly useful for the treatment or prevention of pathological conditions or diseases selected from leukemia, lymphomas and solid tumors, rheumatoid arthritis, multiple sclerosis, amyotrophic lateral sclerosis, Crohn's disease, ulcerative colitis, systemic lupus erythematosis, autoimmune hemolytic anemia, type I diabetes, asthma, chronic obstructive pulmonary disease, cystic fibrosis, idiopathic pulmonary fibrosis, sarcoidosis, allergic rhinitis, atopic dermatitis, contact dermatitis, eczema, psoriasis, basal cell carcinoma, squamous cell carcinoma and actinic keratosis.

It has now been found that certain imidazopyridine derivatives are novel and potent PI3K inhibitors and can therefore be used in the treatment or prevention of these diseases.

Thus the present invention is directed to compounds of formula (I), or a pharmaceutically acceptable salt, or solvate, or N-oxide, or stereoisomer or deuterated derivative thereof: 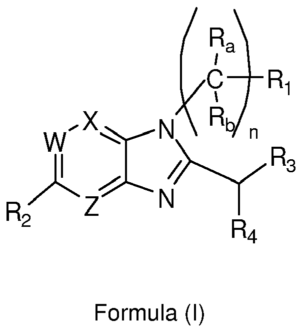 wherein,
X represents a nitrogen atom, W represents a -CR₆ group and Z represents a -CR₇ group; or X represents a -CR₅ group, W represents a nitrogen atom and Z represents a -CR₇ group; or X represents a -CR₅ group, W represents a -CR₆ group and Z represents nitrogen atom;
Rₐ and R_{b} each independently represent a hydrogen atom, a C₁-C₄ haloalkyl group, a C₁-C₄ hydroxyalkyl group or a linear or branched C₁-C₄ alkyl group;
n represents 0, 1, 2 or 3;
R₁ represents a linear or branched C₁-C₄ alkyl group, a C₃-C₁₀ cycloalkyl group, a C₃-C₁₀ cycloalkenyl group, a monocyclic or bicyclic C₆-C₁₄ aryl group, a 5- to 14-membered monocyclic or bicyclic heteroaryl group containing at least one heteroatom selected from O, S and N, a 5- to 14- membered monocyclic or bicyclic heterocyclyl group containing at least one heteroatom selected from O, S and N, or a 2-pyridinone group,
wherein the cycloalkyl, cycloalkenyl, aryl, heteroaryl, and heterocyclyl groups are unsubstituted or substituted by one or more substituents selected from a halogen atom, a hydroxy group, a cyano group, a linear or branched C₁-C₄ alkyl group, a C₁-C₄ haloalkyl group, a C₁-C₄ hydroxyalkyl group, a C₃-C₄ cycloalkyl group, a -(CH₂)₁₋₃CN group, a -(CH₂)₀₋₃OR₈ group, a -(CH₂)₀₋₃NR₈R₉ group, a -C(O)-(CH₂)₁₋₃-CN group, a -C(O)-(CH₂)₀₋₃-R₈ group, a -C(O)-(CH₂)₀₋₃-NR₈R₉ group, a -S(O)₂(CH₂)₀₋₃R₈ group or a -S(O)₂(CH₂)₀₋₃NR₈R₉ group;
R₂ represents a hydrogen atom, a halogen atom, a hydroxy group, a cyano group, a C₁-C₄ alkoxy group, a C₁-C₄ haloalkyl group, a C₁-C₄ hydroxyalkyl group, a C₃-C₇ cycloalkyl group, a -NR'R" group, or a linear or branched C₁-C₆ alkyl group, which alkyl group is unsubstituted or substituted by one or more substituents selected from a C₁-C₄ alkoxy group, a cyano group or a C₃-C₇ cycloalkyl group;
R₃ represents a hydrogen atom, a C₁-C₄ alkoxy group, a C₁-C₄ haloalkyl group, a C₁-C₄ hydroxyalkyl group, a C₃-C₇ cycloalkyl group, a -(CH₂)₁₋₄NR'R" group, or a linear or branched C₁-C₄ alkyl group, which alkyl group is unsubstituted or substituted by one or more substituents selected from a C₁-C₄ alkoxy group, a cyano group, a C₃-C₄ cycloalkyl group, a -C(O)-(CH₂)₀₋₃-R' group or a -C(O)-(CH₂)₀₋₃-NR'R" group;
R₅, R₆ and R₇ each independently represent a hydrogen atom, a halogen atom, a hydroxy group, a cyano group, a C₁-C₄ alkoxy group, a C₁-C₄ haloalkyl group, a C₁-C₄ hydroxyalkyl group, a C₃-C₇ cycloalkyl group, a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group;
R₈ and R₉ each independently represent a hydrogen atom, a C₁-C₄ haloalkyl group, a C₁-C₄ hydroxyalkyl group or a linear or branched C₁-C₄ alkyl group, which alkyl group is unsubstituted or substituted by one or more substituents selected from a C₁-C₄ alkoxy group, a cyano group or a C₃-C₄ cycloalkyl group;
R' and R" each independently represent a hydrogen atom, a hydroxy group, a C₁-C₄ alkoxy group or a linear or branched C₁-C₄ alkyl group.
R₄ represents a group selected from:
i) a group of formula (IIa) or (IIa') 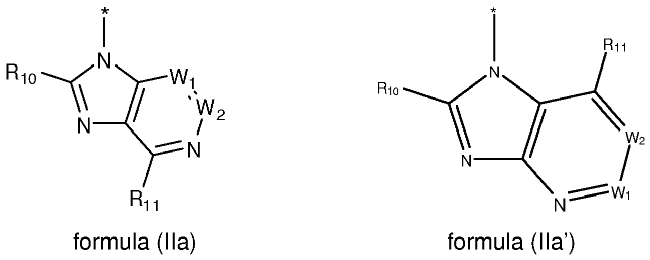
ii) a group of formula (IIb) 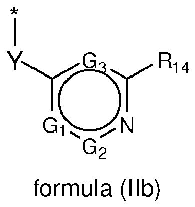 and
iii) a group of formula (IIc) 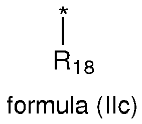 wherein
   Y represents a linker selected from a -NR'- group, -O- or -S-; wherein R' is as defined above;
   (*) represents where R₄ is bonded to the carbon atom attached to R₃ and to the imidazole group;
   W₁ represents a -CR₁₂ group and W₂ represents a nitrogen atom, or W₁ represents a nitrogen atom and W₂ represents a -CR₁₃ group;
   G₁ represents a -CR₁₅ group and G₂ represents a nitrogen atom, or G₁ represents a nitrogen atom and G₂ represents a -CR₁₆ group, or G₁ represents a -CR₁₅ group and G₂ represents a -CR₁₆ group;
   G₃ represents a nitrogen atom or a -CR₁₇ group;
   R₁₀, R₁₁, R₁₂ R₁₃, R₁₄, R₁₅, R₁₆ and R₁₇ each independently represent a hydrogen atom, a C₁-C₄ alkoxy group, a C₁-C₄ haloalkyl group, a C₁-C₄ hydroxyalkyl group, a C₃-C₄ cycloalkyl group, a -(CH₂)₀₋₃CN group,
   a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group; wherein R' and R" are as defined above;
   R₁₈ represents a selected from
   a) a group of formula (IIIa), b) a group of formula (IIIb), 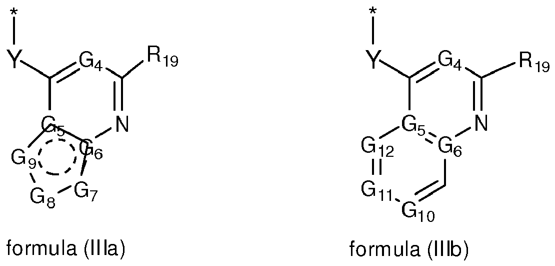
   c) a group of formula (IIIc), and d) a group of formula (IIId), 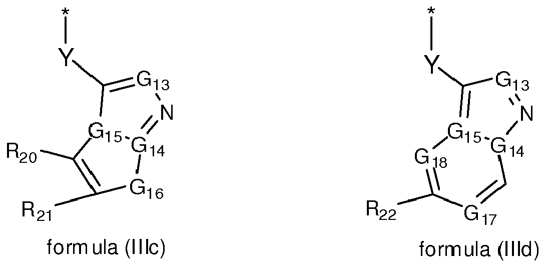 wherein
      G₄ represents a nitrogen atom or a -CR₂₃ group;
      G₅ and G₆ each independently represents a nitrogen atom or a -carbon atom, wherein when one of G₅ and G₆ represents a nitrogen atom the remaining represents a carbon atom;
      G₇ represents a -NH group or a -CH group;
      G₈ represents a nitrogen atom or a -CR₂₄ group;
      G₉ represents a nitrogen atom or a -CR₂₅ group;
      G₁₀ represents a nitrogen atom or a -CR₂₆ group;
      G₁₁ represents a nitrogen atom or a -CR₂₇ group;
      G₁₂ represents a nitrogen atom or a -CR₂₈ group;
      G₁₃ represents a nitrogen atom or a -CR₂₉ group;
      G₁₄ and G₁₅ each independently represents a nitrogen atom or a carbon atom, wherein when one of G₁₄ and G₁₅ represents a nitrogen atom the remaining represents a carbon atom;
      G₁₆ represents a -NH group or a -CH group;
      G₁₇ represents a nitrogen atom or a -CR₃₀ group;
      G₁₈ represents a nitrogen atom or a -CR₃₁ group;
      R₁₉, R₂₀, R₂₁, R₂₂, R₂₃, R₂₄, R₂₅, R₂₆, R₂₇, R₂₈, R₂₉, R₃₀ and R₃, each independently represent a hydrogen atom, a C₁-C₄ alkoxy group, a C₁-C₄ haloalkyl group, a C₁-C₄ hydroxyalkyl group, a C₃-C₄ cycloalkyl group,
      a -(CH₂)₀₋₃CN group, a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group; wherein R' and R" are as defined above; and wherein Y is as defined above;
      or in the case that Y represents a -NR'- group, R₃ together with the -NR'- group and the carbon atom to which both R₃ and the -NR'- group are bonded form a 4- to 7-membered, saturated N-containing heterocyclyl group, which heterocyclyl group is unsubstituted or substituted by one or more substituents selected from a halogen atom, a hydroxyl group, a cyano group, a -CHF₂ group or a -CF₃ group.

The dotted line in the group of formula (IIIa) 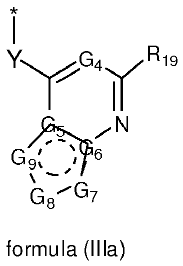 denotes that there are two double bounds in the C₅ heteroaryl ring, whose position may vary depending on which G₅, G₆, G₇, G₈ or G₉ represents a nitrogen atom or a carbon atom.

The invention further provides synthetic processes and intermediates described herein, which are useful for preparing said compounds.

The invention is also directed to a compound of the invention as described herein for use in the treatment of the human or animal body by therapy.

The invention also provides a pharmaceutical composition comprising the compounds of the invention and a pharmaceutically-acceptable diluent or carrier.

The invention is also directed to the compounds of the invention as described herein, for use in the treatment of a pathological condition or disease susceptible to amelioration by inhibiton of Phosphoinositide 3-Kinases (PI3Ks), in particular wherein the pathological condition or disease is selected from respiratory diseases; allergic diseases; inflammatory or autoimmune-mediated diseases; function disorders and neurological disorders; cardiovascular diseases; viral infection; metabolism/endocrine function disorders; neurological disorders and pain; bone marrow and organ transplant rejection; myelo-dysplastic syndrome; myeloproliferative disorders (MPDs); cancer and hematologic malignancies, leukemia, lymphomas and solid tumors; more in particular wherein the pathological condition or disease is selected from leukemia, lymphomas and solid tumors, rheumatoid artritis (RA), multiple sclerosis (MS), amyotrophic lateral sclerosis, Crohn's disease, ulcerative colitis, systemic lupus erythematosis, autoimmune hemolytic anemia, type I diabetes, asthma, chronic obstructive pulmonary disease (COPD), cystic fibrosis (CF), idiopathic pulmonary fibrosis, sarcoidosis, atopic dermatitis, allergic rhinitis, contact dermatitis, eczema, psoriasis, basal cell carcinoma (BCC), squamous cell carcinoma (SCC) and actinic keratosis (AK).

The invention is also directed to use of the compounds of the invention as described herein, in the manufacture of a medicament for treatment of a pathological condition or disease susceptible to amelioration by inhibiton of Phosphoinositide 3-Kinases (PI3Ks), in particular wherein the pathological condition or disease is selected from respiratory diseases; allergic diseases; inflammatory or autoimmune-mediated diseases; function disorders and neurological disorders; cardiovascular diseases; viral infection; metabolism/endocrine function disorders; neurological disorders and pain; bone marrow and organ transplant rejection; myelo-dysplastic syndrome; myeloproliferative disorders (MPDs); cancer and hematologic malignancies, leukemia, lymphomas and solid tumors; more in particular wherein the pathological condition or disease is selected from leukemia, lymphomas and solid tumors, rheumatoid artritis (RA), multiple sclerosis (MS), amyotrophic lateral sclerosis, Crohn's disease, ulcerative colitis, systemic lupus erythematosis, autoimmune hemolytic anemia, type I diabetes, asthma, chronic obstructive pulmonary disease (COPD), cystic fibrosis (CF), idiopathic pulmonary fibrosis, sarcoidosis, allergic rhinitis, atopic dermatitis, contact dermatitis, eczema, psoriasis, basal cell carcinoma (BCC), squamous cell carcinoma (SCC) and actinic keratosis (AK).

The invention also provides a method of treatment of a pathological condition or disease susceptible to amelioration by inhibiton of Phosphoinositide 3-Kinases (PI3Ks), in particular wherein the pathological condition or disease is selected from respiratory diseases; allergic diseases; inflammatory or autoimmune-mediated diseases; function disorders and neurological disorders; cardiovascular diseases; viral infection; metabolism/endocrine function disorders; neurological disorders and pain; bone marrow and organ transplant rejection; myelo-dysplastic syndrome; myeloproliferative disorders (MPDs); cancer and hematologic malignancies, leukemia, lymphomas and solid tumors; more in particular wherein the pathological condition or disease is selected from leukemia, lymphomas and solid tumors, rheumatoid artritis (RA), multiple sclerosis (MS), amyotrophic lateral sclerosis, Crohn's disease, ulcerative colitis, systemic lupus erythematosis, autoimmune hemolytic anemia, type I diabetes, asthma, chronic obstructive pulmonary disease (COPD), cystic fibrosis (CF), idiopathic pulmonary fibrosis, sarcoidosis, allergic rhinitis, atopic dermatitis, contact dermatitis, eczema, psoriasis, basal cell carcinoma (BCC), squamous cell carcinoma (SCC) and actinic keratosis (AK).

The invention also provides a combination product comprising (i) the compounds of the invention as described herein; and (ii) one or more additional active substances which are known to be useful in the treatment of respiratory diseases; allergic diseases; inflammatory or autoimmune-mediated diseases; function disorders and neurological disorders; cardiovascular diseases; viral infection; metabolism/endocrine function disorders; neurological disorders and pain; bone marrow and organ transplant rejection; myelo-dysplastic syndrome; myeloproliferative disorders (MPDs); cancer and hematologic malignancies, leukemia, lymphomas and solid tumors; more in particular wherein the pathological condition or disease is selected from leukemia, lymphomas and solid tumors, rheumatoid artritis (RA), multiple sclerosis (MS), amyotrophic lateral sclerosis, Crohn's disease, ulcerative colitis, systemic lupus erythematosis, autoimmune hemolytic anemia, type I diabetes, asthma, chronic obstructive pulmonary disease (COPD), cystic fibrosis (CF), idiopathic pulmonary fibrosis, sarcoidosis, allergic rhinitis, atopic dermatitis, contact dermatitis, eczema, psoriasis, basal cell carcinoma (BCC), squamous cell carcinoma (SCC) and actinic keratosis (AK).

As used herein the term C₁-C₆ alkyl embraces linear or branched radicals having 1 to 6 carbon atoms, preferably 1 to 4 carbon atoms. Examples include methyl, ethyl, n-propyl, i-propyl, n-butyl, sec-butyl, t-butyl, n-pentyl, 1-methylbutyl, 2-methylbutyl, isopentyl, 1-ethylpropyl, 1,1-dimethylpropyl, 1,2-dimethylpropyl, n-hexyl, 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, 2-methylpentyl, 3-methylpentyl and iso-hexyl radicals.

When it is mentioned that the alkyl radical may be optionally substituted it is meant to include linear or branched alkyl radical as defined above, which may be unsubstituted or substituted in any position by one or more substituents, for example by 1, 2 or 3 substituents. When two or more substituents are present, each substituent may be the same or different.

As used herein, the term C₁-C₄ haloalkyl group is an alkyl group, for example a C₁-C₄ or C₁-C₂ alkyl group, which is bonded to one or more, preferably 1, 2 or 3 halogen atoms. Preferably, said haloakyl group is chosen from -CCl₃, -CHF₂ and -CF₃.

As used herein, the term C₁-C₄ hydroxyalkyl embraces linear or branched alkyl radicals having 1 to 4 carbon atoms, any one of which may be substituted by one or more, preferably 1 or 2, more preferably 1 hydroxyl radicals. Examples of such radicals include hydroxymethyl, hydroxyethyl, hydroxypropyl, and hydroxybutyl.

As used herein, the term C₁-C₄ alkoxy (or alkyloxy) embraces linear or branched oxy-containing radicals each having alkyl portions of 1 to 4 carbon atoms.

As used herein, the term C₃-C₁₀ cycloalkyl embraces saturated monocyclic or polycyclic carbocyclic radicals having from 3 to 10 carbon atoms, preferably from 3 to 7 carbon atoms. An optionally substituted C₃-C₁₀ cycloalkyl radical is typically unsubstituted or substituted by 1, 2 or 3 substituents which may be the same or different. When a C₃-C₁₀ cycloalkyl radical carries 2 or more substituents, the substituents may be the same or different. Typically the substituents on a C₃-C₁₀ cycloalkyl group are themselves unsubstituted. Polycyclic cycloalkyl radicals contains two or more fused cycloalkyl groups, preferably two cycloalkyl groups. Typically, polycyclic cycloalkyl radicals are selected from decahydronaphthyl (decalyl), bicyclo[2.2.2]octyl, adamantly, camphyl or bornyl groups.
Examples of monocyclic cycloalkyl groups include cyclopropyl, cyclobutyl, cyclopentyl, cyclohexyl, cycloheptyl, cyclooctyl, cyclononyl and cyclodecyl.

As used herein, the term C₃-C₁₀ cycloalkenyl embraces partially unsaturated carbocyclic radicals having from 3 to 10 carbon atoms, preferably from 3 to 7 carbon atoms. A C₃-C₁₀ cycloalkenyl radical is typically unsubstituted or substituted by 1, 2 or 3 substituents which may be the same or different. When a C₃-C₁₀ cycloalkenyl radical carries 2 or more substituents, the substituents may be the same or different. Typically, the substituents on a cycloalkenyl group are themselves unsubstituted. Examples include cyclobutenyl, cyclopentenyl, cyclohexenyl, cycloheptenyl, cyclooctenyl, cyclononenyl and cyclodecenyl.

As used herein, the term C₆-C₁₄ aryl radical embraces typically a C₆-C₁₄, more preferably C₆-C₁₀ monocyclic or bicyclic aryl radical such as phenyl, naphthyl, anthranyl and phenanthryl. Phenyl is preferred. A said optionally substituted C₆-C₁₄ aryl radical is typically unsubstituted or substituted by 1, 2 or 3 substituents which may be the same or different. When a C₆-C₁₄ aryl radical carries 2 or more substituents, the substituents may be the same or different. Unless otherwise specified, the substituents on a C₆-C₁₄ aryl group are typically themselves unsubstituted.

As used herein, the term 5- to 14- membered heteroaryl radical embraces typically a 5-to 14- membered ring system, preferably a 5- to 10- membered ring system, more preferably a 5- to 6- membered ring system, comprising at least one heteroaromatic ring and containing at least one heteroatom selected from O, S and N. A 5- to 14-membered heteroaryl radical may be a single ring or two fused rings wherein at least one ring contains a heteroatom.

A said optionally substituted 5- to 14- membered heteroaryl radical is typically unsubstituted or substituted by 1, 2 or 3 substituents which may be the same or different. When a 5- to 14- membered heteroaryl radical carries 2 or more substituents, the substituents may be the same or different. Unless otherwise specified, the substituents on a 5- to 14- membered heteroaryl radical are typically themselves unsubstituted.

Examples include pyridyl, pyrazinyl, pyrimidinyl, pyridazinyl, furyl, benzofuranyl, oxadiazolyl, oxazolyl, isoxazolyl, benzoxazolyl, imidazolyl, benzimidazolyl, thiazolyl, thiadiazolyl, thienyl, pyrrolyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl, thianthrenyl, pyrazolyl, 2H-pyrazolo[3,4-d]pyrimidinyl, 1H-pyrazolo[3,4-d]pyrimidinyl, thieno[2,3-d] pyrimidinyl and the various pyrrolopyridyl radicals.

As used herein, the term 5- to 14-membered heterocyclyl radical embraces typically a non-aromatic, saturated or unsaturated C₅-C₁₄ carbocyclic ring system, preferably C₅-C₁₀ carbocyclic ring system, more preferably C₅-C₆ carbocyclic ring system, in which one or more, for example 1, 2, 3 or 4 of the carbon atoms preferably 1 or 2 of the carbon atoms are replaced by a heteroatom selected from N, O and S. A heterocyclyl radical may be a single ring or two fused rings wherein at least one ring contains a heteroatom. When a 5 to 14-membered heterocyclyl radical carries 2 or more substituents, the substituents may be the same or different.

A said optionally substituted 5- to 14-membered heterocyclyl radical is typically unsubstituted or substituted by 1, 2 or 3 substituents which may be the same or different. Typically, the substituents on a 5 to 14-membered heterocyclyl radical are themselves unsubstituted.

Examples of 5- to 14-membered heterocyclyl radicals include piperidyl, pyrrolidyl, pyrrolinyl, piperazinyl, morpholinyl, thiomorpholinyl, pyrrolyl, pyrazolinyl, pirazolidinyl, quinuclidinyl, triazolyl, pyrazolyl, tetrazolyl, imidazolidinyl, imidazolyl, oxiranyl, thiaranyl, aziridinyl, oxetanyl, thiatanyl, azetidinyl, 4,5-dihydro-oxazolyl, 2-benzofuran-1 (3H)-one, 1,3-dioxol-2-one, tetrahydrofuranyl, 3-aza-tetrahydrofuranyl, tetrahydrothiophenyl, tetrahydropyranyl, tetrahydrothiopyranyl, 1,4-azathianyl, oxepanyl, thiephanyl, azepanyl, 1,4-dioxepnayl, 1,4-oxathiepanyl, 1,4-oxaazepanyl, 1,4-dithiepanyl, 1,4-thiezepanyl, 1,4-diazepanyl, tropanyl, (1S,5R)-3-aza-bicyclo[3.1.0]hexyl, 3,4-dihydro-2H-pyranyl, 5,6-dihydro-2H-pyranyl, 2H-pyranyl, 2,3-hydrobenzofuranyl, 1,2,3,4-tetrahydropyridinyl, 1,2,5,6-tetrahydropyridinyl, isoindolinyl and indolinyl.

Where a 5- to 14-membered heterocyclyl radical carries 2 or more substituents, the substituents may be the same or different.

As used herein, the bicyclic N-containing heteroaryl group is a C₈-C₁₀ membered ring system where two rings have been fused and wherein at least in one ring one of the carbon atoms is replaced by N and optionally in which 1, 2, 3, or 4, preferably 1, 2, or 3 further carbon atoms of any ring which form the group are replaced by N.

Examples include indolyl, benzimidazolyl, indazolyl, benzotriazolyl, pyrrolo[2,3-b]pyridinyl, pyrrolo[2,3-c]pyridinyl, pyrrolo[3,2-c]pyridinyl, pyrrolo[3,2-b]pyridinyl, imidazo[4,5-b]pyridinyl, imidazo[4,5-c]pyridinyl, pyrazolo[4,3-d]pyridinyl, pyrazolo[4,3-d]pyridinyl, pyrazolo[3,4-c]pyridinyl, pyrazolo[3,4-b]pyridinyl, isoindolinyl, indazolyl, purinyl, indolinyl, imidazo[1,2-a]pyridinyl, imidazo[1,5-a]pyridinyl, pyrazolo[1,5-a]pyridinyl, pyrrolo[1,2-b]pyridazinyl, imidazo[1,2-c]pyrimidinyl, quinolyl, isoquinolyl, cinnolinyl, azaquinazolinyl, quinoxalinyl, phthalazinyl, naphthyridinyl, pyrido[3,2-d]pyrimidinyl, pyrido[4,3-d]pyrimidinyl, pyrido[3,4-d]pyrimidinyl, pyrido[2,3-d]pyrimidinyl, pyrazolo[1,5-a]pyrimidinyl, pyrido[2,3-b]pyrazinyl, pyrido[3,4-b]pyrazinyl, pyrimido[5,4-d]pyrimidinyl, pyrazino[2,3-b]pyrazinyl and pyrimido[4,5-d]pyrimidinyl.

As used herein, some of the atoms, radicals, moieties, chains and cycles present in the general structures of the invention are "optionally substituted". This means that these atoms, radicals, moieties, chains and cycles can be either unsubstituted or substituted in any position by one or more, for example 1, 2, 3 or 4, substituents, whereby the hydrogen atoms bound to the unsubstituted atoms, radicals, moieties, chains and cycles are replaced by chemically acceptable atoms, radicals, moieties, chains and cycles. When two or more substituents are present, each substituent may be the same or different. The substituents are typically themselves unsubstituted.

As used herein, the term halogen atom embraces chlorine, fluorine, bromine and iodine atoms. A halogen atom is typically a fluorine, chlorine or bromine atom, most preferably chlorine or fluorine. The term halo when used as a prefix has the same meaning.

Compounds containing one or more chiral centre may be used in enantiomerically or diastereoisomerically pure form, in the form of racemic mixtures and in the form of mixtures enriched in one or more stereoisomer. The scope of the invention as described and claimed encompasses the racemic forms of the compounds as well as the individual enantiomers, diastereomers, and stereoisomer-enriched mixtures.

Conventional techniques for the preparation/isolation of individual enantiomers include chiral synthesis from a suitable optically pure precursor or resolution of the racemate using, for example, chiral high pressure liquid chromatography (HPLC). Alternatively, the racemate (or a racemic precursor) may be reacted with a suitable optically active compound, for example, an alcohol, or, in the case where the compound contains an acidic or basic moiety, an acid or base such as tartaric acid or 1-phenylethylamine. The resulting diastereomehc mixture may be separated by chromatography and/or fractional crystallization and one or both of the diastereoisomers converted to the corresponding pure enantiomer(s) by means well known to one skilled in the art. Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% isopropanol, typically from 2 to 20%, and from 0 to 5% of an alkylamine, typically 0.1 % diethylamine. Concentration of the eluate affords the enriched mixture. Stereoisomer conglomerates may be separated by conventional techniques known to those skilled in the art. See, e.g. "Stereochemistry of Organic Compounds" by Ernest L. Eliel (Wiley, New York, 1994).

Atropisomers are stereoisomers resulting from hindered rotation about single bonds where the steric strain barrier to rotation is high enough to allow for the isolation of the conformers. Oki (Oki, M; Topics in Stereochemistry 1983, 1) defined atropisomers as conformers that interconvert with a half-life of more than 1000 seconds at a given temperature. The scope of the invention as described and claimed encompasses the racemic forms of the compounds as well as the individual atropisomers (an atropisomer "substantially free" of tis corresponding enantionmer) and stereoisomer-enriched mixtures, i.e. mixtures of atropisomers.

Separation of atropisomers is possibly by chiral resolution methods such as selective crystallization. In an atropo-enantioselective or atroposelective synthesis one atropisomer is formed at the expense of the other. Atroposelective synthesis may be carried out by use of chiral auxiliaries like a Corey-Bakshi-Shibata (CBS) catalyst (asymmetric catalyst derived from proline) in the total synthesis of knipholone or by approaches based on thermodynamic equilibration when an isomerization reaction favors one atropisomer over the other.

As used herein, the term pharmaceutically acceptable salt refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

Pharmaceutically acceptable acids include both inorganic acids, for example hydrochloric, sulphuric, phosphoric, diphosphoric, hydrobromic, hydroiodic and nitric acid; and organic acids, for example citric, fumaric, gluconic, glutamic, lactic, maleic, malic, mandelic, mucic, ascorbic, oxalic, pantothenic, succinic, tartaric, benzoic, acetic, methanesulphonic, ethanesulphonic, benzenesulphonic, p-toluenesulphonic acid, xinafoic (1-hydroxy-2-naphthoic acid), napadisilic (1,5-naphthalenedisulfonic acid) and the like. Particularly preferred are salts derived from fumaric, hydrobromic, hydrochloric, acetic, sulfuric, methanesulfonic, xinafoic, and tartaric acids.

Salts derived from pharmaceutically-acceptable inorganic bases include aluminum, ammonium, calcium, copper, ferric, ferrous, lithium, magnesium, manganic, manganous, potassium, sodium, zinc and the like. Particularly preferred are ammonium, calcium, magnesium, potassium and sodium salts.

Salts derived from pharmaceutically-acceptable organic bases include salts of primary, secondary and tertiary amines, including alkyl amines, arylalkyl amines, heterocyclyl amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

Other preferred salts according to the invention are quaternary ammonium compounds wherein an equivalent of an anion (X⁻) is associated with the positive charge of the N atom. X⁻ may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X⁻ is preferably an anion selected from chloride, bromide, iodide, sulphate, nitrate, acetate, maleate, oxalate, succinate or trifluoroacetate. More preferably X⁻ is chloride, bromide, trifluoroacetate or methanesulphonate.

As used herein, an N-oxide is formed from the tertiary basic amines or imines present in the molecule, using a convenient oxidising agent.

The present invention also embraces tautomeric forms of the compounds of formula (I), or pharmaceutically acceptable salts, solvates, N-oxides, stereoisomers or deuterated derivatives thereof.

The compounds of the invention may exist in both unsolvated and solvated forms. The term solvate is used herein to describe a molecular complex comprising a compound of the invention and an amount of one or more pharmaceutically acceptable solvent molecules. The term hydrate is employed when said solvent is water. Examples of solvate forms include, but are not limited to, compounds of the invention in association with water, acetone, dichloromethane, 2-propanol, ethanol, methanol, dimethylsulfoxide (DMSO), ethyl acetate, acetic acid, ethanolamine, or mixtures thereof. It is specifically contemplated that in the present invention one solvent molecule can be associated with one molecule of the compounds of the present invention, such as a hydrate.

Furthermore, it is specifically contemplated that in the present invention, more than one solvent molecule may be associated with one molecule of the compounds of the present invention, such as a dihydrate. Additionally, it is specifically contemplated that in the present invention less than one solvent molecule may be associated with one molecule of the compounds of the present invention, such as a hemihydrate. Furthermore, solvates of the present invention are contemplated as solvates of compounds of the present invention that retain the biological effectiveness of the non-solvate form of the compounds.

The invention also includes isotopically-labeled compounds of the invention, wherein one or more atoms is replaced by an atom having the same atomic number, but an atomic mass or mass number different from the atomic mass or mass number usually found in nature. Examples of isotopes suitable for inclusion in the compounds of the invention include isotopes of hydrogen, such as ²H and ³H, carbon, such as ¹¹C, ¹³C and ¹⁴C, chlorine, such as ³¹Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulfur, such as ³⁵S. Certain isotopically-labeled compounds of the invention, for example, those incorporating a radioactive isotope, are useful in drug and/or substrate tissue distribution studies. The radioactive isotopes tritium, ³H, and carbon-14, ¹⁴C, are particularly useful for this purpose in view of their ease of incorporation and ready means of detection. Substitution with heavier isotopes such as deuterium, ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of the invention can generally be prepared by conventional techniques known to those skilled in the art or by processes analogous to those described herein, using an appropriate isotopically-labeled reagent in place of the non-labeled reagent otherwise employed.

Preferred isotopically-labeled compounds include deuterated derivatives of the compounds of the invention. As used herein, the term deuterated derivative embraces compounds of the invention where in a particular position at least one hydrogen atom is replaced by deuterium. Deuterium (D or ²H) is a stable isotope of hydrogen which is present at a natural abundance of 0.015 molar %.

Hydrogen deuterium exchange (deuterium incorporation) is a chemical reaction in which a covalently bonded hydrogen atom is replaced by a deuterium atom. Said exchange (incorporation) reaction can be total or partial.

Typically, a deuterated derivative of a compound of the invention has an isotopic enrichment factor (ratio between the isotopic abundance and the natural abundance of that isotope, i.e. the percentage of incorporation of deuterium at a given position in a molecule in the place of hydrogen) for each deuterium present at a site designated as a potential site of deuteration on the compound of at least 3500 (52.5% deuterium incorporation).

In a preferred embodiment, the isotopic enrichment factor is at least 5000 (75% deuterium). In a more preferred embodiment, the isotopic enrichment factor is at least 6333.3 (95% deuterium incorporation). In a most preferred embodiment, the isotopic enrichment factor is at least 6633.3 (99.5% deuterium incorporation). It is understood that the isotopic enrichment factor of each deuterium present at a site designated as a site of deuteration is independent from the other deuteration sites.

The isotopic enrichment factor can be determined using conventional analytical methods known too en ordinary skilled in the art, including mass spectrometry (MS) and nuclear magnetic resonance (NMR).

Prodrugs of the compounds described herein are also within the scope of the invention. Thus certain derivatives of the compounds of the present invention, which derivatives may have little or no pharmacological activity themselves, when administered into or onto the body may be converted into compounds of the present invention having the desired activity, for example, by hydrolytic cleavage. Such derivatives are referred to as 'prodrugs'. Further information on the use of prodrugs may be found in Pro-drugs as Novel Delivery Systems, Vol. 14, ACS Symposium Series (T. Higuchi and W. Stella) and Bioreversible Carriers in Drug Design, Pergamon Press, 1987 (ed. E. B. Roche, American Pharmaceutical Association).

Prodrugs in accordance with the invention can, for example, be produced by replacing appropriate functionalities present in the compounds of the present invention with certain moieties known to those skilled in the art as 'pro-moieties' as described, for example, in Design of Prodrugs by H. Bundgaard (Elsevier, 1985).

In the case of compounds that are solids, it is understood by those skilled in the art that the inventive compounds and salts may exist in different crystalline or polymorphic forms, or in an amorphous form, all of which are intended to be within the scope of the present invention.

As used herein, the term PI3Kd inhibitor generally refers to a compound that inhibits the activity of the PI3Kd isoform more effectively than other isoforms of the PI3K family.

As used herein, the term PI3Kd/g inhibitor generally refers to a compound that inhibits the activity of both the PI3Kd isoform and the PI3Kd isoform more effectively than other isoforms of the PI3K family.

The relative efficacies of compounds as inhibitors of an enzyme activity (or other biological activity) can be established by determining the concentrations at which each compound inhibits the activity to a predefined extent and then comparing the results. Typically, the preferred determination is the concentration that inhibits 50% of the activity in a biochemical assay, i.e., the 50% inhibitory concentration or "IC₅₀." IC₅₀ determinations can be accomplished using conventional techniques known in the art. In general, an IC₅₀ can be determined by measuring the activity of a given enzyme in the presence of a range of concentrations of the inhibitor under study. The experimentally obtained values of enzyme activity then are plotted against the inhibitor concentrations used. The concentration of the inhibitor that shows 50% enzyme activity (as compared to the activity in the absence of any inhibitor) is taken as the IC₅₀ value.

Accordingly, a PI3Kd inhibitor alternatively can be understood to refer to a compound that exhibits a 50% inhibitory concentration (IC₅₀) with respect to PI3Kd that is at least of less than about 100 µM, preferably of less than about 50 µM, more preferably of less than about 20 µM, even more preferably of less than about 10 µM PI3K HTRF assay (as described in Gray et al. Anal Biochem, 2003; 313: 234-45)

Typically, in the compound of formula (I), X represents a nitrogen atom, W represents a -CR₆ group and Z represents a -CR₇ group; or X represents a -CR₅ group, W represents a nitrogen atom and Z represents a -CR₇ group; or X represents a -CR₅ group, W represents a -CR₆ group and Z represents nitrogen atom;

In one embodiment, in the compound of formula (I) X represents a nitrogen atom, W represents a -CR₆ group and Z represents a -CR₇ group.

In other embodiment, in the compound of formula (I) X represents a -CR₅ group, W represents a nitrogen atom and Z represents a -CR₇ group.

In another embodiment, in the compound of formula (I) X represents a -CR₅ group, W represents a -CR₆ group and Z represents nitrogen atom.

Typically, in the compound of formula (I), Rₐ and R_{b} each independently represent a hydrogen atom or a linear or branched C₁-C₃ alkyl group.

Preferably, Rₐ and R_{b} each independently represent a hydrogen atom, a methyl group or an ethyl group.

Typically, n represents 0, 1 or 2, preferably 0 or 1, more preferably 0.

Typically, in the compound of formula (I) R₁ represents a C₁-C₃ alkyl group, a C₃-C₇ cycloalkyl group, a phenyl group, a naphtyl group, a 5- to 10- membered monocyclic or bicyclic heteroaryl group containing containing one, two or three heteroatoms selected from O, S and N, or a 5- to 10- membered monocyclic or bicyclic heterocyclyl group containing containing one, two or three heteroatoms selected from O, S and N,
wherein the cycloalkyl, phenyl, naphtyl, heteroaryl and heterocyclyl groups are unsubstituted or substituted by one or more substituents selected from a halogen atom, a hydroxy group, a cyano group, a linear or branched C₁-C₄ alkyl group, a C₁-C₄ haloalkyl group, a C₁-C₄ hydroxyalkyl group, a C₃-C₄ cycloalkyl group, a -(CH₂)₀₋₃OR₈ group, a -(CH₂)₀₋₃NR₈R₉ group, a -C(O)-(CH₂)₀₋₃-R₈ group or a -C(O)-(CH₂)₀₋₃-NR₈R₉ group; wherein R₇ and R₈ are as defined above.

Preferably, R₁ represents a C₃-C₇ cycloalkyl group, a phenyl group, a 5- to 10-membered monocyclic or bicyclic heteroaryl group containing containing one, two or three heteroatoms selected from O, S and N, a pyrrolidinyl group, a piperidinyl group, a piperazinyl group, a tetrahydropyranyl group or a morpholinyl group,
wherein the cycloalkyl, phenyl, heteroaryl, pyrrolidinyl, piperidinyl, piperazinyl, tetrahydropyranyl or morpholinyl groups are unsubstituted or substituted by one or more substituents selected from a halogen atom, a hydroxy group, a linear or branched C₁-C₄ alkyl group, a C₁-C₄ haloalkyl group, a C₁-C₄ hydroxyalkyl group, a C₃-C₄ cycloalkyl group, a -(CH₂)₀₋₃OR₈ group, a -(CH₂)₀₋₃NR₈R₉ group, a -C(O)-(CH₂)₀₋₃-R₈ group or a -C(O)-(CH₂)₀₋₃-NR₈R₉ group; wherein R₈ and R₉ each independently represent a hydrogen atom or a C₁-C₄ alkyl group.

More preferably R₁ preferably represents a phenyl group, a pyridinyl group or a thiazolyl group, which phenyl, pyridinyl or thiazolyl groups are unsubstituted or substituted by one, two or three substituents selected from a halogen atom, a hydroxy group, a linear or branched C₁-C₃ alkyl group, a C₁-C₃ haloalkyl group or a -(CH₂)₀₋₃OCH₃ group.

Most preferably R₁ preferably represents a phenyl group or a pyridinyl group, which phenyl or pyridinyl groups are unsubstituted or substituted by one, two or three substituents selected from a halogen atom, a hydroxy group, a linear or branched C₁-C₃ alkyl group, a C₁-C₃ haloalkyl group or a -(CH₂)₀₋₃OCH₃ group.

Preferably, when R₁ represents a phenyl group or a pyridinyl group, said phenyl and pyridinyl groups are directly bonded to the imidazopyridine group. In other words, the linker -(Rₐ-C-R_{b})₀₋₃- is not present. More preferably, R₁ represents a phenyl group.

Preferably, when R₁ is a phenyl group, it is unsubstituted or substituted by one, two or three substituents selected from a halogen atom (preferably a fluorine atom or a chlorine atom), a linear or branched C₁-C₃ alkyl group (preferably a methyl group), or a -OCH₃ group.

Preferably, when R₁ is a pyridinyl group, it is unsubstituted or substituted by one, two or three substituents selected from a halogen atom (preferably a fluorine atom or a chlorine atom), a hydroxy group, a linear or branched C₁-C₃ alkyl group (preferably a methyl group), or a -OCH₃ group.

Preferably, when R₁ is a pyridinyl group or a thiazolyl group, said groups are linked to the rest of the molecule via a ring carbon atom, in other words they are linked to the imidazopyridine group via a ring carbon atom. Substituents on a pyridinyl or a thiazolyl groups may be present on any ring atom but are preferably present on a carbon atom.

Typically, in the compound of formula (I) R₂ represents a hydrogen atom, a halogen atom, a hydroxy group, a cyano group, a C₁-C₄ alkoxy group, a C₁-C₄ haloalkyl group, a C₁-C₄ hydroxyalkyl group, a C₃-C₄ cycloalkyl group, a -(CH₂)₀₋₃NR'R" group, or a linear or branched C₁-C₄ alkyl group, which alkyl group is unsubstituted or substituted by a C₁-C₃ alkoxy group; wherein R' and R" each independently represent a hydrogen atom, a hydroxy group, or a linear or branched C₁-C₃ alkyl group.

Preferably R₂ represents a hydrogen atom, a halogen atom, a hydroxy group, a C₁-C₃ alkoxy group, a C₁-C₃ haloalkyl group, a C₃-C₄ cycloalkyl group, a -NH₂ group, a -N(CH₃)H group, a -N(CH₃)₂ group, or a linear or branched C₁-C₄ alkyl group, which alkyl group is unsubstituted or substituted by a C₁-C₂ alkoxy group.

More preferably R₂ represents a hydrogen atom, a halogen atom (preferably a fluorine atom or a chlorine atom, -NH₂ group, a -N(CH₃)H group, a -N(CH₃)₂ group, or a linear or branched C₁-C₃ alkyl group. Most preferably R₂ represents a hydrogen atom or a methyl group.

Typically, in the compound of formula (I), R₃ represents a hydrogen atom, a C₁-C₄ alkoxy group, a C₁-C₄ haloalkyl group, a C₁-C₄ hydroxyalkyl group, a C₃-C₄ cycloalkyl group, a -(CH₂)₁₋₄NR'R" group, or a linear or branched C₁-C₄ alkyl group, which alkyl group is unsubstituted or substituted by a C₁-C₃ alkoxy group, a -C(O)-(CH2)₀₋₃-R' group or a -C(O)-(CH₂)₀₋₃-NR'R" group; wherein R' and R" each independently represent a hydrogen atom, a hydroxy group, or a linear or branched C₁-C₃ alkyl group. Preferably, R₃ represents a hydrogen atom, a C₁-C₃ alkoxy group, a C₁-C₃ haloalkyl group, a C₃-C₄ cycloalkyl group, or a linear or branched C₁-C₃ alkyl group. More preferably R₃ represents a hydrogen atom, a C₁-C₃ haloalkyl group or a linear or branched C₁-C₃ alkyl group.

Typically, in the compound of formula (I), R₅, R₆ and R₇ each independently represent a hydrogen atom, a halogen atom, a hydroxy group, a cyano group, a C₁-C₄ alkoxy group, a C₁-C₄ haloalkyl group, a C₁-C₄ hydroxyalkyl group, a C₃-C₄ cycloalkyl group, a -(CH₂)₀₋₃NR'R" group, or a linear or branched C₁-C₄ alkyl group, which alkyl group is unsubstituted or substituted by a C₁-C₃ alkoxy group; wherein R' and R" each independently represent a hydrogen atom, a hydroxy group, or a linear or branched C₁-C₃ alkyl group. Preferably, R₅, R₆ and R₇ each independently represent a hydrogen atom, a halogen atom, a hydroxy group, a C₁-C₃ alkoxy group, a C₁-C₃ haloalkyl group, a C₃-C₄ cycloalkyl group, a -NH₂ group, a -N(CH₃)H group, a -N(CH₃)₂ group, or a linear or branched C₁-C₄ alkyl group, which alkyl group is unsubstituted or substituted by a C₁-C₂ alkoxy group. More preferably, R₅, R₆ and R₇ each independently represent a hydrogen atom, a halogen atom, a hydroxy group, a C₁-C₃ haloalkyl group (preferably a -CHF₂ group or a -CF₃ group), a -NH₂ group, a -N(CH₃)H group, a -N(CH₃)₂ group or a linear or branched C₁-C₃ alkyl group.

In a particular embodiment, R₄ represents a group selected from
i) a group of formula (IIa-1), 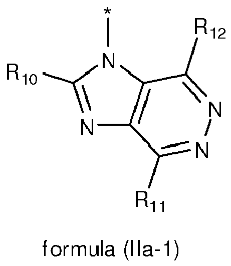
ii) a group of formula (IIa-2), 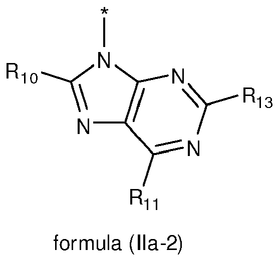 and iii) a group of formula (IIa'-1) 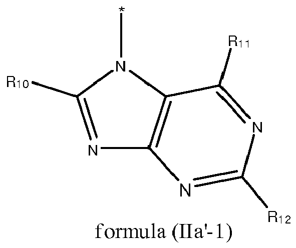
wherein
R₁₀, R₁₁, R₁₂ and R₁₃ each independently represent a hydrogen atom, a C₁-C₄ alkoxy group, a C₁-C₄ haloalkyl group, a C₁-C₄ hydroxyalkyl group, a C₃-C₄ cycloalkyl group, a -(CH₂)₀₋₃CN group, a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group; wherein R' and R" each independently represent a hydrogen atom, a hydroxy group, a C₁-C₄ alkoxy group or a linear or branched C₁-C₄ alkyl group.

In this particular embodiment, preferably R₄ represents a group of formula (IIa-2) wherein R₁₀, R₁₁, and R₁₃ each independently represent a hydrogen atom, a -(CH₂)₀₋₃CN group, a -C(O)-(CH₂)₁-₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group; wherein R' and R" each independently represent a hydrogen atom, a hydroxy group, a C₁-C₄ alkoxy group or a linear or branched C₁-C₄ alkyl group. More preferably, R₁₀ and R₁₃ each independently represent a hydrogen atom and R₁₁ represents a -(CH₂)₀₋₃NR'R" group wherein R' and R" each independently represent a hydrogen atom or methyl group. Even more preferably, R₁₀ and R₁₃ each independently represent a hydrogen atom and R₁₁ represents a -NH₂ group.

In another particular embodiment, R₄ represents a group selected from 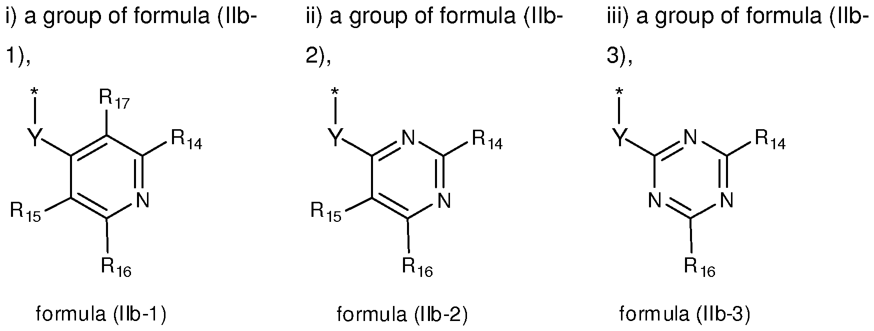 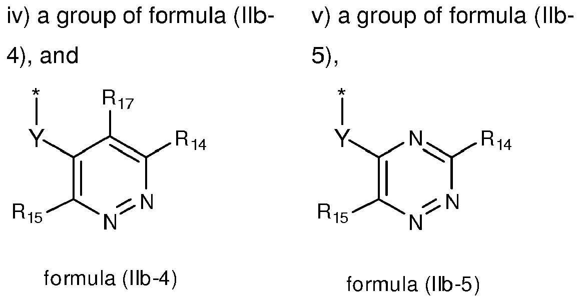 wherein
R₁₄, R₁₅, R₁₆ and R₁₇ each independently represent a hydrogen atom, a C₁-C₄ alkoxy group, a C₁-C₄ haloalkyl group, a C₁-C₄ hydroxyalkyl group, a C₃-C₄ cycloalkyl group, a -(CH₂)₀₋₃CN group, a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group; wherein R' and R" each independently represent a hydrogen atom, a hydroxy group, a C₁-C₄ alkoxy group or a linear or branched C₁-C₄ alkyl group; and wherein Y represents a linker selected from a -NR'-group, -O- or -S-; wherein R' is as defined above;
or in the case that Y represents a -NR'- group, R₃ together with the -NR'- group and the carbon atom to which both R₃ and the -NR'- group are bonded form a 4- to 7-membered, saturated N-containing heterocyclyl group, which heterocyclyl group is unsubstituted or substituted by one or more substituents selected from a halogen atom, a hydroxyl group, a cyano group, a -CHF₂ group or a -CF₃ group.

In this particular embodiment, preferably R₄ represents a group selected from a group of formula (IIb-1) and a group of formula (IIb-2) wherein
R₁₄, R₁₅, R₁₆ and R₁₇ each independently represent a hydrogen atom, a -(CH₂)₀₋₃CN group, a -C(O)-(CH₂)₀₋₃-NR'R" or a -(CH₂)₀₋₃NR'R" group; wherein R' and R" each independently represent a hydrogen atom, a hydroxy group, or a linear or branched C₁-C₄ alkyl group; and wherein Y represents a -NR'- group, wherein R' is as defined before.

Preferably, when R₄ is a group of formula (IIb-1) R₁₅ and R₁₇ each independently represent a hydrogen atom, and R₁₄ and R₁₆ each independently represent a hydrogen atom, a -C(O)-(CH₂)₀₋₃-NR'R" or a -(CH₂)₀₋₃NR'R" group; wherein R' and R" each independently represent a hydrogen atom, a hydroxy group, or a linear or branched C₁-C₃ alkyl group; wherein Y represents a -NR'- group, wherein R' is as defined before. Even more preferably, R₁₅ and R₁₇ each independently represent a hydrogen atom, and R₁₃ and R₁₅ each independently represent a hydrogen atom, a -C(O)-NR'R' group or a -NR'R" group; wherein R' and R" each independently represent a hydrogen atom or a methyl group; wherein Y represents a -NH- group.

Preferably, when R₄ is a group of formula (IIb-2) R₁₄ represents a hydrogen atom or a -(CH₂)₀₋₃NR'R" group, and R₁₅ and R₁₆ each independently represent a hydrogen atom, a -(CH₂)₀₋₃CN group, a -C(O)-(CH₂)₀₋₃-NR'R" or a -(CH₂)₀₋₃NR'R" group; wherein R' and R" each independently represent a hydrogen atom, a hydroxy group, or a linear or branched C₁-C₃ alkyl group; wherein Y represents a -NR'- group, wherein R' is as defined before. More preferably, R₁₄ represents a hydrogen atom or a -NR'R" group, and R₁₅ and R₁₆ each independently represent a hydrogen atom, a -CN group or a -NR'R" group; wherein R' and R" each independently represent a hydrogen atom or a methyl group; and wherein Y represents a -NH- group. Even more preferably, R₁₄ represents a hydrogen atom, and R₁₅ and R₁₆ each independently represent a hydrogen atom, a -CN group or a -NH₂ group; and wherein Y represents a -NH- group.

In a further particular embodiment, R₄ represents a group selected from 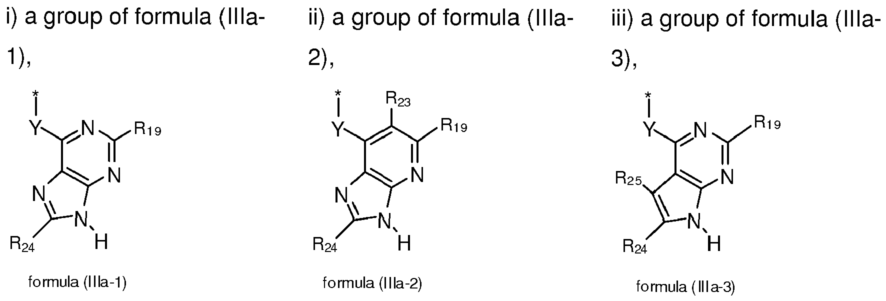 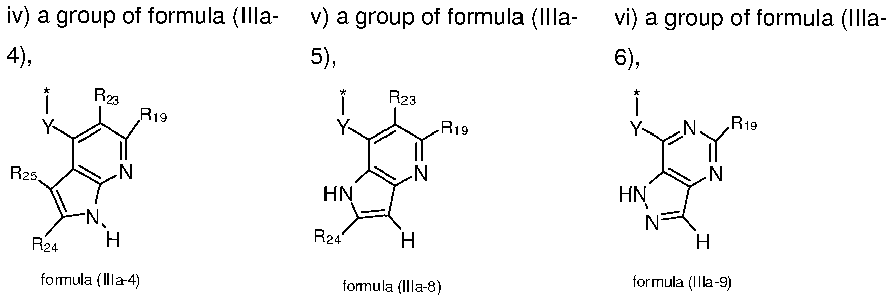 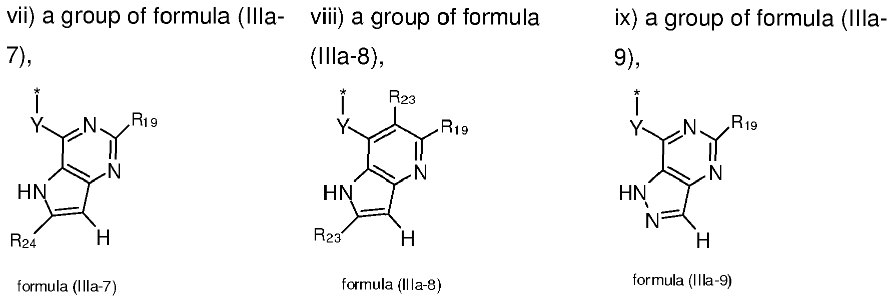 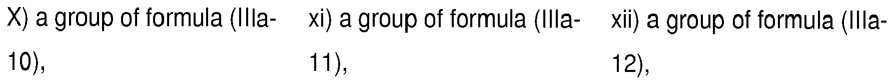 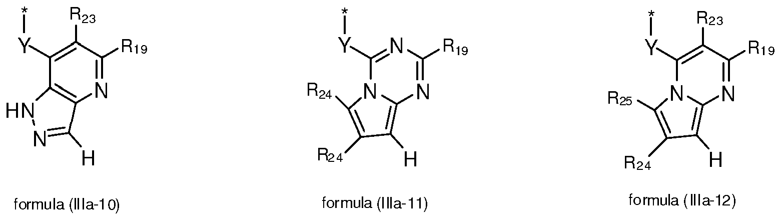 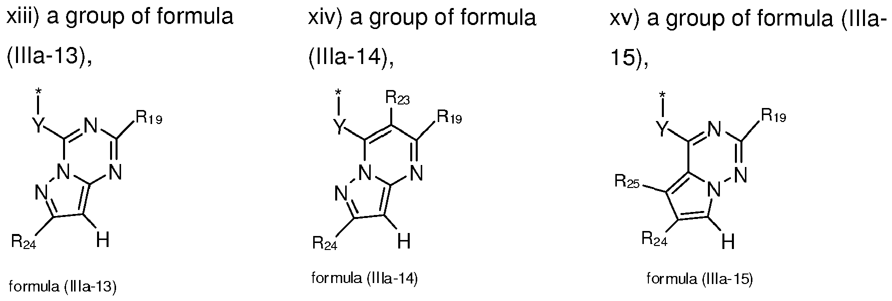 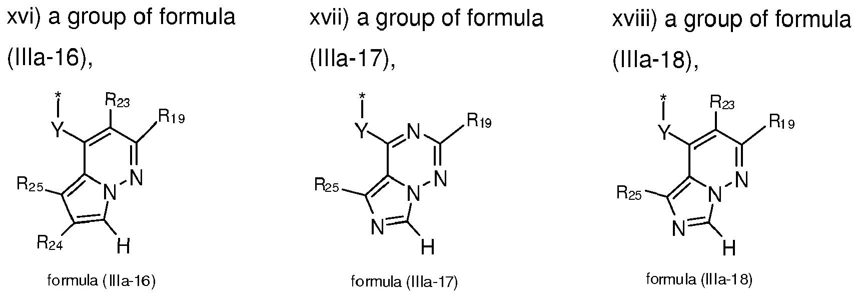 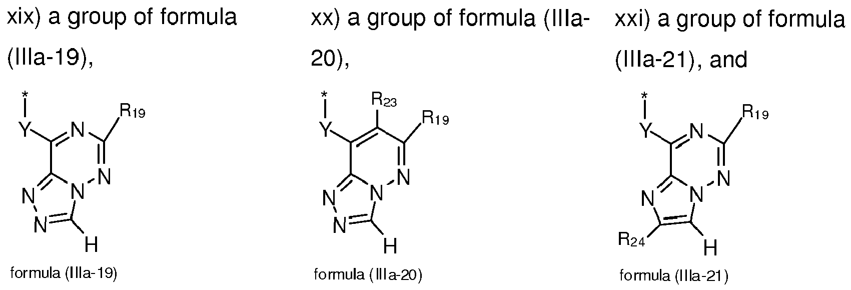 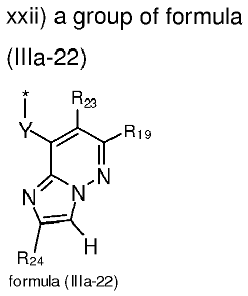 wherein
R₁₉, R₂₂, R₂₃, R₂₄, and R₂₅ each independently represent a hydrogen atom, a C₁-C₄ alkoxy group, a C₁-C₄ haloalkyl group, a C₁-C₄ hydroxyalkyl group, a C₃-C₄ cycloalkyl group, a -(CH₂)₀₋₃CN group, a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group; wherein R' and R" each independently represent a hydrogen atom, a hydroxy group, a C₁-C₄ alkoxy group or a linear or branched C₁-C₄ alkyl group; and wherein Y represents a linker selected from a -NR'-group, -O- or -S-; wherein R' is as defined above;
or in the case that Y represents a -NR'- group, R₃ together with the -NR'- group and the carbon atom to which both R₃ and the -NR'- group are bonded form a 4- to 7-membered, saturated N-containing heterocyclyl group, which heterocyclyl group is unsubstituted or substituted by one or more substituents selected from a halogen atom, a hydroxyl group, a cyano group, a -CHF₂ group or a -CF₃ group.

In this particular embodiment, preferably R₄ represents a group selected from a group of formula (IIIa-1) and a group of formula (IIIa-14) wherein
R₁₉, R₂₃ and R₂₄ each independently represent a hydrogen atom, a -(CH₂)₀₋₃CN group, a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group; wherein R' and R" each independently represent a hydrogen atom, a hydroxy group, a C₁-C₄ alkoxy group or a linear or branched C₁-C₄ alkyl group; and wherein Y is as defined above;
or in the case that Y represents a -NR'- group, R₃ together with the -NR'- group and the carbon atom to which both R₃ and the -NR'- group are bonded form a 4- to 7-membered, saturated N-containing heterocyclyl group, which heterocyclyl group is unsubstituted or substituted by one or more substituents selected from a halogen atom, a hydroxyl group, a cyano group, a -CHF₂ group or a -CF₃ group.

Preferably, when R₄ is a group of formula (IIIa-1) R₁₉ and R₂₄ each independently represent a hydrogen atom, a -C(O)-(CH₂)₀₋₃-NR'R" or a -(CH₂)₀₋₃NR'R" group; wherein R' and R" each independently represent a hydrogen atom, a hydroxy group, or a linear or branched C₁-C₃ alkyl group; and wherein Y represents a -NR'- group or -S-, wherein R' is as defined before. Even more preferably, R₁₉ and R₂₄ each independently represent a hydrogen atom or a -NR'R" group; wherein R' and R" each independently represent a hydrogen atom or a methyl group; and wherein Y represents a -NR'- group or -S-, wherein R' is as defined before. Most preferably, R₁₉ and R₂₄ each independently represent a hydrogen atom, a -NH₂ group, a -N(CH₃)H group or a -N(CH₃)₂ group; and Y represents a -NH- group.

Preferably, when R₄ is a group of formula (IIIa-1) wherein Y represents a -NR'- group, wherein R' is a linear or branched C₁-C₄ alkyl group; R₄ together with the -NR'- group and the carbon atom to which both R₄ and the -NR'- group are bonded form a 4- to 7-membered, saturated N-containing heterocyclyl group, which heterocyclyl group is unsubstituted or substituted by one or more substituents selected from a halogen atom, a hydroxyl group, a cyano group, a -CHF₂ group or a -CF₃ group. More preferably, R₄ together with the -NR'- group of R₅ and the carbon atom to which both R₄ and the -NR'-group are bonded form an azetidinyl group, a pyrrolidinyl group, a piperidinyl group or a piperazinyl group; even more preferably a pyrrolidinyl group or a piperidinyl group.

Preferably, when R₄ is a group of formula (IIIa-14) R₁₉, R₂₃ and R₂₄ each independently represent a hydrogen atom, a -C(O)-(CH₂)₀₋₃-NR'R" or a -(CH₂)₀₋₃NR'R" group; wherein R' and R" each independently represent a hydrogen atom, a hydroxy group, or a linear or branched C₁-C₃ alkyl group; and wherein Y represents a -NR'- group, wherein R' is as defined before. Even more preferably, R₁₉, R₂₃ and R₂₄ each independently represent a hydrogen atom or a -NR'R" group; wherein R' and R" each independently represent a hydrogen atom or a methyl group; and wherein Y represents a -NR'- group, wherein R' is as defined before. Most preferably, R₁₉, R₂₃ and R₂₄ each independently represent a hydrogen atom and Y represents a -NH- group.

In another particular embodiment, R₄ represents a group selected from 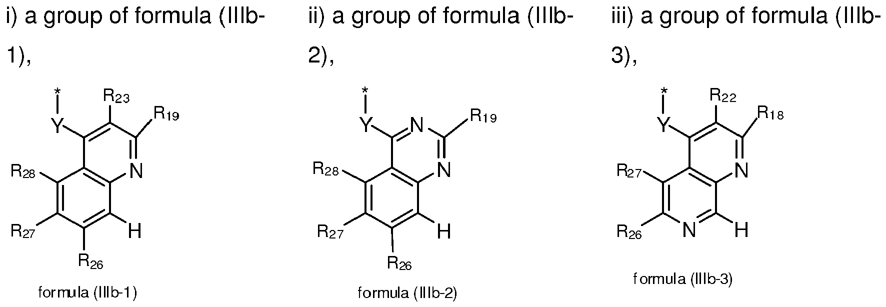 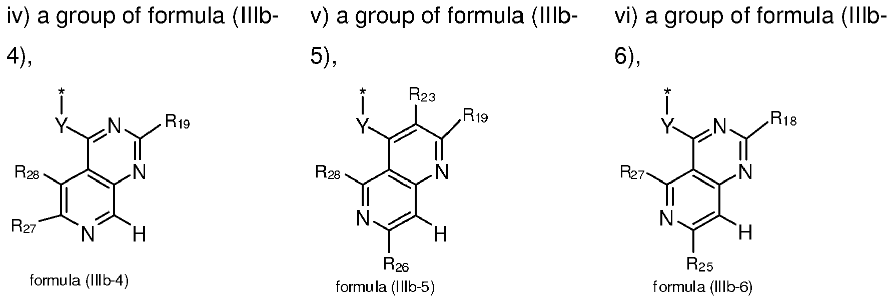 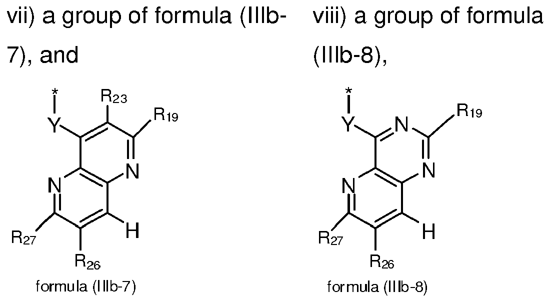 wherein
R₁₉, R₂₃, R₂₆, R₂₇, and R₂₈ each independently represent a hydrogen atom, a C₁-C₄ alkoxy group, a C₁-C₄ haloalkyl group, a C₁-C₄ hydroxyalkyl group, a C₃-C₄ cycloalkyl group, a -(CH₂)₀₋₃CN group, a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group; wherein R' and R" each independently represent a hydrogen atom, a hydroxy group, a C₁-C₄ alkoxy group or a linear or branched C₁-C₄ alkyl group; and wherein Y represents a linker selected from a -NR'-group, -O- or -S-; wherein R' is as defined above;
or in the case that Y represents a -NR'- group, R₃ together with the -NR'- group and the carbon atom to which both R₃ and the -NR'- group are bonded form a 4- to 7-membered, saturated N-containing heterocyclyl group, which heterocyclyl group is unsubstituted or substituted by one or more substituents selected from a halogen atom, a hydroxyl group, a cyano group, a -CHF₂ group or a -CF₃ group.

In a further particular embodiment, R₄ represents a group selected from 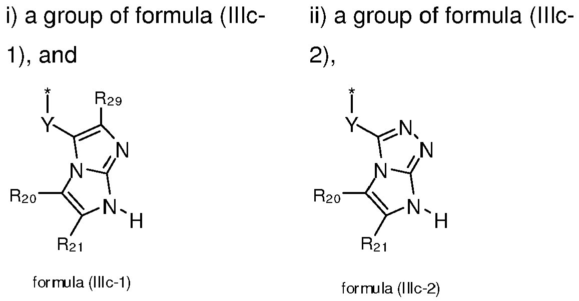 wherein
R₂₀, R₂₁, and R₂₉ each independently represent a hydrogen atom, a C₁-C₄ alkoxy group, a C₁-C₄ haloalkyl group, a C₁-C₄ hydroxyalkyl group, a C₃-C₄ cycloalkyl group, a -(CH₂)₀₋₃CN group, a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group; wherein R' and R" each independently represent a hydrogen atom, a hydroxy group, a C₁-C₄ alkoxy group or a linear or branched C₁-C₄ alkyl group; and wherein Y represents a linker selected from a -NR'-group, -O- or -S-; wherein R' is as defined above;
or in the case that Y represents a -NR'- group, R₃ together with the -NR'- group and the carbon atom to which both R₃ and the -NR'- group are bonded form a 4- to 7-membered, saturated N-containing heterocyclyl group, which heterocyclyl group is unsubstituted or substituted by one or more substituents selected from a halogen atom, a hydroxyl group, a cyano group, a -CHF₂ group or a -CF₃ group.

In another particular embodiment, R₄ represents a group selected from 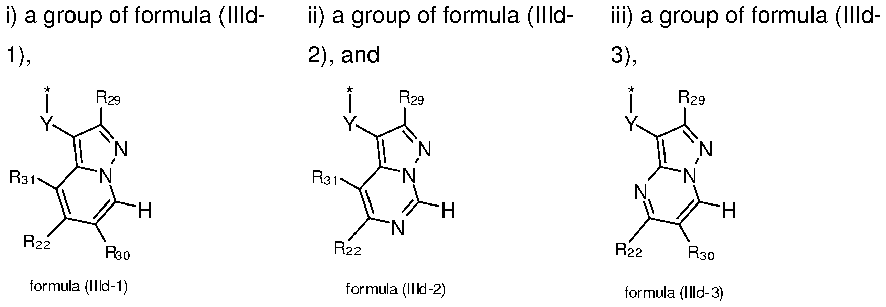 wherein
R₂₂, R₂₉, R₃₀, and R₃, each independently represent a hydrogen atom, a C₁-C₄ alkoxy group, a C₁-C₄ haloalkyl group, a C₁-C₄ hydroxyalkyl group, a C₃-C₄ cycloalkyl group, a -(CH₂)₀₋₃CN group, a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group; wherein R' and R" each independently represent a hydrogen atom, a hydroxy group, C₁-C₄ alkoxy group or a linear or branched C₁-C₄ alkyl group; and wherein Y represents a linker selected from a -NR'- group, - O- or -S-; wherein R' is as defined above;
or in the case that Y represents a -NR'- group, R₃ together with the -NR'- group and the carbon atom to which both R₃ and the -NR'- group are bonded form a 4- to 7-membered, saturated N-containing heterocyclyl group, which heterocyclyl group is unsubstituted or substituted by one or more substituents selected from a halogen atom, a hydroxyl group, a cyano group, a -CHF₂ group or a -CF₃ group.

Typically, in the compound of formula (I), Y represents a linker selected from a -NR'-group, -O- or -S-; wherein R' represents a hydrogen atom, a hydroxy group, a C₁-C₄ alkoxy group or a linear or branched C₁-C₄ alkyl group. Preferably Y represents a linker selected from a -NR'- group or -S-; wherein R' represents a hydrogen atom, a hydroxy group, a C₁-C₄ alkoxy group or a linear or branched C₁-C₄ alkyl group. More preferably Y represents a linker selected from a -NR'- group or -S-; wherein R' represents a hydrogen atom, a hydroxy group or a linear or branched C₁-C₃ alkyl group. Most preferably Y represents a -NR'- group; wherein R' represents a hydrogen atom or a linear or branched C₁-C₃ alkyl group.

When R' and/or R" are attached to a nitrogen atom, preferably R' and/or R" do not represent a hydroxy group or alkoxy group.

Where any of the above moieties represent C(O)-(CH₂)₀₋₃-R₈ or -C(O)-(CH₂)₀₋₃-R', it is preferable that R₈ and R' do not represent a hydrogen atom if the alkylene spacer moiety is absent.

Preferably, in the compound of formula (I):
Rₐ and R_{b} each independently represent a hydrogen atom or a linear or branched C₁-C₃ alkyl group;
n represents 0, 1 or 2;
R₁ represents a C₃-C₇ cycloalkyl group, a phenyl group, a 5- to 10- membered monocyclic or bicyclic heteroaryl group containing containing one, two or three heteroatoms selected from O, S and N, a pyrrolidinyl group, a piperidinyl group, a piperazinyl group, a tetrahydropyranyl group, a 2-pyridone group or a morpholinyl group,
   wherein the cycloalkyl, phenyl, heteroaryl, pyrrolidinyl, piperidinyl, piperazinyl, tetrahydropyranyl, 2-pyridone or morpholinyl groups are unsubstituted or substituted by one or more substituents selected from a halogen atom, a hydroxy group, a linear or branched C₁-C₄ alkyl group, a C₁-C₄ haloalkyl group, a C₁-C₄ hydroxyalkyl group, a C₃-C₄ cycloalkyl group, a -(CH₂)₀₋₃OR₈ group, a -(CH₂)₀₋₃NR₈R₉ group, a -C(O)-(CH₂)₀₋₃-R₈ group or a -C(O)-(CH₂)₀₋₃-NR₈R₉ group; wherein R₈ and R₉ each independently represent a hydrogen atom or a C₁-C₄ alkyl group;
R₂ represents a hydrogen atom, a halogen atom, a hydroxy group, a C₁-C₃ alkoxy group, a C₁-C₃ haloalkyl group, a C₃-C₄ cycloalkyl group, a -NH₂ group, a -N(CH₃)H group, a -N(CH₃)₂ group, or a linear or branched C₁-C₄ alkyl group, which alkyl group is unsubstituted or substituted by a C₁-C₂ alkoxy group;
R₃ represents a hydrogen atom, a C₁-C₃ alkoxy group, a C₁-C₃ haloalkyl group, a C₃-C₄ cycloalkyl group, or a linear or branched C₁-C₃ alkyl group;
R₅, R₆ and R₇ each independently represent a hydrogen atom, a halogen atom, a hydroxy group, a C₁-C₃ alkoxy group, a C₁-C₃ haloalkyl group, a C₃-C₄ cycloalkyl group, a -NH₂ group, a -N(CH₃)H group, a -N(CH₃)₂ group, or a linear or branched C₁-C₄ alkyl group, which alkyl group is unsubstituted or substituted by a C₁-C₂ alkoxy group;
R₄ represents a group of formula (IIa-2), (IIa'-1), (IIb-2), (IIIa-1) or (IIIa-14) 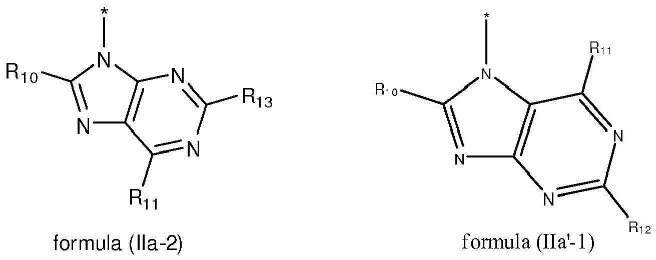 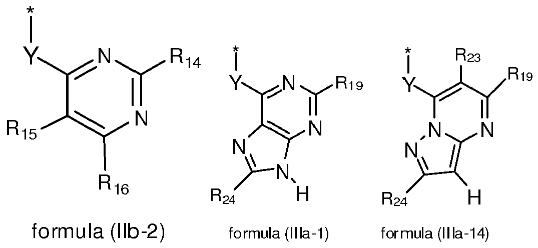 wherein:
   - $: R₁₀, R₁₁, and R₁₃ each independently represent a hydrogen atom, a -(CH₂)₀₋₃CN group, a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group; wherein R' and R" each independently represent a hydrogen atom, a hydroxy group, a C₁-C₄ alkoxy group or a linear or branched C₁-C₄ alkyl group;
   - $: R₁₄, R₁₅ and R₁₆ each independently represent a hydrogen atom, a -(CH₂)₀₋₃CN group, a -C(O)-(CH₂)₀₋₃-NR'R" or a -(CH₂)₀₋₃NR'R" group; wherein R' and R" each independently represent a hydrogen atom, a hydroxy group, or a linear or branched C₁-C₄ alkyl group;
   - $: R₁₉, R₂₃ and R₂₄ each independently represent a hydrogen atom, a -(CH₂)₀₋₃CN group, a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group;wherein R' and R" each independently represent a hydrogen atom, a hydroxy group, a C₁-C₄ alkoxy group or a linear or branched C₁-C₄ alkyl group; and
   - $: Y represents a -NR'- group, -O- or -S- wherein R' represents hydrogen or a linear or branched C₁-C₄ alkyl group; or in the case that Y represents a -NR'-group, R₃ together with the -NR'- group and the carbon atom to which both R₃ and the -NR'- group are bonded form a 4- to 7- membered, saturated N-containing heterocyclyl group.

In a particularly preferred embodiment, in the compound of formula (I)
X represents a nitrogen atom, W represents a -CR₆ group and Z represents a -CR₇ group; or X represents a -CR₅ group, W represents a -CR₆ group and Z represents nitrogen atom;
Rₐ and R_{b} each independently represent a hydrogen atom or a methyl group;
n represents 0 or 1;
R₁ represents a C₃-C₇ cycloalkyl group, a phenyl group, a pyridinyl group, a thiazolyl group, a piperidinyl group or a 2-pyridone group;
wherein the cycloalkyl, phenyl, pyridinyl, thiazolyl,piperidinyl groups and 2-pyridone group are unsubstituted or substituted by one or more substituents selected from a halogen atom, a hydroxy group, a C₁-C₃ haloalkyl group, a linear or branched C₁-C₃ alkyl group, a -NR₈R₉ group or a -OR₉ group; wherein R₈ and R₉ each independently represent a hydrogen atom or a linear or branched C₁-C₃ alkyl group;
R₂ represents a hydrogen atom, a halogen atom, a -NR'R" group or a linear or branched C₁-C₃ alkyl group;
R₃ represents a hydrogen atom or a linear or branched C₁-C₃ alkyl group;
R₅, R₆ and R₇ each independently represent a hydrogen atom, a hydroxy group, a C₁-C₃ alkoxy group, a -(CH₂)₀₋₃NR'R" group, or a linear or branched C₁-C₃ alkyl group;
R₄ represents a group selected from:
i) a group of formula (IIa), which group is a purinyl group unsubstituted or substituted by a -NR'R" group;
ii) a group of formula (IIb), which group is selected from a -NH-pyrimidinyl group, a -N(CH₃)-pyrimidinyl group or a -S-pyrimidinyl group; wherein the pyrimidinyl group is unsubstituted or substituted by one, two or three substituents selected from a -(CH₂)₀₋₃CN group, a -C(O)-(CH₂)₀₋₃-NR'R" group or a -(CH₂)₀₋₃NR'R" group, preferably from a -CN group, a -CONH₂ group or a -NH₂ group;
iii) a group of formula (IIc), which group is selected from a -NH-purinyl group, a -S-purinyl group, a -N(CH₃)-purinyl group or a -NH-pyrazolo[1,5-a]pyrimidinyl group; wherein the purinyl and pyrazolo[1,5-a]pyrimidinyl groups are unsubstituted or substituted by a -(CH₂)₀₋₃NR'R" group; or
R₃ and R₄ together with the carbon atom to which they are attached form (a) a pyrrolidinyl-pyrimidinyl group in which the pyrimidinyl group is unsubstituted or substituted by one, two or three substituents selected from a -(CH₂)₀₋₃CN group, a -C(O)-(CH₂)₀₋₃-NR'R" group or a -(CH₂)₀₋₃NR'R" group, or (b) a pyrrolidinyl-purinyl group wherein the purinyl group is unsubstituted or substituted by a -(CH₂)₀₋₃NR'R" group;
R' and R" each independently represent a hydrogen atom, a hydroxy group, a C₁-C₃ alkoxy group or a linear or branched C₁-C₃ alkyl group, preferably a hydrogen atom or a linear or branched C₁-C₃ alkyl group.

In another particularly preferred embodiment, in the compound of formula (I)
X represents a nitrogen atom, W represents a -CR₆ group and Z represents a -CR₇ group; or X represents a -CR₅ group, W represents a -CR₆ group and Z represents nitrogen atom;
Rₐ and R_{b} each independently represent a hydrogen atom or a methyl group;
n represents 0 or 1;
R₁ represents a C₃-C₇ cycloalkyl group, a phenyl group, a pyridinyl group, a thiazolyl group, a piperidinyl group or a 2-pyridone group;
wherein the cycloalkyl, phenyl, pyridinyl, thiazolyl,piperidinyl groups and 2-pyridone group are unsubstituted or substituted by one or more substituents selected from a halogen atom, a hydroxy group, a C₁-C₃ haloalkyl group, a linear or branched C₁-C₃ alkyl group, a -NR₈R₉ group or a -OR₉ group; wherein R₈ and R₉ each independently represent a hydrogen atom or a linear or branched C₁-C₃ alkyl group;
R₂ represents a hydrogen atom, a halogen atom, a -NR'R" group or a linear or branched C₁-C₃ alkyl group;
R₃ represents a hydrogen atom or a linear or branched C₁-C₃ alkyl group;
R₅, R₆ and R₇ each independently represent a hydrogen atom, a hydroxy group, a C₁-C₃ alkoxy group, a -(CH₂)₀₋₃NR'R" group, or a linear or branched C₁-C₃ alkyl group;
R₄ represents a group of formula (IIa-2), (IIa'-1), (IIb-2), (IIIa-1) or (IIIa-14) 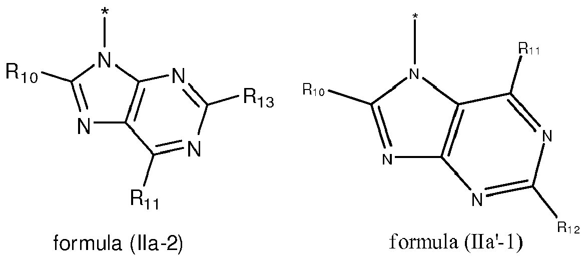 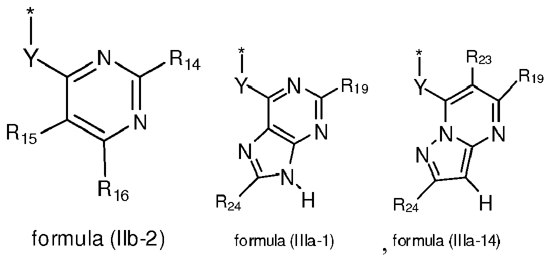 wherein:
- $: R₁₀, R₁₁ and R₁₃ each independently represent a hydrogen atom or a -NR'R" group;
- $: R₁₄, R₁₅ and R₁₆ each independently represent a hydrogen atom, a -NR'R" group or a CN group,
- $: R₁₉, R₂₃ and R₂₄ each independently represent a hydrogen atom or NR'R" group;
- $: R' and R" each independently represent a hydrogen atom or a linear or branched C₁-C₃ alkyl group;
- $: Y represents -NH-, -NCH₃- or -S-; or Y represents N and Y, R₃ and the carbon atom to which both R₃ and Y are bonded form a pyrrolidinyl ring.

In a particularly preferred embodiment, the compound of the invention is of formula (Ia) 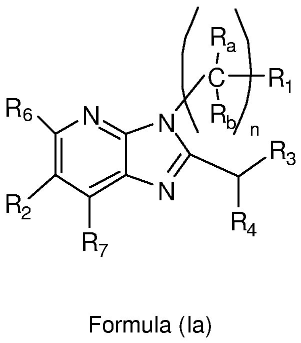 wherein R₁, R₂, R₃, R₄, R₆, R₇, Rₐ, R_{b} and n are as defined above.

In an alternative particularly preferred embodiment, the compound is of formula (Ib): 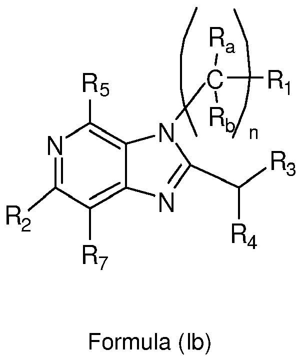 wherein R₁, R₂, R₃, R₄, R₅, R₇, Rₐ, R_{b} and n are as defined above.

In a further particularly preferred embodiment, the compound of the invention is of formula (Ic) 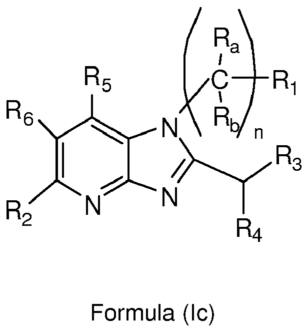 wherein R₁, R₂, R₃, R₄, R₅, R₆, Rₐ, R_{b} and n are as defined above.

Particular individual compounds of the invention include:
(S)-N-(1-(3-Phenyl-3H-imidazo[4,5-b]pyridin-2-yl)ethyl)-9H-purin-6-amine;
9-((3-Phenyl-3H-imidazo[4,5-b]pyridin-2-yl)methyl)-9H-purin-6-amine;
9-(1-(3-Phenyl-3H-imidazo[4,5-b]pyridin-2-yl)ethyl)-9H-purin-6-amine;
6-(1-(3-Phenyl-3H-imidazo[4,5-b]pyridin-2-yl)ethylthio)-9H-purine;
(S)-N-(1-(3-Phenyl-3H-imidazo[4,5-b]pyridin-2-yl)propyl)-9H-purin-6-amine;
(S)-N4-(1-(3-Phenyl-3H-imidazo[4,5-b]pyridin-2-yl)ethyl)pyrimidine-4,6-diamine;
(S)-6-(2-(3-Phenyl-3H-imidazo[4,5-b]pyridin-2-yl)pyrrolidin-1-yl)-9H-purine;
(S)-6-(2-(3-Phenyl-3H-imidazo[4,5-b]pyridin-2-yl)pyrrolidin-1-yl)pyrimidin-4-amine;
(S)-N6-(1-(3-Phenyl-3H-imidazo[4,5-b]pyridin-2-yl)ethyl)-9H-purine-2,6-diamine;
(S)-N-(1-(5-Methoxy-3-phenyl-3H-imidazo[4,5-b]pyridin-2-yl)ethyl)-9H-purin-6-amine;
(S)-N-(1-(3-(3,5-Difluorophenyl)-3H-imidazo[4,5-b]pyridin-2-yl)propyl)-9H-purin-6-amine;
(S)-N-(1-(3-(3,5-Difluorophenyl)-3H-imidazo[4,5-b]pyridin-2-yl)ethyl)-9H-purin-6-amine;
(S)-N4-(1-(3-Phenyl-3H-imidazo[4,5-b]pyridin-2-yl)ethyl)pyrimidine-2,4,6-triamine;
(S)-N-(1-(5-Methoxy-3-phenyl-3H-imidazo[4,5-b]pyridin-2-yl)propyl)-9H-purin-6-amine;
(S)-N-(1-(3-(3,5-Difluorophenyl)-5-methoxy-3H-imidazo[4,5-b]pyridin-2-yl)ethyl)-9H-purin-6-amine;
(S)-N-(1-(3-(3,5-Difluorophenyl)-5-methoxy-3H-imidazo[4,5-b]pyridin-2-yl)propyl)-9H-purin-6-amine;
9-((5-Methyl-3-o-tolyl-3H-imidazo[4,5-b]pyridin-2-yl)methyl)-9H-purin-6-amine;
9-((5-Methoxy-3-phenyl-3H-imidazo[4,5-b]pyridin-2-yl)methyl)-9H-purin-6-amine;
(S)-4-Amino-6-(1-(3-phenyl-3H-imidazo[4,5-b]pyridin-2-yl)ethylamino)pyrimidine-5-carbonitrile;
(S)-N6-(1-(5-Methoxy-3-phenyl-3H-imidazo[4,5-b]pyridin-2-yl)ethyl)-9H-purine-2,6-diamine;
(S)-4-Amino-6-(1-(5-methoxy-3-phenyl-3H-imidazo[4,5-b]pyridin-2-yl)ethylamino)pyrimidine-5-carbonitrile;
(S)-N-(1-(5-Methoxy-3-phenyl-3H-imidazo[4,5-b]pyridin-2-yl)ethyl)pyrazolo[1,5-a]pyrimidin-7-amine;
2-(1-(9H-Purin-6-ylamino)ethyl)-3-phenyl-3H-imidazo[4,5-b]pyridin-5-ol;
(S)-N-(1-(5-Methyl-3-phenyl-3H-imidazo[4,5-b]pyridin-2-yl)ethyl)-9H-purin-6-amine;
(S)-4-Amino-6-(1-(5-methyl-3-phenyl-3H-imidazo[4,5-b]pyridin-2-yl)ethylamino)pyrimidine-5-carbonitrile;
(S)-N-(1-(5-Methyl-3-phenyl-3H-imidazo[4,5-b]pyridin-2-yl)ethyl)pyrazolo[1,5-a]pyrimidin-7-amine;
9-{[3-(2-Methylphenyl)-3H-imidazo[4,5-b]pyridin-2-yl]methyl}-9H-purin-6-amine;
6-{[(3-Phenyl-3H-imidazo[4,5-b]pyridin-2-yl)methyl]thio}-9H-purine;
6-({[3-(2-Methylphenyl)-3H-imidazo[4,5-b]pyridin-2-yl]methyl}thio)-9H-purine;
9-((3-(Piperidin-4-yl)-3H-imidazo[4,5-b]pyridin-2-yl)methyl)-9H-purin-6-amine;
9-((3-(1-Methylpiperidin-4-yl)-3H-imidazo[4,5-b]pyridin-2-yl)methyl)-9H-purin-6-amine;
9-((6-Amino-3-phenyl-3H-imidazo[4,5-b]pyridin-2-yl)methyl)-9H-purin-6-amine;
9-((6-Methyl-3-phenyl-3H-imidazo[4,5-b]pyridin-2-yl)methyl)-9H-purin-6-amine;
9-((5-Amino-3-phenyl-3H-imidazo[4,5-b]pyridin-2-yl)methyl)-9H-purin-6-amine;
7-((5-Amino-3-phenyl-3H-imidazo[4,5-b]pyridin-2-yl)methyl)-7H-purin-6-amine;
2-((9H-Purin-6-ylthio)methyl)-3-phenyl-3H-imidazo[4,5-b]pyridin-5-amine;
N-[(1S)-1-(6-Fluoro-3-phenyl-3H-imidazo[4,5-b]pyridin-2-yl)ethyl]-9H-purin-6-amine;
N-[(1S)-1-(6-Fluoro-3-phenyl-3H-imidazo[4,5-b]pyridin-2-yl)propyl]-9H-purin-6-amine;
N-{(1S)-1-[3-(2-Chlorophenyl)-3H-imidazo[4,5-b]pyridin-2-yl]ethyl}-9H-purin-6-amine;
N-{[3-(2-Chlorophenyl)-3H-imidazo[4,5-b]pyridin-2-yl]methyl}-9H-purin-6-amine;
9-((1-Phenyl-1H-imidazo[4,5-b]pyridin-2-yl)methyl)-9H-purin-6-amine;
6-((1-Phenyl-1H-imidazo[4,5-b]pyridin-2-yl)methylthio)-9H-purine;
(S)-N-(1-(1-Phenyl-1H-imidazo[4,5-b]pyridin-2-yl)ethyl)-9H-purin-6-amine;
(S)-N-(1-(3-(2,6-Difluorophenyl)-3H-imidazo[4,5-b]pyridin-2-yl)ethyl)-9H-purin-6-amine;
N-((1S)-1-(3-(2-Methoxyphenyl)-3H-imidazo[4,5-b]pyridin-2-yl)ethyl)-9H-purin-6-amine;
(S)-N-(1-(1-Phenyl-1H-imidazo[4,5-b]pyridin-2-yl)propyl)-9H-purin-6-amine;
N-{(1S)-1-[3-(3-chlorophenyl)-3H-imidazo[4,5-b]pyridin-2-yl]ethyl}-9H-purin-6-amine;
N-{(1S)-1-[3-(3-methoxyphenyl)-3H-imidazo[4,5-b]pyridin-2-yl]ethyl}-9H-purin-6-amine;
N-[(1S)-1-(3-pyridin-2-yl-3H-imidazo[4,5-b]pyridin-2-yl)ethyl]-9H-purin-6-amine;
N-[(1S)-1-(3-pyridin-3-yl-3H-imidazo[4,5-b]pyridin-2-yl)ethyl]-9H-purin-6-amine;
N-[(1S)-1-(3-cyclohexyl-3H-imidazo[4,5-b]pyridin-2-yl)ethyl]-9H-purin-6-amine;
N-[(1S)-1-(3-benzyl-3H-imidazo[4,5-b]pyridin-2-yl)ethyl]-9H-purin-6-amine;
N-{(1S)-1-[3-(3-fluorophenyl)-3H-imidazo[4,5-b]pyridin-2-yl]ethyl}-9H-purin-6-amine;
N-((1S)-1-{3-[3-(trifluoromethyl)phenyl]-3H-imidazo[4,5-b]pyridin-2-yl}ethyl)-9H-purin-6-amine;
N-{(1S)-1-[3-(3-methylphenyl)-3H-imidazo[4,5-b]pyridin-2-yl]ethyl}-9H-purin-6-amine;
4-{2-[(1S)-1-(9H-purin-6-ylamino)ethyl]-3H-imidazo[4,5-b]pyridin-3-yllpyridin-2(1H)-one;
N-{(1S)-1-[3-(1,3-thiazol-4-yl)-3H-imidazo[4,5-b]pyridin-2-yl]ethyl}-9H-purin-6-amine;
4-amino-6-({(1S)-1-[3-(3-chlorophenyl)-3H-imidazo[4,5-b]pyridin-2-yl]ethyl}amino)pyrimidine-5-carbonitrile;
4-amino-6-({(1S)-1-[3-(3-methoxyphenyl)-3H-imidazo[4,5-b]pyridin-2-yl]ethyl}amino)pyrimidine-5-carbonitrile;
4-amino-6-{[(1S)-1-(3-pyridin-2-yl-3H-imidazo[4,5-b]pyridin-2-yl)ethyl]amino}pyrimidine-5-carbonitrile;
4-amino-6-{[(1S)-1-(3-pyridin-3-yl-3H-imidazo[4,5-b]pyridin-2-yl)ethyl]amino}pyrimidine-5-carbonitrile;
4-amino-6-{[(1S)-1-(3-cyclohexyl-3H-imidazo[4,5-b]pyridin-2-yl)ethyl]amino}pyrimidine-5-carbonitrile;
4-amino-6-{[(1S)-1-(3-benzyl-3H-imidazo[4,5-b]pyridin-2-yl)ethyl]amino}pyrimidine-5-carbonitrile;
4-amino-6-({(1S)-1-[3-(3-fluorophenyl)-3H-imidazo[4,5-b]pyridin-2-yl]ethyl}amino)pyrimidine-5-carbonitrile;
4-amino-6-[((1S)-1-{3-[3-(trifluoromethyl)phenyl]-3H-imidazo[4,5-b]pyridin-2-yl}ethyl)amino]pyrimidine-5-carbonitrile;
4-amino-6-({(1S)-1-[3-(3-methylphenyl)-3H-imidazo[4,5-b]pyridin-2-yl]ethyl}amino)pyrimidine-5-carbonitrile;
4-amino-6-({(1S)-1-[3-(2-oxo-1,2-dihydropyridin-4-yl)-3H-imidazo[4,5-b]pyridin-2-yl]ethyl}amino)pyrimidine-5-carbonitrile;
4-amino-6-({(1S)-1-[3-(1,3-thiazol-4-yl)-3H-imidazo[4,5-b]pyridin-2-yl]ethyl}amino)pyrimidine-5-carbonitrile;
N-{(1S)-1-[5-(methylamino)-3-phenyl-3H-imidazo[4,5-b]pyridin-2-yl]ethyl}-9H-purin-6-amine;
4-amino-6-({(1S)-1-[5-(methylamino)-3-phenyl-3H-imidazo[4,5-b]pyridin-2-yl]ethyl}amino)pyrimidine-5-carbonitrile;
N-[(1S)-1-(5-ethoxy-3-phenyl-3H-imidazo[4,5-b]pyridin-2-yl)ethyl]-9H-purin-6-amine;
4-amino-6-{[(1S)-1-(5-ethoxy-3-phenyl-3H-imidazo[4,5-b]pyridin-2-yl)ethyl]amino}pyrimidine-5-carbonitrile;
N-[(1S)-1-(6-fluoro-5-methoxy-3-phenyl-3H-imidazo[4,5-b]pyridin-2-yl)ethyl]-9H-purin-6-amine;
4-amino-6-{[(1S)-1-(6-fluoro-5-methoxy-3-phenyl-3H-imidazo[4,5-b]pyridin-2-yl)ethyl]amino}pyrimidine-5-carbonitrile;
N-[(1 S)-1-(5-methoxy-3-phenyl-3H-imidazo[4,5-b]pyridin-2-yl)ethyl]-N-methyl-9H-purin-6-amine;
4-amino-6-[[(1S)-1-(5-methoxy-3-phenyl-3H-imidazo[4,5-b]pyridin-2-yl)ethyl](methyl)amino]pyrimidine-5-carbonitrile;
N-[(1S)-1-(7-methyl-3-phenyl-3H-imidazo[4,5-b]pyridin-2-yl)ethyl]-9H-purin-6-amine;
4-amino-6-{[(1S)-1-(7-methyl-3-phenyl-3H-imidazo[4,5-b]pyridin-2-yl)ethyl]amino}pyrimidine-5-carbonitrile;
or a pharmaceutically acceptable salt, or solvate, or N-oxide, or stereoisomer or deuterated derivative thereof.

Examples of the preferred compounds are:
(S)-N6-(1-(3-Phenyl-3H-imidazo[4,5-b]pyridin-2-yl)ethyl)-9H-purine-2,6-diamine;
(S)-N-(1-(5-Methoxy-3-phenyl-3H-imidazo[4,5-b]pyridin-2-yl)ethyl)-9H-purin-6-amine;
(S)-N-(1-(5-Methoxy-3-phenyl-3H-imidazo[4,5-b]pyridin-2-yl)propyl)-9H-purin-6-amine;
(S)-N-(1-(3-(3,5-Difluorophenyl)-5-methoxy-3H-imidazo[4,5-b]pyridin-2-yl)ethyl)-9H-purin-6-amine;
(S)-N-(1-(3-(3,5-Difluorophenyl)-5-methoxy-3H-imidazo[4,5-b]pyridin-2-yl)propyl)-9H-purin-6-amine;
(S)-N6-(1-(5-Methoxy-3-phenyl-3H-imidazo[4,5-b]pyridin-2-yl)ethyl)-9H-purine-2,6-diamine;
(S)-4-Amino-6-(1-(5-methoxy-3-phenyl-3H-imidazo[4,5-b]pyridin-2-yl)ethylamino)pyrimidine-5-carbonitrile;
(S)-N-(1-(5-Methoxy-3-phenyl-3H-imidazo[4,5-b]pyridin-2-yl)ethyl)pyrazolo[1,5-a]pyrimidin-7-amine;
2-(1-(9H-Purin-6-ylamino)ethyl)-3-phenyl-3H-imidazo[4,5-b]pyridin-5-ol;
(S)-N-(1-(5-Methyl-3-phenyl-3H-imidazo[4,5-b]pyridin-2-yl)ethyl)-9H-purin-6-amine;
(S)-4-Amino-6-(1-(5-methyl-3-phenyl-3H-imidazo[4,5-b]pyridin-2-yl)ethylamino)pyrimidine-5-carbonitrile;
or a pharmaceutically acceptable salt, or solvate, or N-oxide, or stereoisomer or deuterated derivative thereof.

The compounds of the invention can be prepared using the methods and procedures described herein, or using similar methods and procedures. It will be appreciated that where typical or preferred process conditions (i.e., reaction temperatures, times, mole ratios of reactants, solvents, pressures, etc.) are given; other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group, as well as suitable conditions for protection and deprotection, are well known in the art. For example, numerous protecting groups, and their introduction and removal are described in T. W. Greene and G. M. Wuts, Protecting Groups in Organic Synthesis, Third Edition, Wiley, New York, 1999, and references cited therein.

The term amino-protecting group refers to a protecting group suitable for preventing undesired reactions at amino nitrogen. Representative amino-protecting groups include, but are not limited to, formyl; acyl groups, for example alkanoyl groups such as acetyl; alkoxycarbonyl groups such as tert-butoxycarbonyl (Boc); arylmethoxycarbonyl groups such as benzyloxycarbonyl (Cbz) and 9-fluorenylmethoxycarbonyl (Fmoc); arylmethyl groups such as benzyl (Bn), trityl (Tr), and 1,1-di-(4'-methoxyphenyl)methyl; silyl groups such as trimethylsilyl (TMS) and tert-butyldimethylsilyl (TBS); and the like.

The term hydroxy-protecting group refers to a protecting group suitable for preventing undesired reactions at a hydroxy group. Representative hydroxy-protecting groups include, but are not limited to, alkyl groups, such as methyl, ethyl, and tert-butyl; acyl groups, for example alkanoyl groups, such as acetyl; arylmethyl groups, such as benzyl (Bn), p-methoxybenzyl (PMB), 9-fluorenylmethyl (Fm), and diphenylmethyl (benzhydryl, DPM); silyl groups, such as trimethylsilyl (TMS) and tert-butyldimethylsilyl (TBS); and the like.

According to one embodiment of the present invention, compounds of general Formula (I) may be prepared by the synthetic route illustrated in Scheme 1.

For the avoidance of doubt, the group 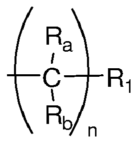 has been represented as -[C(Rₐ)R_{b})]ₙ-R₁ in the Schemes depicted herebelow.

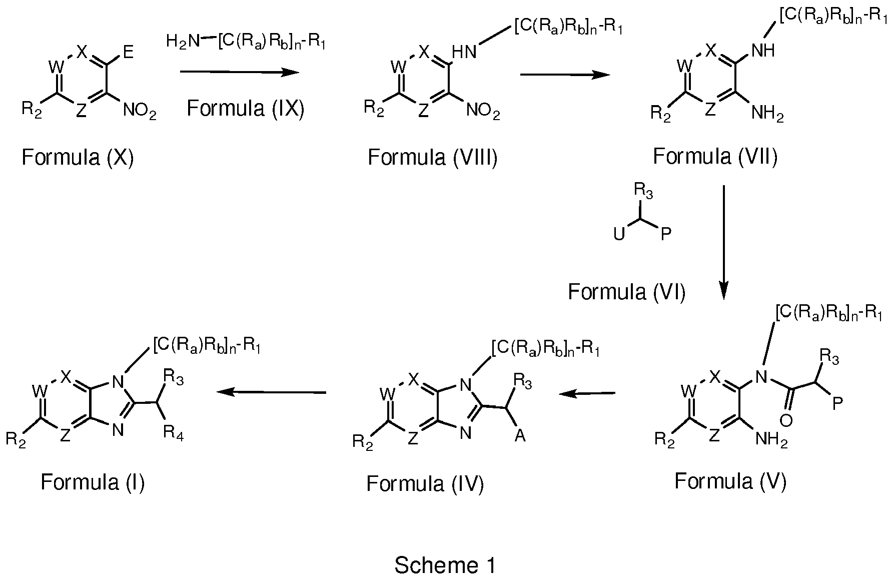

Compounds of formula (I) may be obtained from heteroaromatic compounds of formula (IV). In a first case, A represents an halogen atom such as chlorine, bromine and iodine or another suitable leaving group such as methanesulfonate or trifluoromethanesulfonate or other groups such as hydroxyl, that can be converted to suitable leaving groups by standard methods described in the literature, such as the Mitsunobu reaction and others. Compounds of Formula (I) can be obtained directly from compounds of Formula (IV) by treatment of (IV) with compounds of formula R₄, where R₄ represents a group selected from:
i) a group of formula (IIa) or (IIa') 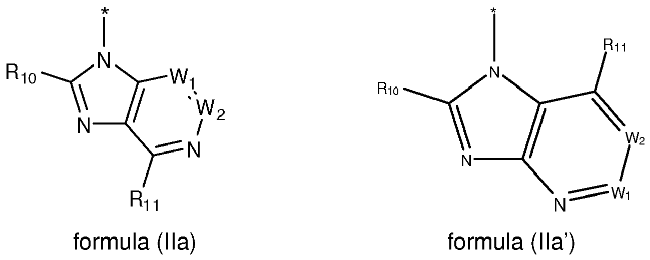
ii) a group of formula (IIb) 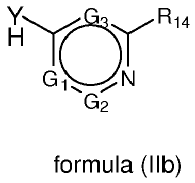 and
iii) a group of formula (IIc) 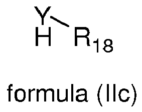 wherein
   Y represents a linker selected from a -NR'- group, -O- or -S-; wherein R' is a hydrogen atom, a hydroxy group, a C₁-C₄ alkoxy group or a linear or branched C₁-C₄ alkyl group.

As depicted in Scheme 2, compound of Formula (I) can be obtained from compounds of Formula (IV) by reaction with compounds of formula R₄, in the presence of a suitable base such as potassium carbonate, diisopropylethylamine or sodium hydride in an appropriate solvent such as *tert*-butanol, *N,N*-dimethylformamide or tetrahydrofurane at temperatures ranging from room temperature to 160 ºC, with or without the use of microwaves irradiation.

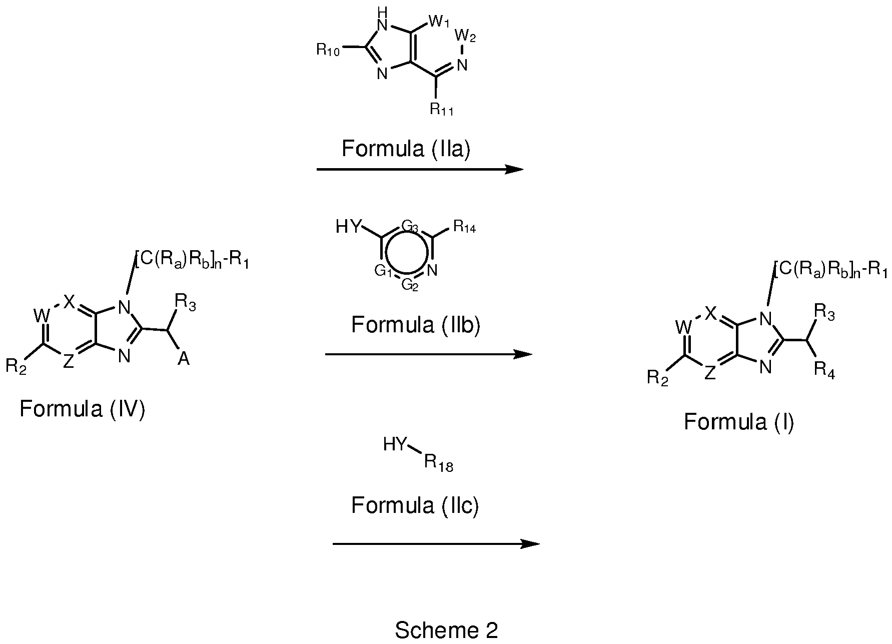

Alternatively, as depicted in Scheme 3, in the case where A represents a -NR'- group, -O- or -S-, wherein R' is as defined above; and W, X, Z, R₁, R₂ and R₃ are as previously defined, compounds of Formula (I) may be prepared by reaction of compounds of Formula (IV) with a compound of formula R₅, where L represents a leaving group such as an halogen atom such as chlorine, bromine and iodine or another suitable leaving group such as methanesulfonate or trifluoromethanesulfonate or other groups such as hydroxyl, that can be converted to suitable leaving groups by standard methods described in the literature, such as the Mitsunobu reaction and others. Compounds of Formula (IV) can be treated with electrophiles of formula R₅ in the presence of a suitable base such as potassium carbonate, diisopropylethylamine or sodium hydride in an appropriate solvent such as *tert*-butanol, *N,N*-dimethylformamide or tetrahydrofurane at temperatures ranging from room temperature to 220 ºC, with or without the use of microwaves irradiation.

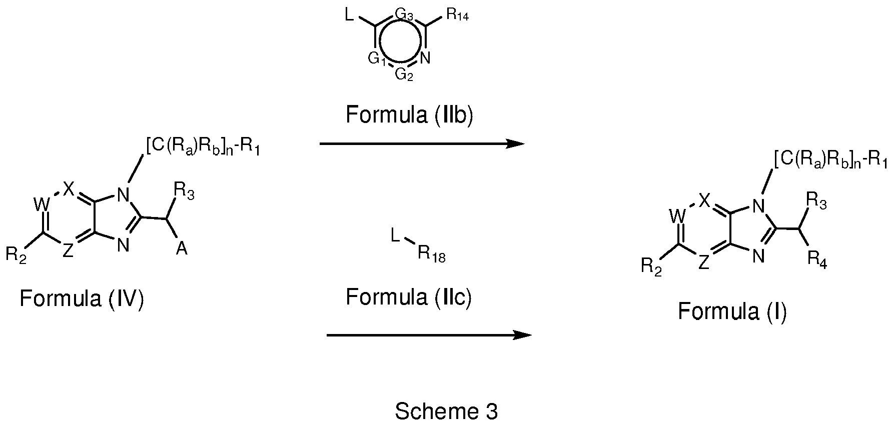

Compounds of formula (IV) may be prepared by treatment of compounds of formula (V), where P is a chlorine atom or an amine bearing a protective group (such as acetyl or tert-butoxy carbonyl) and W, X, Z, R₁, R₂ and R₃ as previously defined, with a suitable cyclization agent, for example acetic acid, diluted hydrogen chloride or p-toluensulfonic acid at temperatures ranging from room temperature to 130 ºC in a solvent such as glacial acetic acid, dichloromethane or diluted chlorohydric acid with or without the use of microwave irradiation. In the case of P being an amine moiety functionalized with an appropriate protecting group such as *tert*-butoxycarbonyl (BOC) or benzyloxycarbonyl (CBZ), once the cyclization is complete may be deprotected at the amine moiety under standard conditions (Greene's Protective Groups in Organic Synthesis, ISBN: 0471697540), for example by using an acidic agent such as aqueous hydrogen chloride or trifluoroacetic acid.

Compounds of formula (V) may be obtained directly from compounds of formula (VI), where P is a chlorine atom or an amine bearing a protective group (such as acetyl or tert-butoxy carbonyl) and U can be a carboxylic acid, an acetyl chloride or a 1,1,1-trimethoxymethyl moiety. When U is a carboxylic acid, compound of formula (V) can be obtained by treatment with an appropriate activating agent such as benzotriazolyloxy-tris-(dimethylamino) phosphonium hexafluorophosphate in the presence of a suitable base such as 1,8-diazabiciclo[5.4.0]undec-7-ene at temperatures ranging from 25 to 80 ºC in a suitable solvent such as *N,N'*-dimethylformamide in the presence of a nucleophile of formula (VII) following the protocol as described in the literature (J. Org. Chem., 2007, 72 (26),10194-10210). In the case of U being an acetyl chloride, compound of formula (V) can be obtained in the presence of a nucleophile of formula (VII) by heating at 130 ºC in a solvent such as acetic acid. Alternatively, when U is a 1,1,1-trimethoxymethyl moiety, compound of formula (V) can be obtained at room temperature in a solvent such as dichloromethane in the presence of p-toluensulfonic acid monohydrate. U being an acetyl chloride or a 1,1,1-trimethoxymethyl moiety, in both cases compounds of formula (IV) are directly formed from compounds of formula (VII) without isolating the compounds of formula (V).

Compounds of general formula (VII) may be obtained from compounds (VIII) in which the nitro group may undergo further reaction with hydrogen gas at atmospheric pressure using a suitable catalyst such as palladium or platinum on carbon in a solvent such as ethanol or methanol at ambient temperature to furnish an amino group. Alternatively, this nitro group may be reduced in the presence of other reducing agents, such as iron, tin(II) chloride dihydrate or sodium sulfide nonahydrate, under acidic conditions, furnished by aqueous hydrogen chloride or ammonium chloride, in solvents like ethanol, methanol, acetone, tetrahydrofurane or water at temperatures ranging from room temperature to 80 ºC.

Compounds of general formula (VII) may also be obtained as described in Scheme 4. 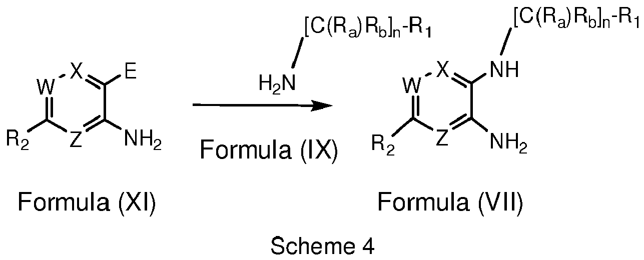 Aryl amines of formula (XI), where E is chlorine or bromine and W, X, Z and R₂ have been previously defined, may be reacted with amines of formula (IX) (where R₁ has been previously defined) to give compounds of formula (VII). Such reactions may be carried out in a solvent such as diethylene glycol or toluene at temperatures ranging from 80 to 180 ºC with or without the use of microwave irradiation, in the presence of aqueous bromhydric acid under acidic conditions or, alternatively, in the presence of tris(dibenzylideneacetone)dipalladium(0) and 2,2'-bis(diphenylphosphino)-1,1'-binaphthyl with sodium tert-butoxyde acting as a base.

Nitro intermediates of general formula (VIII) may be prepared from starting compounds of formula (X) by replacement of the leaving group E, such as chlorine of bromine, with an amine of general formula (IX), as previously defined. This substitution takes place at temperatures ranging from 80 to 150ºC with or without the use of microwave irradiation and was performed in the absence of solvents or using solvents such as ethanol, dimethylsulfoxide or dimethylformamide. An excess of the amine of formula (IX) can acts as a base or, alternatively, introducing other bases such as potassium carbonate or triethylamine.

### EXAMPLES

### General

The syntheses of the compounds of the invention and of the intermediates for use therein are illustrated by the following Examples (1-78) (including Preparation Examples (Preparations 1-81)) are given in order to provide a person skilled in the art with a sufficiently clear and complete explanation of the present invention, but should not be considered as limiting of the essential aspects of its subject, as set out in the preceding portions of this description.

Reagents, starting materials, and solvents were purchased from commercial suppliers and used as received. Concentration or evaporation refers to evaporation under vacuum using a Büchi rotatory evaporator.

Reaction products were purified, when necessary, by flash chromatography on silica gel (40-63 µm) with the solvent system indicated. Purifications in reverse phase were made in a Biotage SP1^{®} automated purification system equipped with a C₁₈ column and using a gradient of water-acetonitrile/MeOH (1:1) (0.1 % v/v ammonium formate both phases) from 0% to 100% acetonitrile/MeOH (1:1) in 40 column volumes. The appropriate fractions were collected and the solvents evaporated under reduced pressure and/or liofilized.

Preparative HPLC-MS were performed on a Waters instrument equipped with a 2767 injector/collector, a 2525 binary gradient pump, a 2996 PDA detector, a 515 pump as a make-up pump and a ZQ4000 Mass spectrometer detector or on a Agilent 1200 Series coupled to an Agilent 6120 Mass spectrometer detector. Both systems were equipped with a Symmetry Prep C₁₈ (19 x 300 mm, 7 µm) column or a XBridge Prep C₁₈ (19 x 100 mm, 5 µm) column. The mobile phase was formic acid (0.4 mL), ammonia (0.1 mL), methanol (500 mL) and acetonitrile (500 mL) (B) and formic acid (0.5 mL), ammonia (0.125 mL) and water (1000 mL) (A), the specific gradients used are specified in each particular case. The flow rate was 20 mL/min.

Purity and MS identification was performed in a Waters 2795 system coupled to a 2996 Diode array detector and to a Waters ZQ mass spectrometer detector or in a Waters Acquity UPLC system coupled to a SQD mass spectrometer detector. The injection volume was 5 microliter on the HPLC and 0.5 microliter on the UPLC. Chromatograms were processed at 210 nM or 254 nM. Mass spectra of the chromatograms were acquired using positive and negative electrospray ionization. The mobile phase was formic acid (0.4 mL), ammonia (0.1 mL), methanol (500 mL) and acetonitrile (500 mL) (B) and formic acid (0.5 mL), ammonia (0.125 mL) and water (1000 mL) (A) and a gradient between 0 to 95% of B was used. Columns: HPLC: Waters Symmetry (2.1x50mm, 3.5 □m); UPLC: ACQUITY UPLC BEH C-18 (2.1x50mm, 1.7 □m)

¹H Nuclear Magnetic Resonance Spectra were recorded on a Varian Gemini-2000 spectrometer operating at a frequency of 300 MHz for the ¹H spectra or in a Varian Mercury plus operating at a frequency of 400 MHz for the ¹H spectra. Samples were dissolved in the specified deuterated solvent. Tetramethylsilane was used as reference.

### Abbreviations:

- DMF: Dimethylformamide
- DMSO: Dimethylsulfoxide
- CDCl₃: Deuterated chloroform
- NMR: Nuclear magnetic resonance
- s: Singlet
- d: Doublet
- dd: Doublet doublet
- td: Triple doublet
- br: Broad
- q: Quarted
- t: Triplet
- m: Multiplet
- LRMS: Low resolution mass spectrometry
- h: hour
- min: minutes
- DMF: *N*,*N*-dimethylformamide
- DCM: dichloromethane, methylene chloride
- AcOEt: ethyl acetate
- DMSO: dimethylsufoxide
- EDC-HCl: 3-((ethylimino)methyleneamino)-*N*,*N*-dimethylpropan-1-aminium chloride
- THF: tetrahydrofurane
- DIEA: diisopropylethyamine
- HOBt: 1-Hydroxybenzotriazole hydrate
- MeOH: methanol
- DPPONH₂: *P*,*P*-diphenylphosphinic amide
- PPTS: pyridinium *p*-toluenesulphonate
- Pd(PPh₃)₄: Tetrakis(triphenylphosphane) palladium(0)
- HMPA: hexamethylphosphoramide
- Celite®: diatomaceous earth

### PREPARATION 1

### 3-Nitro-N-phenylpyridin-2-amine

A stirred mixture of 2-chloro-3-nitropyridine (15.00 g, 90.00 mmols) and aniline (17.3 mL, 190.00 mmols) in absolute ethanol (37.0 mL) was heated in the microwave reactor for 10 min at 150ºC. The precipitated solid was filtered, washed with ethanol and dried. The expected product was obtained (15.35 g, 76%).
LRMS (m/z): 216 (M+1)⁺
1H NMR (400 MHz, DMSO-d₆) d ppm 7.00 (dd, 1 H) 7.08 - 7.25 (m, 1 H) 7.38 (t, 2 H) 7.66 (d, 2 H) 8.29 - 8.66 (m, 2 H) 9.97 (s, 1 H)

### PREPARATION 2

### N²-Phenylpyridine-2,3-diamine

A mixture of 3-nitro-N-phenylpyridin-2-amine (15.31 g, 70.00 mmols, Preparation 1) and Pd/C 10% (2.35 g, 20.00 mmols) in ethanol (500 mL) was stirred under an atmosphere of hydrogen (ca.1 atm) for 1h, then filtered through celite and concentrated in vacuo. The expected compound was obtained (13.10 g, 99%).
LRMS (m/z): 186 (M+1)⁺
1H NMR (400 MHz, DMSO-d₆) d ppm 5.07 (s, 2 H) 6.62 (dd, 1 H) 6.74 - 7.00 (m, 2 H) 7.10 - 7.33 (m, 2 H) 7.42 - 7.53 (m, 1 H) 7.62 (d, 2 H) 7.71 (s, 1 H)

### PREPARATION 3

### (S)-tert-Butyl 1-((3-aminopyridin-2-yl)(phenyl)amino)-1-oxopropan-2-ylcarbamate

A mixture of N²-phenylpyridine-2,3-diamine (13.10g, 70.00 mmols, Preparation 2), (S)-2-(tert-butoxycarbonylamino)propanoic acid (13.50g, 70.00 mmols), N-(3-dimethylaminopropyl)-N'-ethylcarbodiimide hydrochloride (EDC.HCl, 16.30g, 90.00 mmols), 1-hydroxybenzotriazole hydrate (HOBt, 10.00g, 70.00 mmols) and N-methylmorpholine (NMM, 32.0 mL, 290.00 mmols) in dry N,N-dimethylformamide (DMF, 130 mL) was stirred at room temperature for 3 days.
It was poured into a mixture of with water/brine (200 mL/200 mL) and extracted with ethyl acetate. The combined organic layers were washed with saturated NaHCO₃ (4%), water and brine and were dried, filtered and concentrated in vacuo to afford the expected product (17.24 g, 48%). The sample was considered approx. 70% pure. It was used in the next reaction step without further purification.
LRMS (m/z): 357 (M+1)⁺.

### PREPARATION 4

### (S)-1-(3-Phenyl-3H-imidazo[4,5-b]pyridin-2-yl)ethanamine

A solution of (S)-tert-butyl 1-((3-aminopyridin-2-yl)(phenyl)amino)-1-oxopropan-2-ylcarbamate (17.24 g, 33.86 mmols, Preparation 3) in glacial acetic acid (80.0 mL) was stirred at 130ºC overnight. The HPLC-MS of the crude shows a main peak with a mass corresponding to the expected product but acetylated (M+1: 281). It was concentrated in vacuo and the residue obtained was hydrolysed by dissolving the crude in methanol (225 mL), adding a 5N HCl solution (225 mL) and stirring the mixture at 80ºC for 4.5h.lt was concentrated in vacuo. The resulting aqueous solution was washed with dichloromethane (2X). It was neutralised with a 2N NaOH solution and washed again with dicloromethane (2X). A saturated potassium carbonate solution was added (to reach pH 10-12) and it was extracted with ethyl acetate (3X). The organic phase was dried, filtered and concentrated in vacuo to afford the expected product (4.82 g, 60%).
LRMS (m/z): 239 (M+1)⁺
1H NMR (400 MHz, DMSO-d₆) d ppm 1.34 (d, 3 H) 2.00 - 2.17 (s, 2 H) 4.04 (q, 1 H) 7.30 (dd, 1 H) 7.53 - 7.67 (m, 5 H) 8.04 - 8.12 (m, 1 H) 8.20 - 8.25 (m, 1 H)

### PREPARATION 5

### 2-(Chloromethyl)-3-phenyl-3H-imidazo[4,5-b]pyridine

2-Chloroacetyl chloride (0.5 mL, 5.94 mmols) was added to a solution of N²-phenylpyridine-2,3-diamine (1.00g, 5.40 mmols, Preparation 2) in glacial acetic acid (AcOH, 62 mL). It was stirred at 130ºC overnight. The reaction mixture was allowed to reach room temperature and it was concentrated in vacuo. The residue obtained was dissolved in ethylacetate and it was washed with a 4% aqueous solution of NaHCO₃. The organic phase was washed with water and brine, dried filtered and concentrated in vacuo. The mass of the expected compound was detected (HPLC-MS) in the main peak (approx.54%) of the crude chromatogram. This crude (1.31 g, 54%) was used in the next reaction steps without further purification.
LRMS (m/z): 244 (M+1)⁺.

### PREPARATION 6

### 2-(1-Chloroethyl)-3-phenyl-3H-imidazo[4,5-b]pyridine

2-Chloropropanoyl chloride (0.3 mL, 2.99 mmols) was added to a solution of N²-phenylpyridine-2,3-diamine (0.50 g, 2.70 mmols, Preparation 2) in glacial acetic acid (AcOH) (30 mL). It was stirred at 130ºC overnight. The reaction mixture was allowed to reach room temperature and it was concentrated in vacuo. The residue obtained was dissolved in ethylacetate and it washed with a 4% aqueous solution of NaHCO₃, water and brine. It was dried, filtered and concentrated in vacuo. The mass of the expected compound was detected (HPLC-MS) in the main peak (approx.60%) of the crude chromatogram. This crude (0.65 g, 56%) was used in the next reaction step without further purification.
LRMS (m/z): 258 (M+1)⁺

### PREPARATION 7

### (S)-tert-Butyl 1-((3-aminopyridin-2-yl)(phenyl)amino)-1-oxobutan-2-ylcarbamate

A mixture of N²-phenylpyridine-2,3-diamine (0.50g, 2.70 mmols, Preparation 2), (S)-2-(tert-butoxycarbonylamino)butanoic acid (0.55g, 2.71 mmols), EDC.HCl (0.62g, 3.23 mmols), 1-hydroxy-7-azabenzotriazole (HOAt, 0.37g, 2.68 mmols) and NMM (1.2mL, 10.79 mmols) in dry DMF (5 mL) was stirred at room temperature overnight. The mixture was diluted with water and extracted with ethyl acetate. The combined organic layers were washed with saturated NaHCO₃ (4%) and brine and were dried, filtered and concentrated in vacuo to afford the expected product (1.22 g) according to HPLC-MS (traces of DMF were detected in the sample). It was used in the next reaction step without further purification.
LRMS (m/z): 371 (M+1)⁺

### PREPARATION 8

### (S)-1-(3-Phenyl-3H-imidazo[4,5-b]pyridin-2-yl)propan-1-amine

A solution of (S)-tert-butyl 1-((3-aminopyridin-2-yl)(phenyl)amino)-1-oxobutan-2-ylcarbamate (1.22g, 3.29 mmols, Preparation 7) in glacial acetic acid (7.5mL) was stirred at 130ºC overnight. The HPLC-MS of the crude shows a main peak with a mass corresponding to the expected product but acetylated (M+1: 295). It was concentrated in vacuo and the residue obtained was hydrolysed by dissolving the crude in methanol (15 mL), adding a 5N HCl solution (15 mL) and stirring the mixture at 80ºC for 4.5h. It was concentrated in vacuo. The resulting aqueous solution was neutralised with a 2N NaOH solution and thereafter extracted with dichloromethane. The organic layer was dried, filtered and concentrated in vacuo. The expected compound was not detected in the organic phase residue but in the aqueous phase. It was lyophilised and this residue was suspended in a saturated potassium carbonate solution and extracted with ethyl acetate. The organic phase was dried, filtered and concentrated in vacuo to afford the expected product (0.38 g, 46%).
LRMS (m/z): 253 (M+1)⁺
1H NMR (400 MHz, DMSO-*d*₆) d ppm 0.75 (t, 3 H) 1.52 - 1.70 (m, 1 H) 1.71 - 1.90 (m, 1 H) 2.05 (s, 2 H) 3.77 (t, 1 H) 7.31 (dd, 1 H) 7.46 - 7.69 (m, 5 H) 8.10 (dd, 1 H) 8.16 - 8.29 (m, 1 H)

### PREPARATION 9

### (S)-tert-Butyl 2-((3-aminopyridin-2-yl)(phenyl)carbamoyl)pyrrolidine-1-carboxylate

A mixture of N²-phenylpyridine-2,3-diamine (2.00 g, 10.80 mmols, Preparation 2), (S)-1-(tert-butoxycarbonyl)pyrrolidine-2-carboxylic acid (2.35 g, 10.92 mmols), EDC.HCl (2,50 g, 13.04 mmols), HOAt (1.50 g, 11.02 mmols) and NMM (4.80 mL, 43.52 mmols) in dry DMF (20mL) was stirred at room temperature overnight. The mixture was diluted with water and extracted with ethyl acetate. The combined organic layers were washed with saturated NaHCO₃ (4%), brine and were dried, filtered and concentrated in vacuo to afford the expected product (4.10 g, 50%). The sample was considered approx. 50% pure (HPLC-MS). It was used in the next reaction step without further purification.
LRMS (m/z): 383 (M+1)⁺

### PREPARATION 10

### (S)-3-Phenyl-2-(pyrrolidin-2-yl)-3H-imidazo[4,5-b]pyridine

A solution of (S)-tert-butyl 2-((3-aminopyridin-2-yl)(phenyl)carbamoyl)pyrrolidine-1-carboxylate (4.10 g, 5.36 mmols, Preparation 9) in glacial acetic acid (35 mL) was stirred at 130ºC overnight. The HPLC-MS of the crude shows a main peak with a mass corresponding to the expected product but acetylated (M+1=307). It was concentrated in vacuo and the residue obtained was hydrolysed by dissolving the crude in methanol (70 mL), adding a 5N HCl solution (70 mL) and stirring the mixture at 80ºC for 4.5h. It was concentrated in vacuo. The resulting aqueous solution was washed with dichloromethane (2X). It was neutralised with a 2N NaOH solution and washed again with dichloromethane (2X). A saturated potassium carbonate solution was added (to reach pH 10-12) and it was extracted with ethyl acetate (3X). The organic phase was dried, filtered and concentrated in vacuo to afford the expected product (0.34g, 24%).
LRMS (m/z): 265 (M+1)⁺
1H NMR (400 MHz, DMSO-*d*₆) d ppm 1.57 - 1.75 (m, 1 H) 1.76 - 1.97 (m, 2 H) 2.07 (dd, 1 H) 2.69 - 2.85 (m, 1 H) 2.88 - 3.07 (m, 1 H) 4.11 - 4.28 (m, 1 H) 7.31 (dd, 1 H) 7.50 - 7.68 (m, 5 H) 8.02 - 8.14 (m, 1 H) 8.16 - 8.31 (m, 1 H)

### PREPARATION 11

### 6-Methoxy-3-nitro-N-phenylpyridin-2-amine

Aniline (6.9 mL, 80.00 mmols) was added to a solution of 2-chloro-6-methoxy-3-nitropyridine (11.00 g, 60.00 mmols) in dry DMF and it was stirred in the microwave at 140ºC for 1 h. It was concentrated in vacuo. Water and ethyl acetate were added. The organic phase was dried, filtered and concentrated in vacuo. It was purified by chromatography (Silica gel, Hexane:ethyl acetate 20:1) to afford the expected compound (1 0.46g, 73%).
LRMS (m/z): 246 (M+1)⁺
1H NMR (400 MHz, DMSO-*d*₆) d ppm 3.90 (s, 3 H) 6.38 (d, 1 H) 7.11 - 7.22 (m, 1 H) 7.27 - 7.48 (m, 2 H) 7.72 (d, 2 H) 8.45 (d, 1 H) 10.46 (s, 1 H)

### PREPARATION 12

### 6-Methoxy-N²-phenylpyridine-2,3-diamine

Same procedure as described in Preparation 2 was used from 6-methoxy-3-nitro-N-phenylpyridin-2-amine (10.46 g, 42.65 mmols, Preparation 11) stirring the mixture at room temperature for 6h. The expected compound was obtained (9.09g, 91%).
LRMS (m/z): 216 (M+1)⁺
1H NMR (400 MHz, DMSO-*d*₆) d ppm 3.78 (s, 3 H) 4.51 (s, 2 H) 6.07 (d, 1 H) 6.76 - 6.89 (m, 1 H) 6.98 (d, 1 H) 7.14 - 7.33 (m, 2 H) 7.65 (d, 2 H) 7.72 (s, 1 H)

### PREPARATION 13

### (S)-tert-Butyl 1-((3-amino-6-methoxypyridin-2-yl)(phenyl)amino)-1-oxopropan-2-ylcarbamate

Same procedure as described in Preparation 3 was used from 6-methoxy-N²-phenylpyridine-2,3-diamine (3.00 g, 13.94 mmols, Preparation 12). The expected compound was obtained (5.46 g, 97%).
LRMS (m/z): 387 (M+1)⁺
1H NMR (400 MHz, DMSO-*d*₆) d ppm 1.28 (d, 3 H) 1.41 (s, 9 H) 3.80 (s, 3 H) 3.93 - 4.15 (m, 1 H) 6.20 (d, 1 H) 6.75 - 7.02 (m, 1 H) 7.14 - 7.45 (m, 4 H) 7.62 - 7.94 (m, 3 H) 9.44 (s, 1 H)

### PREPARATION 14

### (S)-1-(5-Methoxy-3-phenyl-3H-imidazo[4,5-b]pyridin-2-yl)ethanamine

Same procedure as described in Preparation 4 was used from (S)-tert-butyl 1-((3-amino-6-methoxypyridin-2-yl)(phenyl)amino)-1-oxopropan-2-ylcarbamate (5.38 g, 13.92 mmols, Preparation 13). The expected compound was obtained (3.14 g, 84%).
LRMS (m/z): 269 (M+1)⁺
1H-NMR (400 MHz, DMSO-*d*₆) d ppm 1.28 (d, 3 H) 2.05 (s, 2H) 3.70 (s, 3H) 4.00 (q, 1H) 6.70 (d, 1H) 7.50 - 7.75 (m, 5H) 8.00 (d, 1H)

### PREPARATION 15

### N-(3,5-Difluorophenyl)-3-nitropyridin-2-amine

Same procedure as described in Preparation 1 was used from 3,5-difluoroaniline (22.00 g, 170.00 mmols). The expected compound was obtained (21.10 g, 88%).
LRMS (m/z): 252 (M+1)⁺
1H NMR (400 MHz, DMSO-*d*₆) d ppm 6.84 - 7.02 (m, 1 H) 7.12 (dd, 1 H) 7.57 (m, 2 H) 8.47 - 8.73 (m, 2 H) 10.04 (s, 1 H)

### PREPARATION 16

### N²-(3,5-Difluorophenyl)pyridine-2,3-diamine

Same procedure as described in Preparation 2 was used from N-(3,5-difluorophenyl)-3-nitropyridin-2-amine (21.10 g, 80.00 mmols, Preparation 15). The expected compound was obtained (16.78 g, 90%).
LRMS (m/z): 222 (M+1)⁺
1H NMR (400 MHz, DMSO-*d*₆) d ppm 5.16 (s, 2 H) 6.43 - 6.67 (m, 1 H) 6.73 (dd, 1 H) 6.97 (dd, 1 H) 7.22 - 7.45 (m, 2 H) 7.57 (dd, 1 H) 8.23 (s, 1 H)

### PREPARATION 17

### (S)-tert-Butyl 1-((3-aminopyridin-2-yl)(3,5-difluorophenyl)amino)-1-oxobutan-2-ylcarbamate

Same procedure as described in Preparation 3 was used from N²-(3,5-difluorophenyl)pyridine-2,3-diamine (4.00 g, 18.08 mmols, Preparation 16) and (S)-2-(tert-butoxycarbonylamino)butanoic acid (3.68 g, 18.11 mmols). After 36h 0.5 equivalents more of all reactants were added in this case and it was stirred at room temperature for 24h more. The expected compound was obtained (6.98 g, 34%).
LRMS (m/z): 407 (M+1)⁺
1H NMR (400 MHz, DMSO-*d*₆) d ppm 0.96 (t, 3 H) 1.42 (s, 9 H) 1.59 - 1.86 (m, 2 H) 4.00 (m, 1 H) 6.60 (m, 1H) 6.65 - 6.80 (m, 1 H) 6.88 - 7.07 (m, 1 H) 7.38 (d, 1 H) 7.51 - 7.70 (m, 2 H) 8.05 - 8.32 (m, 2 H) 9.80 (s, 1H)

### PREPARATION 18

### (S)-1-(3-(3,5-Difluorophenyl)-3H-imidazo[4,5-b]pyridin-2-yl)propan-1-amine

Same procedure as described in Preparation 4 was used from (S)-tert-butyl 1-((3-aminopyridin-2-yl)(3,5-difluorophenyl)amino)-1-oxobutan-2-ylcarbamate (6.98 g, 6.18 mmols, Preparation 17). The expected compound was obtained (0.41 g, 22%).
LRMS (m/z): 289 (M+1)⁺
1H NMR (400 MHz, DMSO-d₆) d ppm 0.75 (t, 3 H) 1.60 - 1.75 (m, 1 H) 1.80 - 1.90 (m, 1 H) 2.10 (s, 2 H) 3.80 (t, 1 H) 7.30 (m, 1 H) 7.50 - 7.60 (m, 3H) 8.20 (m, 1 H) 8.30 (m, 1 H)

### PREPARATION 19

### (S)-tert-Butyl 1-((3-aminopyridin-2-yl)(3,5-difluorophenyl)amino)-1-oxopropan-2-ylcarbamate

Same procedure as described in Preparation 17 was used from (S)-2-(tert-butoxycarbonylamino)propanoic acid (3.42 g, 18.08 mmols). The expected compound was obtained (6.49 g, 41%).
LRMS (m/z): 393 (M+1)⁺
1H NMR (400 MHz, DMSO-*d*₆) d ppm 1.31 (d, 3 H) 1.42 (s, 9 H) 4.00 (m, 1 H) 6.61 - 6.82 (m, 1 H) 6.97 (dd, 1 H) 7.38 (dd, 1 H) 7.53 - 7.72 (m, 3 H) 8.04 - 8.34 (m, 2 H) 9.80 (s, 1H)

### PREPARATION 20

### (S)-1-(3-(3,5-Difluorophenyl)-3H-imidazo[4,5-b]pyridin-2-yl)ethanamine

Same procedure as described in Preparation 4 was used from (S)-tert-butyl 1-((3-aminopyridin-2-yl)(3,5-difluorophenyl)amino)-1-oxopropan-2-ylcarbamate (6.50 g, 7.45 mmols, Preparation 19). The expected compound was obtained (1.44 g, 68%).
LRMS (m/z): 275 (M+1)⁺
1H NMR (400 MHz, DMSO-*d*₆) d ppm 1.40 (d, 3 H) 2.00 (s, 2H) 4.10 (q, 1H) 7.40 (m, 1H) 7.50 (m, 3H) 8.10 (m, 1H) 8.30 (m, 1H)

### PREPARATION 21

### (S)-tert-Butyl 1-((3-amino-6-methoxypyridin-2-yl)(pheny)amino)-1-oxobutan-2-ylcarbamate

Same procedure as described in Preparation 13 was used from (S)-2-(tert-butoxycarbonylamino)butanoic acid (1.89 g, 9.30 mmols). The expected compound was obtained (3.42 g, 92%).
LRMS (m/z): 401 (M+1)⁺
1H NMR (400 MHz, DMSO-*d*₆) d ppm 0.96 (t, 3 H) 1.37 - 1.45 (s, 9 H) 1.57 - 1.79 (m, 2 H) 3.78 - 3.88 (m, 3 H) 3.88 - 3.99 (m, 1 H) 6.12 - 6.26 (m, 1 H) 6.81 - 7.03 (m, 1 H) 7.15 - 7.52 (m, 4 H) 7.67 - 7.90 (m, 3 H) 9.45 (s, 1 H)

### PREPARATION 22

### (S)-1-(5-Methoxy-3-phenyl-3H-imidazo[4,5-b]pyridin-2-yl)propan-1-amine

Same procedure as described in Preparation 4 was used from (S)-tert-butyl 1-((3-amino-6-methoxypyridin-2-yl)(phenyl)amino)-1-oxobutan-2-ylcarbamate (3.42 g, 8.54 mmols, Preparation 21). The expected compound was obtained (1.58 g, 66%).
LRMS (m/z): 283 (M+1)⁺
1H NMR (400 MHz, DMSO-*d*₆) d ppm 0.73 (t, 3 H) 1.43 - 1.68 (m, 1 H) 1.68 - 1.87 (m,1 H) 2.09 (s, 2 H) 3.60 - 3.82 (m, 4 H) 6.73 (d, 1 H) 7.42 - 7.74 (m, 5 H) 8.01 (d, 1 H)

### PREPARATION 23

### N-(3,5-Difluorophenyl)-6-methoxy-3-nitropyridin-2-amine

Same procedure as described in Preparation 11 was used from 3,5-difluoroaniline (13.35 g, 100.00 mmols). The expected compound was obtained (7.20 g, 32%).
LRMS (m/z): 282 (M+1)⁺
1H NMR (400 MHz, DMSO-*d*₆) d ppm 3.94 (s, 3 H) 6.48 (d, 1 H) 6.77 - 7.20 (m, 1 H) 7.60 (dd, 2 H) 8.48 (d, 1 H) 10.48 (s, 1 H)

### PREPARATION 24

### N²-(3,5-Difluorophenyl)-6-methoxypyridine-2,3-diamine

Same procedure as described in Preparation 12 was used from N-(3,5-difluorophenyl)-6-methoxy-3-nitropyridin-2-amine (7.20 g, 25.60 mmols, Preparation 23). After 4h the expected compound was obtained (6.30 g, 98%).
LRMS (m/z): 252 (M+1)⁺
1H NMR (400 MHz, DMSO-*d*₆) d ppm 3.75 (s, 3 H) 4.57 (s, 2 H) 6.20 (d, 1 H) 6.51 - 6.68 (m, 1 H) 7.05 (d, 1 H) 7.39 (d, 2 H) 8.22 (s, 1 H)

### PREPARATION 25

### (S)-tert-Butyl 1-((3-amino-6-methoxypyridin-2-yl)(3,5-difluorophenyl)amino)-1-oxopropan-2-ylcarbamate

Same procedure as described in Preparation 13 was used from N²-(3,5-difluorophenyl)-6-methoxypyridine-2,3-diamine (2.00 g, 7.96 mmols, Preparation 24). The expected compound was obtained (2.86 g, 85%).
LRMS (m/z): 423 (M+1)⁺
1H NMR (400 MHz, DMSO-*d*₆) d ppm 1.28 (d, 3 H) 1.42 (s, 9 H) 3.88 (s, 3 H) 3.94 - 4.13 (m, 1 H) 6.32 (d, 1 H) 6.62 - 6.77 (m, 1 H) 7.40 (d, 1 H) 7.47 (d, 1 H) 7.51 - 7.67 (m, 2 H) 8.15 (s, 1 H) 9.53 (s, 1 H)

### PREPARATION 26

### (S)-1-(3-(3,5-Difluorophenyl)-5-methoxy-3H-imidazo[4,5-b]pyridin-2-yl)ethanamine

Same procedure as described in Preparation 4 was used from (S)-tert-butyl 1-((3-amino-6-methoxypyridin-2-yl)(3,5-difluorophenyl)amino)-1-oxopropan-2-ylcarbamate (2.86 g, 6.77 mmols, Preparation 25). The expected compound was obtained (1.31 g, 60%).
LRMS (m/z): 305 (M+1)⁺
1H NMR (400 MHz, DMSO-*d*₆) d ppm 1.40 (d, 3 H) 2.00 (s, 2 H) 3.75 (s, 3 H) 4.10 (m, 1H) 6.75 (m, 1H) 7.50 (m, 1 H) 7.60 (m, 2H) 8.00 (m, 1H)

### PREPARATION 27

### (S)-tert-Butyl 1-((3-amino-6-methoxypyridin-2-yl)(3,5-difluorophenyl)amino)-1-oxobutan-2-ylcarbamate

Same procedure as described in Preparation 25 was used from (S)-2-(tert-butoxycarbonylamino)butanoic acid (1.62 g, 7.97 mmols). After 2 days the expected compound was obtained (3.68 g, 96%).
LRMS (m/z): 437 (M+1)⁺
1H NMR (400 MHz, DMSO-*d*₆) d ppm 0.96 (t, 3 H) 1.42 (s, 9 H) 1.56 - 1.77 (m, 2 H) 3.80 - 4.03 (m, 4 H) 6.32 (d, 1 H) 6.54 - 6.84 (m, 1 H) 7.27 - 7.46 (m, 2 H) 7.49 - 7.69 (m, 2 H) 8.16 (s, 1 H) 9.57 (s, 1 H)

### PREPARATION 28

### (S)-1-(3-(3,5-Difluorophenyl)-5-methoxy-3H-imidazo[4,5-b]pyridin-2-yl)propan-1-amine

Same procedure as described in Preparation 26 was used from (S)-tert-butyl 1-((3-amino-6-methoxypyridin-2-yl)(3,5-difluorophenyl)amino)-1-oxobutan-2-ylcarbamate (3.47 g, 7.95 mmols, Preparation 27). In this case after 24h of hydrolysis in HCl 5N/methanol the expected compound was obtained (0.37 g, 15%).
LRMS (m/z): 319 (M+1)⁺
1H NMR (400 MHz, DMSO-*d*₆) d ppm 0.77 (t, 3 H) 1.60 - 1.71 (m, 1 H) 1.73 - 1.89 (m, 1 H) 2.01 (s, 2 H) 3.61 - 3.90 (m, 4 H) 6.76 (d, 1 H) 7.45 - 7.54 (m, 1 H) 7.54 - 7.69 (m, 2 H) 7.90 - 8.10 (m, 1 H)

### PREPARATION 29

### 6-Methyl-N²-o-tolylpyridine-2,3-diamine

An aqueous solution of HBr (48%) (2.7 mL) was added to a solution of 2-chloro-6-methylpyridin-3-amine (3.00 g, 21.04 mmols) and o-toluidine (2.5 mL, 23.14 mmols) in diethylene glycol (42 mL). It was stirred at 180ºC for 36h. It was concentrated in vacuo and it was extracted with ethyl acetate. The organic phase was washed with a saturated aqueous solution of NaHCO₃, dried, filtered and concentrated in vacuo. It was purified by chromatography (Silica gel, Hexane:Ethyl acetate 0-20%). The expected compound was obtained (0.8 6g, 16%).
LRMS (m/z): 314 (M+1)⁺
¹H NMR (400 MHz, CHLOROFORM-*d*) d ppm 2.16 (s, 3 H) 2.40 (s, 3 H) 6.67 (dd, 2 H) 6.82 - 7.00 (m, 2 H) 7.03 - 7.21 (m, 2 H)

### PREPARATION 30

### 2-(Chloromethyl)-5-methyl-3-o-tolyl-3H-imidazo[4,5-b]pyridine

Same procedure as described in Preparation 5 was used from 6-methyl-N²-o-tolylpyridine-2,3-diamine (0.86 g, 4.04 mmols, Preparation 29). The mass of the expected compound was detected (HPLC-MS) in the main peak (approx.50%) of the crude chromatogram. This crude (1.11 g, 51 %) was used in the next reaction steps without further purification.
LRMS (m/z): 272 (M+1)⁺.

### PREPARATION 31

### 2-(Chloromethyl)-5-methoxy-3-phenyl-3H-imidazo[4,5-b]pyridine

Same procedure as described in Preparation 5 was used from 6-methoxy-N²-phenylpyridine-2,3-diamine (0.69 g, 3.21 mmols, Preparation 12). After 3h the expected compound was obtained (0.86 g, 89%) according to HPLC-MS. This crude was used in the next reaction steps without further purification.
LRMS (m/z): 274 (M+1)⁺.

### PREPARATION 32

### 7-Chloropyrazolo[1,5-a]pyrimidine

Pyrazolo[1,5-a]pyrimidin-7(4H)-one (0.50g, 3.70 mmols), phosphorus oxychloride (0.88 mL, 9.62 mmols) and diisopropylethylamine (DIEA, 0.13 mL, 0.74 mmols) were mixed and stirred at 90ºC overnight. It was poured onto water/ice, extracted with dichloromethane and washed with brine. It was dried, filtered and concentrated in vacuo. It was purified by chromatography (Silica gel, Hexane/Ethyl acetate 9:1) to afford the expected compound (83 mg, 71%).
LRMS (m/z): 154 (M+1)⁺
1H NMR (400 MHz, DMSO-*d*₆) d ppm 6.93 (d, 1 H) 7.43 (d, 1 H) 8.37 (d, 1 H) 8.52 (d, 1 H)

### PREPARATION 33

### 6-Methyl-3-nitro-N-phenylpyridin-2-amine

Same procedure as described in Preparation 1 was used from 2-chloro-6-methyl-3-nitropyridine (2.00 g, 11.59 mmols). In this case, only 1.33 equivalents of aniline were added and 7 mL of ethanol were used. The expected compound was obtained (2.84 g, 100%).
LRMS (m/z): 230 (M+1)⁺
1H NMR (400 MHz, DMSO-*d*₆) d ppm 2.45 (s, 3 H) 6.87 (d,1 H) 7.04 - 7.24 (m, 1 H) 7.26 - 7.48 (m, 2 H) 7.73 (d, 2 H) 8.44 (d, 1 H) 10.06 (s, 1 H)

### PREPARATION 34

### 6-Methyl-N²-phenylpyridine-2,3-diamine

Iron (3.29 g, 61.95 mmols) was added to a cold (0°C) solution of ammonium chloride (3.15 g, 61.95 mmols) in water (93 mL). A solution of 6-methyl-3-nitro-N-phenylpyridin-2-amine (2.84 g, 12.39 mmols, Preparation 33) in a mixture of methanol/tetrahydrofuran (46 mL/46 mL) was added dropwise. It was stirred at 70ºC for 2h. It was filtered, washed and concentrated in vacuo. The aqueous phase obtained was extracted with ethyl acetate and concentrated in vacuo. The expected compound was obtained (2.24 g, 94%).
LRMS (m/z): 200 (M+1)⁺
1H NMR (400 MHz, DMSO-*d*₆) d ppm 2.24 (s, 3 H) 4.83 (s, 2 H) 6.47 (d, 1 H) 6.71 - 6.96 (m, 2 H) 7.02 - 7.39 (m, 2 H) 7.50 - 7.75 (m, 3 H)

### PREPARATION 35

### (S)-tert-Butyl 1-((3-amino-6-methylpyridin-2-yl)(phenyl)amino)-1-oxopropan-2-ylcarbamate

Same procedure as described in Preparation 3 was used from 6-methyl-N²-phenylpyridine-2,3-diamine (2.24 g, 11.24 mmols, Preparation 34). The expected compound was obtained (2.77g, 67%). This sample was used in the next reaction step without further purification.
LRMS (m/z): 371 (M+1)⁺
1H NMR (600 MHz, DMSO-*d*₆) d ppm 1.28 (d, 3 H) 1.41 (s, 9 H) 2.38 (s, 3 H) 3.93 - 4.12 (m, 1 H) 6.67 (d, 1 H) 6.80 - 6.99 (m, 1 H) 7.15 - 7.29 (m, 2 H) 7.30 - 7.47 (m, 2 H) 7.65 - 7.92 (m, 3 H) 9.57 (s, 1 H)

### PREPARATION 36

### (S)-1-(5-Methyl-3-phenyl-3H-imidazo[4,5-b]pyridin-2-yl)ethanamine

Same procedure as described in Preparation 4 was used from (S)-tert-butyl 1-((3-amino-6-methylpyridin-2-yl)(phenyl)amino)-1-oxopropan-2-ylcarbamate (2.77 g, 7.48 mmols, Preparation 35). The expected compound was obtained (1.62 g, 86%).
LRMS (m/z): 253 (M+1)⁺
1H NMR (600 MHz, DMSO-*d*₆) d ppm 1.31 (d, 3 H) 1.99 (s, 2 H) 2.47 (s, 3 H) 3.97 (q, 1 H) 7.17 (d, 1 H) 7.44 - 7.69 (m, 5 H) 7.96 (d, 1 H)

### PREPARATION 37

### 2-(Chloromethyl)-3-o-tolyl-3H-imidazo[4,5-b]pyridine

N²-o-Tolylpyridine-2,3-diamine (30 mg, 0.15 mmol, prepared according to J. Het. Chem. 1983, 20, 1339) and 2-chloroacetic acid (21 mg, 0.23 mmol) were dissolved in 5N HCl (753 µL) and heated at 130 ºC for 18h in a microwave oven. After cooling to room temperature, acetic acid was removed in vacuo. Ethyl acetate and 4% sodium bicarbonate was added and the organic layer was decanted and washed with water and brine. The resulting material (280 mg) was purified by column chromatography (silica gel, hexane/Ethyl acetate 0 to 50% in 30 CV, and then from 50 to 100% in 10 CV) to yield 170 mg (46%) of the final compound as a palid-yellow oil.
LRMS (m/z): 259 (M+1)⁺.

### PREPARATION 38

### Ethyl 4-(3-nitropyridin-2-ylamino)piperidine-1-carboxylate

2-Chloro-3-nitropyridine (4.0 g, 25.23 mmol), ethyl 4-aminopiperidine-1-carboxylate (5.21 g, 30.28 mmol) and potassium carbonate were mixed in N,N-dimethylformamide (50 mL) and heated overnight at 100 ºC under nitrogen atmosphere. The solvent was

removed under reduced pressure and then dichloromethane and water were added. The organic layer was separated, washed with water and brine, dried over magnesium sulfate and the solvent removed to yield 8.47 g (100%) of the final compound, which was used in the next synthetic step without further purification.
LRMS (m/z): 295 (M+1)⁺.

### PREPARATION 39

### Ethyl 4-(3-aminopyridin-2-ylamino)piperidine-1-carboxylate

Ethyl 4-(3-nitropyridin-2-ylamino)piperidine-1-carboxylate (8.47 g, 25.90 mmol, Preparation 38) and iron powder (5.79 g, 55.85 mmol) were mixed with saturated solution of ammonia chloride (30 mL) and 5N HCl and then heated overnight at 80 ºC. The crude reaction was filtered over Celite® and the solvent was removed in vacuo. Water was added and it was neutralised with sodium bicarbonate. Then dichloromethane was added and the organic layer was washed with water and brine, dried over magnesium sulfate, filtered and the solvent removed under reduced pressure to yield 6.15 g (90%) a dark solid, pure enough to perform the next synthetic step.
LRMS (m/z): 265 (M+1)⁺.

### PREPARATION 40

### Ethyl 4-(2-(chloromethyl)-3H-imidazo[4,5-b]pyridin-3-yl)piperidine-1-carboxylate

Obtained as an oil (1.40 g, 17%) from ethyl 4-(3-aminopyridin-2-ylamino)piperidine-1-carboxylate (2.50 g, 9.46 mmol, Preparation 39) and 2-chloroacetic acid (839 µL, 10.40 mmol) following the experimental procedure as described in Preparation 37.
LRMS (m/z): 323 (M+1)⁺.

### PREPARATION 41

### Ethyl 4-(2-((6-amino-9H-purin-9-yl)methyl)-3H-imidazo[4,5-b]pyridin-3-yl)piperidine-1-carboxylate

Ethyl 4-(2-(chloromethyl)-3H-imidazo[4,5-b]pyridin-3-yl)piperidine-1-carboxylate (150 mg, 0.46 mmol, Preparation 40), 9H-purin-6-amine (75 mg, 0.56 mmol) and potassium carbonate (129 mg, 0.93 mmol) were mixed in DMF (2 mL) and stirred at room temperature for 18h and then overnight at 50 ºC. The solvent was removed under reduced pressure and the residue was purified by column chromatography (silica gel, hexane/Ethyl acetate 0 to 50% in 30 CV, and then from 50 to 100% in 10 CV) to yield of the final compound (325 mg, 99%) as a white solid.
LRMS (m/z): 422 (M+1)⁺.

### PREPARATION 42

### 5-Bromo-3-nitro-N-phenylpyridin-2-amine

To a solution of 5-bromo-2-chloro-3-nitropyridine (1.00 g, 4.17 mmol) and aniline (0.46 mL, 5.00 mmol) in dimethyl sulfoxide (dry) (3 mL) was added triethyl amine (2.90 mL, 20.85 mmol) and the reaction mixture was heated at 80 °C overnight. The reaction mixture was poured into water followed by extraction with CH₂Cl₂. The organic phase was washed with brine, dried (Na₂SO₄), filtered and evaporated to dryness to afford 5-bromo-3-nitro-N-phenylpyridin-2-amine (1.38 g, 4.69 mmol, 112 % yield) as a dark red solid. The product was used without further purification.
LRMS (m/z): 295 (M+1)⁺.

### PREPARATION 43

### 5-Bromo-N²-phenylpyridine-2,3-diamine

A suspension of 5-bromo-3-nitro-N-phenylpyridin-2-amine (1.2 g, 4.17 mmol, Preparation 42), sodium sulfide nonahydrate (4.09 g, 16.68 mmol) and ammonium chloride (0.90 mg, 16.68 mmol) in methanol (45 mL) was refluxed for 2 h. The reaction mixture was cooled to room temperature, filtered and evaporated to dryness under reduced pressure to afford a dark oil which crystallized overnight. The crude was partitioned between CH₂Cl₂ and H₂O and after separation of the layers the organic layer was dried (Na₂SO₄), filtered and evaporated to dryness under reduced pressure to afford 5-bromo-N²-phenylpyridine-2,3-diamine (0.91 g, 3.46 mmol, 83 % yield) as a brown/grey solid.
LRMS (m/z): 265 (M+1)⁺.

### PREPARATION 44

### 6-Bromo-2-(chloromethyl)-3-phenyl-3H-imidazo[4,5-b]pyridine

To a solution of 5-bromo-N²-phenylpyridine-2,3-diamine (0.85 g, 3.21 mmol, Preparation 43) in dichloromethane (16 mL) was added 2-chloro-1,1,1-trimethoxyethane (1.32 mL, 9.64 mmol) and p-toluenesulfonic acid monohydrate (62 mg, 0.32 mmol). The reaction mixture was stirred in a closed system (Radley tube) at 30 °C for 1 h. The reaction mixture was cooled to room temperature followed by the addition of water. After phase separation the organic phase was dried (Na₂SO₄), filtered and evaporated to dryness under reduced pressure to afford the product as a dark solid. Purification by flash chromatography (silica gel, heptane/Ethyl acetate, 5:1) afforded 6-bromo-2-(chloromethyl)-3-phenyl-3H-imidazo[4,5-b]pyridine (0.92 g, 2.86 mmol, 89 % yield) as an off-white solid.
LRMS (m/z): 323 (M+1)⁺.

### PREPARATION 45

### 9-((6-Bromo-3-phenyl-3H-imidazo[4,5-b]pyridin-2-yl)methyl)-9H-purin-6-amine

A mixture of 6-bromo-2-(chloromethyl)-3-phenyl-3H-imidazo[4,5-b]pyridine (150 mg, 0.47 mmol, Preparation 44), adenine (63 mg, 0.47 mmol), potassium carbonate (64 mg, 0.47 mmol) in N,N-dimethylformamide (dry, 3 mL) was stirred at room temperature overnight. The reaction mixture was evaporated to dryness under reduced pressure to afford a white solid that was partitioned between H₂O and Ethyl acetate. The layers were separated, the organic phase was dried (Na₂SO₄), filtered and evaporated to dryness under reduced pressure to afford 9-((6-bromo-3-phenyl-3H-imidazo[4,5-b]pyridin-2-yl)methyl)-9H-purin-6-amine (191 mg, 0.45 mmol, 98 % yield) as a light yellow/white solid.
LRMS (m/z): 422 (M+1)⁺.

### PREPARATION 46

### tert-Butyl 5-nitropyridin-2-ylcarbamate

200 mL of THF was added to 5-nitropyridin-2-amine (5g, 35.90 mmol). The solution was cooled to 0 °C. Sodium hexamethyldisilazide (NaHMDS, 13.18 g, 71.9 mmol) in 30 mL THF was added dropwise. At the same time, di-*tert*-butyl dicarbonate (8.3 mL, 35.90 mmol) in 30 mL THF was added dropwise. The reaction mixture was stirred at 0 °C for 15min and then warmed to room temperature. The reaction mixture stopped stirring due to salt/product formation and was left at room temperature for 20h. 150 mL 0.5N HCl was added and 150 mL ethyl acetate was added. The layers were separated. The organic layer was washed with 50 mL brine, dried with Na₂SO₄, filtered and evaporated to dryness. A yellow solid was isolated, which was used in the next synthetic step without further purification.
LRMS (m/z): 240 (M+1)⁺

### PREPARATION 47

### tert-Butyl 5-aminopyridin-2-ylcarbamate

*tert-*Butyl 5-nitropyridin-2-ylcarbamate (4.00 g, 16.72 mmol, Preparation 46) was dissolved in THF/MeOH. Pd/C (2.03 g, 1.91 mmol) was added and the reaction mixture was put under a hydrogen atmosphere. The reaction mixture was stirred for a total of 6h. The reaction mixture was filtered and evaporated to yield the desired product as a purple solid (3.40 g, 97% yield) which was stored at -20 °C.
LRMS (m/z): 210 (M+1)⁺

### PREPARATION 48

### tert-Butyl 5-amino-6-bromopyridin-2-ylcarbamate

*tert*-Butyl 5-aminopyridin-2-ylcarbamate (3.40 g, 16.25 mmol, Preparation 47) was dissolved in N,N-dimethylformamide (300 mL) and cooled to 0 °C. N-bromosuccinimide (NBS, 2.89 g, 16.25 mmol) was dissolved in 100 mL DMF and added dropwise. The reaction mixture was allowed to warm up to room temperature. DMF was evaporated. The oily residue was coated on isolute and purified by column chromatography (silica gel, Heptane/Ethyl acetate 0-30%). The fractions containing the product were combined and evaporated to dryness. The product was isolated as a yellowish solid (3.11g,66%).
LRMS (m/z): 289 (M+1)⁺

### PREPARATION 49

### tert-Butyl 5-amino-6-(phenylamino)pyridin-2-ylcarbamate

Under argon, *tert*-butyl 5-amino-6-bromopyridin-2-ylcarbamate (1.00 g, 3.47 mmol, Preparation 48) was dissolved in toluene (100 mL), tris(dibenzylideneacetone)dipalladium(0) (Pd₂dba₃, 0.16 g, 0.17 mmol), (S)-(-)-(1,1'-Binaphthalene-2,2'-diyl)bis(diphenylphosphine) (BINAP, 0.11 g, 0.17 mmol) and sodium *tert*-butoxide (0.50 g, 5.21 mmol) and aniline (3.2 mL, 34.7 mmol) were added, then stirred at 80 °C overnight. The reaction mixture was quenched with 5mL 1 M HCl, diluted with Ethyl acetate and then washed with water and brine. The organic layer was evaporated to dryness and purified by column chromatography (silica gel, Heptane/Ethyl acetate 5-50%). The fractions containing the product were evaporated and the final compound (480 mg, 46%) was isolated as a yellow/black foam.
LRMS (m/z): 301 (M+1)⁺

### PREPARATION 50

### tert-Butyl 2-(chloromethyl)-3-phenyl-3H-imidazo[4,5-b]pyridin-5-ylcarbamate

tert-Butyl 5-amino-6-(phenylamino)pyridin-2-ylcarbamate (260 mg, 0.87 mmol, Preparation 49) was dissolved in dichloromethane (10 mL). 2-chloro-1,1,1-trimethoxyethane (0.35 mL, 2.60 mmol) was added and the mixture was divided into 2 batches. *para*-Toluenesulfonic acid (pTsOH, 8 mg) was added to each batch. The reaction mixtures were stirred at room temperature for 72h in a closed vial. The reaction mixture was washed with water and dried over a phase separator. The organic layer was evaporated to dryness and purified by column chromatography (silica gel, Heptane/Ethyl acetate 3-30%) to isolate the final compound (250 mg, 80%).
LRMS (m/z): 360 (M+1)⁺

### PREPARATION 51

### tert-Butyl 2-((6-amino-9H-purin-9-yl)methyl)-3-phenyl-3Himidazo[4,5-b]pyridin-5-ylcarbamate

tert-Butyl 2-(chloromethyl)-3-phenyl-3H-imidazo[4,5-b]pyridin-5-ylcarbamate (80 mg, 0.22 mmol, Preparation 50), adenine (32 mg, 0.23 mmol) and potassium carbonate (32 mg, 0.23 mmol) were dissolved in 3 mL DMF. The reaction mixture was then stirred at room temperature for 2h. The reaction mixture was then diluted with 10 mL dichloromethane and washed with water. The organic layer was separated, and evaporated to dryness. The oily residue was dissolved in Ethyl acetate and washed with water. Evaporation yielded a brownish foam. The crude product was purified by column chromatography (silica gel, DCM/MeOH gradient to 95/5) afforded the product as a white solid (77 mg, 75%).
LRMS (m/z): 458 (M+1)⁺

### PREPARATION 52

### tert-Butyl 2-((9H-purin-6-ylthio)methyl)-3-phenyl-3Himidazo[4,5-b]pyridin-5-ylcarbamate

tert-Butyl 2-(chloromethyl)-3-phenyl-3H-imidazo[4,5-b]pyridin-5-ylcarbamate (84 mg, 0.23 mmol, Preparation 50), 6-mercaptopurine monohydrate (44 mg, 0.26 mmol) and potassium carbonate (32 mg, 0.23 mmol) were dissolved in N,N-dimethylformamide (dry, 5 mL) and stirred at room temperature for 3h. The reaction mixture was diluted

with dichloromethane (5 mL) and filtered. The solution was evaporated to dryness and purified by column chromatography to yield the final compound (118 mg, 100%).
LRMS (m/z): 476 (M+1)⁺

### PREPARATION 53

### 2-Chloro-5-fluoro-3-nitropyridine

5-Fluoro-3-nitro-pyridin-2-ol (1.00 g, 5.33 mmol) was dissolved in acetonitrile (40 mL) and tetraethylammonium chloride (2.10g, 12.65 mmol) was added. The mixture became clear, phosphorous oxytrichloride (1.94 g, 12.65 mmol) was added at room temperature and the mixture was heated at 90 ºC for 24h. The reaction mixture was evaporated to dryness; the residue was taken up with water (100 mL) and extracted with ethyl acetate (2x100 mL). The organic layer was dried with sodium sulfate, filtered and evaporated to dryness. A yellow solid (0.90 g, 69%) was isolated, pure enough to perform the next synthetic step.
LRMS (m/z): 177 (M+1)⁺

### PREPARATION 54

### 5-Fluoro-3-nitro-N-phenylpyridin-2-amine

2-Chloro-5-fluoro-3-nitropyridine (0.90 g, 5.10 mmol, Preparation 53) and aniline (4.7 mL, 51 mmol) were mixed and heated at 100 ºC for 3h. Once at room temperature, the reaction mixture was diluted with DCM, washed with water and the organic layer was dried from sodium sulfate, filtered and evaporated. The oily residue was recrystallized once from Ethyl acetate/Heptane which gave 737 mg of the final product as orange/red crystals. Column chromatography afforded a second batch of 120 mg. Total yield: 857 mg (72%).
LRMS (m/z): 234 (M+1)⁺

### PREPARATION 55

### 5-Fluoro-N²-phenylpyridine-2,3-diamine

Obtained as an oil (767 mg, 100%) from 5-fluoro-3-nitro-N-phenylpyridin-2-amine (737 mg, 3.16 mmol, Preparation 54) following the experimental procedure as described in Preparation 47.
LRMS (m/z): 204 (M+1)⁺.

### PREPARATION 56

### (S)-tert-Butyl 1-(5-fluoro-2-(phenylamino)pyridin-3-ylamino)-1-oxopropan-2-ylcarbamate

(S)-2-(tert-Butoxycarbonylamino)propanoic acid (598 mg, 3.16 mmol) and 2-(1H-7-azabenzotriazol-1-yl)-1,1,3,3-tetramethyl uronium hexafluorophosphate (HATU, 1.32g, 3.48 mmol) were dissolved in dichloromethane (35 mL). Diisopropylethylamine (DIEA, 0.6 mL, 3.16 mmol) was added. Then N,N-dimethylformamide was added till the solution became clear (10 mL). The reaction mixture was stirred for 20 min at rt. 5-Fluoro-N²-phenylpyridine-2,3-diamine (642 mg, 3.16 mmol, Preparation 55) in dichloromethane (10 mL) was added and the reaction mixture was stirred at room temperature (rt) for 2.5 h. The reaction mixture was evaporated to dryness and taken up in ethylacetate. The organic layer was washed with water, dried, filtered and evaporated to dryness. The crude product was purified by flash chromatography (SQ16, silica gel, heptane/Ethyl acetate 0-30%) to yield 319 mg (18%) of the final compound.
LRMS (m/z): 375 (M+1)⁺.

### PREPARATION 57

### (S)-1-(6-Fluoro-3-phenyl-3H-imidazo[4,5-b]pyridin-2-yl)ethanamine

A solution of (S)-tert-butyl 1-(5-fluoro-2-(phenylamino)pyridin-3-ylamino)-1-oxopropan-2-ylcarbamate (310 mg, 0.838 mmol, Preparation 56) in acetic acid (15 mL) was heated to 100 °C and was stirred overnight. Analysis showed conversion to the ring-closed acylated product, but also the presence of product which still contains the tert-butoxycarbonyl group. The acetic acid was evaporated under reduced pressure. To the crude product (247 mg, 0.83 mmol) in methanol (15 mL) was added aqueous 6 M HCl (15 mL, 90 mmol) and the reaction mixture was stirred at 85°C overnight. The reaction mixture was left to cool to room temperature. The reaction mixture was transferred onto SCX columns, first eluting with MeOH to remove impurities followed by retrieving the compound by eluting with ∼2 M NH₃ in MeOH. Evaporation under reduced pressure afforded (S)-1-(6-fluoro-3-phenyl-3H-imidazo[4,5-b]pyridin-2-yl)ethanamine (109 mg, 0.43 mmol, 51%) as a dark brown oil. The product was used in the next step without further purification.
LRMS (m/z): 257 (M+1)⁺.

### PREPARATION 58

### (S)-tert-Butyl 1-(5-fluoro-2-(phenylamino)pyridin-3-ylamino)-1-oxobutan-2-ylcarbamate

Obtained as an oil from 5-fluoro-N²-phenylpyridine-2,3-diamine (Preparation 55) and (S)-2-(tert-butoxycarbonylamino)butanoic acid following the experimental procedure as described in Preparation 56.
LRMS (m/z): 389 (M+1)⁺.

### PREPARATION 59

### (S)-1-(6-Fluoro-3-phenyl-3H-imidazo[4,5-b]pyridin-2-yl)propan-1-amine

Obtained as an oil from (S)-tert-butyl 1-(5-fluoro-2-(phenylamino)pyridin-3-ylamino)-1-oxobutan-2-ylcarbamate (Preparation 58) following the experimental procedure as described in Preparation 57.
LRMS (m/z): 271 (M+1)⁺.

### PREPARATION 60

### N-(2-Chlorophenyl)-3-nitropyridin-2-amine

2-Chloroaniline (6.7 mL, 0.06 mol) and 2-chloro-3-nitropyridine (5.04 g, 0.03 mol) were dissolved in ethanol (10 mL) and heated at 150 ºC for 20 min in a microwave oven. The crude reaction mixture was filtered and washed with ethanol, to yield the title compound (6.72 g, 85%) as an orange solid, pure enough to perform the next synthetic step without further purification.
LRMS (m/z): 251 (M+1)⁺.
1 H NMR (600 MHz, cdcl3) δ 10.64 - 10.44 (m, 1 H), 8.60 - 8.55 (m, 1 H), 8.51 (dt, 1 H), 8.45 (dd, 1 H), 7.47 (dd, 1 H), 7.37 - 7.29 (m, 1 H), 7.15 - 7.07 (m, 1 H), 6.94 - 6.88 (m, 1 H).

### PREPARATION 61

### N²-(2-Chlorophenyl)pyridine-2,3-diamine

N-(2-Chlorophenyl)-3-nitropyridin-2-amine (6.72 g, 26.72 mmol, Preparation 60) and powered iron (24.20 g, 433.38 mmol) were suspended in ethanol (175 mL) and water (35 mL). 35% HCl (83 µL, 2.71 mmol) was added and the mixture heated at 80 ºC for 2h. After cooling to room temperature, the reaction mixture was filtered through Celite® and washed with ethyl ether. The title compound precipitates (4.35 g, 71%) from the filtrated liquid, pure enough to perform the next synthetic step without further purification.
LRMS (m/z): 220 (M+1)⁺.
1 H NMR (400 MHz, dmso) δ 7.93 (d, 1 H), 7.47 (dd, 2H), 7.28 - 7.14 (m, 2H), 7.05 - 6.83 (m, 2H), 6.68 (dt, 1 H), 5.04 (d, 2H).

### PREPARATION 62

### tert-Butyl 1-((3-aminopyridin-2-yl)(2-chlorophenyl)amino)-1-oxopropan-2-ylcarbamate

Obtained as an oil (1.28 g, 30%) from N²-(2-chlorophenyl)pyridine-2,3-diamine (1.00 g, 4.55 mmol, Preparation 61) and (S)-2-(tert-butoxycarbonylamino)propanoic acid (0.86 g, 4.55 mmol) following the experimental procedure as described in Preparation 56.
LRMS (m/z): 391 (M+1)⁺.
1 H NMR (400 MHz, cdcl3) δ 8.27 (s, 1 H), 8.16 (dd, 1 H), 7.87 (dd, 1 H), 7.77 (d, 1 H), 7.36 (dd, 1 H), 7.23 - 7.15 (m, 1 H), 7.07 (s, 1 H), 6.97 - 6.86 (m, 2H), 4.95 (d, 1 H), 4.37 (s, 1 H), 1.46 (t, 3H), 1.42 (s, 9H).

### PREPARATION 63

### (S)-1-(3-(2-Chlorophenyl)-3H-imidazo[4,5-b]pyridin-2-yl)ethanamine

Obtained as an oil (150 mg, 40%) from tert-butyl 1-((3-aminopyridin-2-yl)(2-chlorophenyl)amino)-1-oxopropan-2-ylcarbamate (0.54 g, 1.38 mmol, Preparation 62) following the experimental procedure as described in Preparation 57.
LRMS (m/z): 273 (M+1)⁺.
1 H NMR (400 MHz, cdcl3) δ 8.33 (dd, 1 H), 8.19 - 8.00 (m, 1 H), 7.79 - 7.60 (m, 1 H), 7.58 - 7.47 (m, 3H), 7.33 - 7.19 (m, 1 H), 4.10 - 3.94 (m, 1 H), 1.67(s,2H), 1.51 (d, 3H).

### PREPARATION 64

### tert-Butyl 2-((3-aminopyridin-2-yl)(2-chlorophenyl)amino)-2-oxoethylcarbamate

Obtained as white solid (0.97 g, 59%) from N²-(2-chlorophenyl)pyridine-2,3-diamine (1.00 g, 4.42 mmol, Preparation 61) and (S)-2-(tert-butoxycarbonylamino)acetic acid (0.77 g, 4.40 mmol) following the experimental procedure as described in Preparation 56.
LRMS (m/z): 377 (M+1)⁺.
1 H NMR (400 MHz, cdcl3) δ 8.18 (dd, 1 H), 8.07 (s, 1 H), 7.88 (d, 1 H), 7.75 (dd, 1 H), 7.37 (dd, 1 H), 7.24 - 7.13 (m, 1 H), 7.07 - 6.83 (m, 3H), 5.06 (d, 1 H), 3.97 (d, 2H), 1.44 (s, 9H).

### PREPARATION 65

### (3-(2-Chlorophenyl)-3H-imidazo[4,5-b]pyridin-2-yl)methanamine

Obtained as an oil (0.49 g, 74%) from tert-butyl 2-((3-aminopyridin-2-yl)(2-chlorophenyl)amino)-2-oxoethylcarbamate (0.97 g, 2.57 mmol, Preparation 64) following the experimental procedure as described in Preparation 57.
LRMS (m/z): 259 (M+1)⁺.
1 H NMR (400 MHz, cdcl3) δ 8.33 (dd, 1 H), 8.12 - 8.04 (m, 1 H), 7.70 - 7.60 (m, 1 H), 7.58 - 7.42 (m, 3H), 7.31 - 7.24 (m, 1 H), 3.91 (q, 2H), 3.49 - 3.43 (m, 2H).

### PREPARATION 66

### 2-Nitro-N-phenylpyridin-3-amine

A mixture of 3-chloro-2-nitropyridine (100 mg, 0.62 mmol), aniline (0.57 mL, 6.18 mmol) and potassium carbonate (214 mg, 1.55 mmol) in N,N-dimethylformamide (dry, 1 mL) was heated at 140ºC in a microwave for 3 h (and was left standing overnight). The reaction mixture was added to water. The pH was set at ∼7 using aqueous 1 M HCl. Ethyl acetate was added and the layers were separated. The organic phase was dried (Na₂SO₄), filtered and evaporated to dryness under reduced pressure to afford 1.92 g of crude product. Purification by column chromatography (silica gel, heptane/Ethyl acetate, 9:1) afforded 2-nitro-N-phenylpyridin-3-amine (260 mg, 1.208 mmol, 65.2 % yield) as a dark brown oil.
LRMS (m/z): 216 (M+1)⁺.

### PREPARATION 67

### N³-Phenylpyridine-2,3-diamine

To a with argon flushed solution of 2-nitro-N-phenylpyridin-3-amine (130 mg, 0.60 mmol, Preparation 66) in ethanol (3 mL) was added Pd/C (6 mg, 0.06 mmol). The reaction mixture was put under a hydrogen atmosphere using a balloon and was stirred at room temperature overnight. The reaction mixture was filtered and evaporated to dryness under reduced pressure. Purification by column chromatography (SQ16, silica gel, heptane/Ethyl acetate) afforded N³-phenylpyridine-2,3-diamine (20 mg, 0.11 mmol, 10% yield) as a light brown solid.
LRMS (m/z): 186 (M+1)⁺.

### PREPARATION 68

### 2-(Chloromethyl)-1-phenyl-1H-imidazo[4,5-b]pyridine

To a solution of N³-phenylpyridine-2,3-diamine (33 mg, 0.18 mmol, Preparation 67) in dichloromethane (3 mL) was added 2-chloro-1,1,1-trimethoxyethane (74 µl, 0.54 mmol) and p-toluenesulfonic acid monohydrate (3 mg, 0.02 mmol). The reaction mixture was stirred overrnight at room temperature. The reaction mixture was evaporated to dryness under reduced pressure. Dichloromethane and water were added and the organic phase was separated using a phase separator, washed with aqueous 1M NaOH and dried over magnesium sulphate. Subsequent evaporation of the solvent afforded the title compound (39 mg, 0.16 mmol, 89 % yield) as a dark oil.
LRMS (m/z): 244 (M+1)⁺.

### PREPARATION 69

### (S)-tert-Butyl 1-oxo-1-(3-(phenylamino)pyridin-2-ylamino)propan-2-ylcarbamate

To a solution of (S)-2-(tert-butoxycarbonylamino)propanoic acid (560 mg, 2.96 mmol) in dichloromethane (20 mL) was added O-(7-azabenzotriazol-1-yl)-N,N,N',N'-tetramethyluronium hexafluorophosphate (HATU, 1.24g, 3.25 mmol) and N,N-dimethylformamide (10 mL). The reaction mixture was stirred at room temperature for 10 min. To the reaction mixture were added N³-phenylpyridine-2,3-diamine (0.55 g, 2.96 mmol, Preparation 67) and diisopropylethylamine (DIEA, 0.52 mL, 2.96 mmol) and the reaction mixture was stirred at room temperature overnight. Analysis showed conversion to desired product, but also the presence of the starting material. Additional (S)-2-(tertbutoxycarbonylamino) propanoic acid (224 mg, 1.18 mmol), HATU (0.56 g, 1.48 mmol) and DIEA (0.52 mL, 2.96 mmol) were added and the reaction mixture was left stirring for 48h. The reaction mixture was concentrated under reduced pressure, transferred straight onto a silica gel column and was purified by flash chromatography (SQ16, silica gel, heptane/Ethyl acetate) to afford (S)-tert-butyl 1-oxo-1-(3-(phenylamino)pyridin-2-ylamino)propan-2-ylcarbamate (0.90 g, 2.39 mmol, 81% yield) as a colourless sticky solid.
LRMS (m/z): 357 (M+1)⁺.

### PREPARATION 70

### (S)-N-(1-(1-Phenyl-1H-imidazo[4,5-b]pyridin-2-yl)ethyl)acetamide

A solution of (S)-tert-butyl 1-oxo-1-(3-(phenylamino)pyridin-2-ylamino)propan-2-ylcarbamate (450 mg, 1.26 mmol, Preparation 69) in acetic acid (20 mL) was heated to 100 °C and was stirred overnight. The acetic acid was evaporated under reduced pressure. The crude product was used in the next step without purification process.
LRMS (m/z): 281 (M+1)⁺

### PREPARATION 71

### (S)-1-(1-Phenyl-1H-imidazo[4,5-b]pyridin-2-yl)ethanamine

(S)-N-(1-(1-Phenyl-1H-imidazo[4,5-b]pyridin-2-yl)ethyl)acetamide (354 mg, 1.26 mmol, Preparation 70) was dissolved in methanol (10 mL). 6N HCl (10 mL) was added and the reaction mixture was stirred at 80 ºC overnight. The reaction mixture was transferred to a SCX-3 column (5g) and flushed with methanol. The product (190 mg, 63%) was eluted with 2N NH₃ in methanol.
LRMS (m/z): 239 (M+1)⁺

### PREPARATION 72

### N-(2,6-Difluorophenyl)-3-nitropyridin-2-amine

2,6-Difluoroaniline (13.6 mL, 0.13 mol) was added to a solution of 2-chloro-3-nitropyridine (10g, 0.06 mol) in ethanol (25 mL). It was heated under microwave irratiadion at 150ºC for 10 min. It was concentrated in vacuo and purified by chromatography (Silica gel, Hexane:ethyl acetate 2:1) to afford the expected compound (1.15g, 7%).
LRMS (m/z): 252 (M+1)⁺
1 H NMR (400 MHz, DMSO-*d*₆) d ppm 6.98 (dd, 1 H) 7.20 (dd, 2H) 7.32 - 7.46 (m, 1 H) 8.41 (dd, 1 H) 8.55 (dd, 1 H) 9.60 (s, 1 H)

### PREPARATION 73

### N²-(2,6-Difluorophenyl)pyridin-2,3-diamine

N-(2,6-difluorophenyl)-3-nitropyridin-2-amine (1.15g, 4.58 mmol, Preparation 72) was dissolved in acetone (30 mL) and heated to 60 ºC. A solution of tin(II)chloride monohydrate (3.10g, 13.73 mmol) in concentrated hydrochloric acid (7 mL) was dropwise added at this temperature. After 2h of stirring at 60 ºC, the solvent was evaporated at reduced pressure and the aqueous residue was poured on ice, basified to pH=10 with 32%NaOH and extracted with ethyl acetate. The organic phase was washed with water, dried over magnesium sulfate, filtered and the solvent evaporated. The crude residue (1.22g) was used in the next sythetic step without further purification.
LRMS (m/z): 222 (M+1)⁺
1H NMR (400 MHz, CHLOROFORM-*d*) d ppm 6.09 (m, 1H) 6.74 (m, 1H) 6.95 (m, 5H) 7.05 (m, 2H)

### PREPARATION 74

### (S)-tert-Butyl 1-((3-aminopyridin-2-yl)(2,6-difluorophenyl)amino)-1-oxopropan-2-ylcarbamate

Obtained (22%) from N²-(2,6-difluorophenyl)pyridin-2,3-diamine (1.22g, 3.53 mmol, Preparation 73) and (S)-2-(tert-butoxycarbonylamino)propanoic acid (0.70 g, 3.70 mmol) following the experimental procedure as described in Preparation 69.
LRMS (m/z): 393 (M+1)⁺.
1H NMR (400 MHz, DMSO-*d*₆) d ppm 1.29 (d, 3 H) 1.35 (s, 9 H) 4.04 - 4.20 (m, 1 H) 6.29 - 6.49 (m, 1 H) 7.03 - 7.29 (m, 2 H) 7.34 - 7.41 (m, 1 H) 7.77 - 7.82 (m, 1 H) 7.86 - 7.93 (m, 1 H) 8.01 (br. s., 1 H) 9.52 (br. s., 1 H)

### PREPARATION 75

### (S)-1-(3-(2,6-Difluorophenyl)-3H-imidazo[4,5-b]pyridin-2-yl)ethanamine

Obtained as a brownish solid (50%) from (S)-tert-butyl 1-((3-aminopyridin-2-yl)(2,6-difluorophenyl)-amino)-1-oxopropan-2-yl carbamate (0.30g, 0.76 mmol, Preparation 74) following the experimental procedure as described in Preparation 57.
LRMS (m/z): 275 (M+1)⁺.
1H NMR (400 MHz, cdcl3) δ 7.89 (d, 1H), 7.53 (tt, 1H), 7.16 (dd, 1H), 7.10 - 7.01 (m, 1H), 6.92 (t, 1H), 6.78 (dd, 1H), 6.60 (d, 1H), 3.99 (q, 1H), 1.84 (s, 2H), 1.46 (d, 3H).

### PREPARATION 76

### N-(2-Methoxyphenyl)-3-nitropyridin-2-amine

Obtained (6.52g, 84%) from 2-chloro-3-nitropyridine (5.00g, 31.54 mmol) and 2-methoxyaniline (10.7 mL, 84.88 mmol) by heating both reactants at 150ºC for 3h without solvent and subsequent purification of the crude reaction mixture by chromatography (Silica gel, dichloromethane:methanol from 0 to 5%) to afford the expected compound.
LRMS (m/z): 246 (M+1)⁺.
1H NMR (400 MHz, cdcl3) δ 10.63 (d, 1H), 8.77 - 8.33 (m, 3H), 7.09 (tt, 1H), 7.05 - 6.92 (m, 2H), 6.81 (dd, 1H), 3.96 (s, 3H).

### PREPARATION 77

### N²-(2-Methoxyphenyl)pyridine-2,3-diamine

Obtained (1.24g, 94%) from N-(2-methoxyphenyl)-3-nitropyridin-2-amine (1.50g, 6.12 mmol, Preparation 76) following the experimental procedure as described in Preparation 61.
LRMS (m/z): 216 (M+1)⁺.
1H NMR (400 MHz, cdcl3) δ 8.08 - 7.94 (m, 1H), 7.85 (dd, 1H), 7.00 (dd, 1H), 6.98 - 6.92 (m, 1H), 6.91 - 6.88 (m, 2H), 6.83 (s, 1H), 6.74 (dd, 1H), 3.91 (d, 3H), 3.50 (d, 2H).

### PREPARATION 78

### (S)-tert-Butyl 1-((3-aminopyridin-2-yl)(2-methoxyphenyl)amino)-1-oxopropan-2-ylcarbamate

Obtained (0.83g, 35%) from N²-(2-Methoxyphenyl)pyridine-2,3-diamine (0.83g, 2.04 mmol, Preparation 77) following the experimental procedure as described in Preparation 56.
LRMS (m/z): 387 (M+1)⁺.
1H NMR (400 MHz, cdcl3) δ 8.15 (d, 1H), 7.99 (d, 1H), 7.87 (d, 1H), 7.80 (d, 1H), 7.01 (s, 1H), 6.96 - 6.82 (m, 4H), 4.97 (s, 1H), 4.35 (s, 1H), 3.90 (d, 3H), 1.53 - 1.36 (m, 12H).

### PREPARATION 79

### (S)-1-(3-(2-Methoxyphenyl)-3H-imidazo[4,5-b]pyridin-2-yl)ethanamine

Obtained (0.55g, 100%) from (S)-tert-butyl 1-((3-aminopyridin-2-yl)(2-methoxyphenyl)amino)-1-oxopropan-2-ylcarbamate (0.83g, 2.04 mmol, Preparation 78) following the experimental procedure as described in Preparation 57.
LRMS (m/z): 269 (M+1)⁺.

### PREPARATION 80

### (S)-tert-Butyl 1-oxo-1-(3-(phenylamino)pyridin-2-ylamino)butan-2-ylcarbamate

To a solution of (S)-2-(tert-butoxycarbonylamino)butanoic acid (392 mg, 1.89 mmol) in dichloromethane (15 mL) was added HATU (790 mg, 2.08 mmol) and N,N-dimethylformamide (5 mL). The reaction mixture was stirred at room temperature for 10 min. To the reaction mixture were added N³-phenylpyridine-2,3-diamine (350 mg, 1.89 mmol, Preparation 67) and DIEA (0.3 mL, 1.89 mmol) and the reaction mixture was stirred at room temperature overnight. The reaction mixture was concentrated under reduced pressure, transferred straight onto a silica gel column and was purified by flash chromatography (SQ16, silica gel, heptane/Ethyl acetate) to afford (S)-tert-butyl 1-oxo-1-(3-(phenylamino)pyridin-2-ylamino)butan-2-ylcarbamate (685 mg, 1.71 mmol, 91 % yield) as a colourless sticky solid.

### PREPARATION 81

### (S)-1-(1-Phenyl-1H-imidazo[4,5-b]pyridin-2-yl)propan-1-amine

A solution of (S)-tert-butyl 1-oxo-1-(3-(phenylamino)pyridin-2-ylamino)butan-2-ylcarbamate (685 mg, 1.85 mmol, Preparation 80) in acetic acid (25 mL) was heated to 100 °C and was stirred overnight. The acetic acid was evaporated under reduced pressure. The residue was dissolved in methanol (25 mL), aqueous 6 M HCl (30 mL, 180 mmol) was added and the reaction mixture was stirred at 85 °C overnight. The reaction mixture was left to cool to room temperature. The pH of the reaction mixture was set at ∼8 using solid NaHCO₃ (add slowly due to gas evolution). The mixture was evaporated under reduced pressure to remove the methanol. The aqueous phase was extracted with CH₂Cl₂, the combined organic phases were filtered using a phase separator and finally evaporated to dryness under reduced pressure to afford the title product (332 mg, 1.23 mmol, 35 % yield) as a light brown solid.
LRMS (m/z): 253 (M+1)⁺.

### EXAMPLE 1

### (S)-N-(1-(3-Phenyl-3H-imidazo[4,5-b]pyridin-2-yl)ethyl)-9H-purin-6-amine

To a solution of (S)-1-(3-phenyl-3H-imidazo[4,5-b]pyridin-2-yl)ethanamine (4.62g, 19.39 mmols, Preparation 4) in tert-butanol (185 mL), 6-bromo-9H-purine (3.88g, 19.50 mmols) and DIEA (6.8 mL, 39.04 mmols) were added and it was stirred at 80ºC for 3 days.

The solid obtained was filtered and dried. It was purified by chromatography (Silica gel, DCM:MeOH (7% MeOH 5CV - 9% MeOH 15 CV). It was treated with diethyl ether. The expected compound was obtained (3.44g, 50%).
LRMS (m/z): 357 (M+1)⁺
1H NMR (400 MHz, DMSO-*d*₆) d ppm 1.57 (d, 3 H) 5.52 (m, 1 H) 7.29 (dd, 1 H) 7.40 - 7.71 (m, 5 H) 7.97 - 8.16 (m, 4 H) 8.17 - 8.30 (m, 1 H) 12.92 (s, 1 H)

### EXAMPLE 2

### 9-((3-Phenyl-3H-imidazo[4,5-b]pyridin-2-yl)methyl)-9H-purin-6-amine

K₂CO₃ (0.13 g, 0.93 mmols) was added to a solution of 9H-purin-6-amine (75mg, 0.56 mmols) and 2-(chloromethyl)-3-phenyl-3H-imidazo[4,5-b]pyridine (0.20g, 0.46 mmols, Preparation 5) in DMF (6mL) and it was stirred at room temperature overnight. It was filtered through Celite ® and it was concentrated in vacuo. The residue obtained was purified by reverse phase chromatography. The expected product was obtained (83 mg, 53%).
LRMS (m/z): 343 (M+1)⁺
1H NMR (400 MHz, DMSO-*d*₆) d ppm 5.62 (s, 2H) 7.22 (s, 2 H) 7.32 (dd, 1 H) 7.48 - 7.73 (m, 4 H) 7.96 - 8.13 (m, 2 H) 8.29 (d, 1 H)

### EXAMPLE 3

### 9-(1-(3-Phenyl-3H-imidazo[4,5-b]pyridin-2-yl)ethyl)-9H-purin-6-amine

K₂CO₃ (66mg, 0.48 mmols) was added to a solution of 9H-purin-6-amine (40mg, 0.30 mmols) and 2-(1-chloroethyl)-3-phenyl-3H-imidazo[4,5-b]pyridine (100mg, 0.23 mmols, Preparation 6) in DMF (3mL) and it was stirred at room temperature overnight. It was concentrated in vacuo. The residue obtained was suspended in water and filtered. The solid obtained was washed with diethyl ether (X5) and dried. The expected compound was obtained (49mg, 56%).
LRMS (m/z): 357 (M+1)⁺
1H NMR (400 MHz, DMSO-*d*₆) d ppm 1.90 (d, 3 H) 6.06 (q, 1 H) 7.14 (s, 2 H) 7.26 - 7.38 (m, 3 H) 7.39 - 7.50 (m, 3 H) 7.94 (s, 1 H) 8.05 (s, 1 H) 8.15 (d, 1 H) 8.26 (d, 1 H).

### EXAMPLE 4

### 6-(1-(3-Phenyl-3H-imidazo[4,5-b]pyridin-2-yl)ethylthio)-9H-purine

K₂CO₃ (65 mg, 0.47 mmols) was added to a solution of 9H-purine-6-thiol (45 mg, 0.30 mmols) and 2-(1-chloroethyl)-3-phenyl-3H-imidazo[4,5-b]pyridine (100mg, 0.23 mmols, Preparation 6) in DMF (3 mL) and it was stirred at room temperature overnight. It was concentrated in vacuo. Dichloromethane was added and it was washed with water. The organic phase was dried, filtered and concentrated in vacuo. It was purified by chromatography (Silica gel, DCM:MeOH 10:1). The expected compound was obtained (47 mg, 54%).
LRMS (m/z): 374 (M+1)⁺
1H NMR (400 MHz, DMSO-*d*₆) d ppm 1.88 (d, 3 H) 5.66 (q, 1 H) 7.24 - 7.40 (m, 2 H) 7.42 - 7.64 (m, 4 H) 8.02 - 8.20 (m, 1 H) 8.28 (dd, 1 H) 8.42 (s, 1 H) 8.48 (s, 1 H)

### EXAMPLE 5

### (S)-N-(1-(3-Phenyl-3H-imidazo[4,5-b]pyridin-2-yl)propyl)-9H-purin-6-amine

6-Bromo-9H-purine (80mg, 0.4 mmols) and DIEA (0.14 mL, 0.80 mmols) were added to a solution of (S)-1-(3-phenyl-3H-imidazo[4,5-b]pyridin-2-yl)propan-1-amine (100mg, 0.40 mmols, Preparation 8) in tert-butanol (4mL), and it was stirred at 80ºC overnight. It was concentrated in vacuo. Ethyl acetate was added to the residue and it was washed with water. The aqueous phase was extracted with dichloromethane. The organic phases were collected together, dried, filtered and concentrated in vacuo. It was purified by chromatography (Silica gel, DCM:MeOH 10:1) to afford the expected compound (41 mg, 28%).
LRMS (m/z): 371 (M+1)⁺
1H NMR (400 MHz, CHLOROFORM-*d*) d ppm 0.91 (t, 3 H) 1.87 - 2.38 (m, 2 H) 5.69 (m, 1 H) 6.72 (s, 1 H) 7.48 - 7.66 (m, 5 H) 7.91 (s, 1 H) 7.98 - 8.12 (m, 1 H) 8.22 - 8.41 (m, 2 H) 11.73 (s, 1 H)

### EXAMPLE 6

### (S)-N4-(1-(3-Phenyl-3H-imidazo[4,5-b]pyridin-2-yl)ethyl)pyrimidine-4,6-diamine

6-Bromopyrimidin-4-amine (0.16g, 0.93 mmols) and DIEA (0.3 mL, 1.72 mmols) were added.to a solution of (S)-1-(3-phenyl-3H-imidazo[4,5-b]pyridin-2-yl)ethanamine (0.2g, 0.84 mmols, Preparation 4) in tert-butanol (4mL), It was stirred at 80ºC overnight. Starting material left was mainly detected. Only traces of the expected product were observed. DMF (3 mL) was added and it was heated in the microwave at 180ºC for 10 minutes. Afterwards, it was heated for 20 minutes more. Finally 40 minutes more were

needed to get a crude that was purified by Reverse phase chromatography to afford the expected compound (10 mg, 4%).
LRMS (m/z): 332 (M+1)⁺
1H NMR (400 MHz, DMSO-*d*₆) d ppm 1.36 (d, 3 H) 5.12 (q, 1 H) 5.39 (s, 1 H) 6.00 (s, 2 H) 7.17 (d, 1 H) 7.26 (dd, 1 H) 7.39 - 7.67 (m, 3 H) 7.71 (s, 1 H) 8.02 (dd, 1 H) 8.20 (dd, 1 H) 8.22 - 8.32 (m, 1 H).

### EXAMPLE 7

### (S)-6-(2-(3-Phenyl-3H-imidazo[4,5-b]pyridin-2-yl)pyrrolidin-1-yl)-9H-purine

6-Bromo-9H-purine (60mg, 0.30 mmols) and DIEA (0.1 mL, 0.60 mmols) were added to a solution of (S)-3-phenyl-2-(pyrrolidin-2-yl)-3H-imidazo[4,5-b]pyridine(80mg, 0.30 mmols, Preparation 10) in tert-butanol (3mL) and it was stirred at 80ºC overnight. The precipitate obtained was filtered, suspended in diethyl ether and dried. It was purified by a silica gel cartridge (DCM/MeOH 10:1) to afford the expected compound (56mg, 49%).
LRMS (m/z): 383 (M+1)⁺
1H NMR (400 MHz, DMSO-d₆) d ppm 1.90 - 2.30 (m, 4 H) 3.60 - 3.80 (m, 0.5 H) 4.00 - 4.18 (m, 0.5 H) 4.18 - 4.40 (m, 0.5 H) 4.40 - 4.60 (m, 0.5 H) 5.20 - 5.40 (m, 0.5 H) 6.00 - 6.20 (m, 0.5 H) 7.15- 7.40 (m, 1 H) 7.60 - 7.80 (m, 4 H) 7.80 - 8.15 (m, 3 H) 8.00 - 8.30 (m, 2 H) 12.80 - 12.90 (s, 0.5 H) 12.90 - 13.00 (s, 0.5 H)

### EXAMPLE 8

### (S)-6-(2-(3-Phenyl-3H-imidazo[4,5-b]pyridin-2-yl)pyrrolidin-1-yl)pyrimidin-4-amine

6-Bromopyrimidin-4-amine (53mg, 0.30 mmols) and DIEA (0.1mL, 0.61 mmols) were added to a solution of (S)-3-phenyl-2-(pyrrolidin-2-yl)-3H-imidazo[4,5-b]pyridine (80mg, 0.30 mmols, Preparation 10) in tert-butanol (3 mL) and it was stirred at 80ºC for 5 days. It was concentrated in vacuo and it was purified by chromatography (Silica gel, DCM/MeOH 10:0.5 to 10:1) to afford the expected compound (34mg, 31 %).
LRMS (m/z): 358 (M+1)⁺
1H NMR (400 MHz, DMSO-*d*₆) d ppm 1.74 - 2.13 (m, 2 H) 2.10 - 2.24 (m, 1 H) 2.24 - 2.43 (m, 1 H) 2.90 - 3.22 (m, 1 H) 3.49 - 3.73 (m, 1 H) 4.92 - 5.25 (m, 1 H) 5.36 (s, 1 H) 6.46 (s, 2 H) 7.28 (dd, 1 H) 7.55 - 7.73 (m, 5 H) 7.86 (s, 1 H) 8.02 (d, 1 H) 8.18 - 8.26 (m, 1 H)

### EXAMPLE 9

### (S)-N6-(1-(3-Phenyl-3H-imidazo[4,5-b]pyridin-2-yl)ethyl)-9H-purine-2,6-diamine

6-Chloro-9H-purin-2-amine (0.15g, 0.88 mmols) and triethylamine (0.12mL, 0.86 mmols) were added to a solution of (S)-1-(3-phenyl-3H-imidazo[4,5-b]pyridin-2-yl)ethanamine (0.11 g, 0.46 mmols, Preparation 4) in isopropanol (2.5 mL). The suspension was heated in the microwave at 150ºC for 5h. The mixture was partitioned between saturated aqueous NaHCO₃ and ethyl acetate, the layers were separated, and the aqueous layer was extracted with ethyl acetate. The combined organic layers were dried, filtered and concentrated in vacuo. The crude obtained was purified by reverse phase chromatography to afford the expected product (60 mg, 35%).
LRMS (m/z): 372 (M+1)⁺
1H NMR (600 MHz, DMSO-*d*₆) d ppm 1.46 (d, 3 H) 5.46 (m, 2 H) 5.58 (m, 1 H) 7.31 (dd, 1 H) 7.39 - 7.81 (m, 6 H) 8.08 (d, 1 H) 8.16 - 8.37 (m, 3 H)

### EXAMPLE 10

### (S)-N-(1-(5-Methoxy-3-phenyl-3H-imidazo[4,5-b]pyridin-2-yl)ethyl)-9H-purin-6-amine

Same procedure as described in example 1 was used from (S)-1-(5-methoxy-3-phenyl-3H-imidazo[4,5-b]pyridin-2-yl)ethanamine (0.30 g, 1.12 mmols, Preparation 14). The expected compound was obtained (0.18 g, 41%).
LRMS (m/z): 387 (M+1)⁺
1H NMR (400 MHz, CHLOROFORM-d) d ppm 1.63 (d, 3 H) 3.80 (s, 3 H) 5.50 - 5.98 (m, 1 H) 6.71 (d, 1 H) 6.85 (s, 1 H) 7.35 - 7.71 (m, 5 H) 7.73 - 8.06 (m, 2 H) 8.31 (s, 1 H) 12.48 (s, 1 H)

### EXAMPLE 11

### (S)-N-(1-(3-(3,5-Difluorophenyl)-3H-imidazo[4,5-b]pyridin-2-yl)propyl)-9H-purin-6-amine

Same procedure as described in example 1 was used from (S)-1-(3-(3,5-difluorophenyl)-3H-imidazo[4,5-b]pyridin-2-yl)propan-1-amine (0.15 g, 0.52 mmols, Preparation 18). After 2 days the expected compound was obtained (92 mg, 44%).
LRMS (m/z): 407 (M+1)⁺
1H NMR (400 MHz, DMSO-*d*₆) d ppm 0.93 (t, 3 H) 1.77 - 2.35 (m, 2 H) 5.33 - 5.67 (m, 1 H) 7.16 - 7.56 (m, 4 H) 7.89 - 8.20 (m, 3 H) 8.27 (d, 1 H) 12.91 (s, 1 H)

### EXAMPLE 12

### (S)-N-(1-(3-(3,5-Difluorophenyl)-3H-imidazo[4,5-b]pyridin-2-yl)ethyl)-9H-purin-6-amine

Same procedure as described in example 1 was used from (S)-1-(3-(3,5-difluorophenyl)-3H-imidazo[4,5-b]pyridin-2-yl)ethanamine (0.20g, 0.73 mmols, Preparation 20). After 2 days the expected compound was obtained (90 mg, 31%).
LRMS (m/z): 393 (M+1)⁺
1H NMR (400 MHz, DMSO-*d*₆) d ppm 1.64 (d, 3 H) 5.76 (q, 1 H) 6.91 - 7.57 (m, 4 H) 7.74 - 8.19 (m, 4 H) 8.27 (d, 1 H) 12.89 (s, 1 H)

### EXAMPLE 13

### (S)-N4-(1-(3-Phenyl-3H-imidazo[4,5-b]pyridin-2-yl)ethyl)pyrimidine-2,4,6-triamine

6-Chloropyrimidine-2,4-diamine (61 mg, 0.42 mmols) was added to a solution of (S)-1-(3-phenyl-3H-imidazo[4,5-b]pyridin-2-yl)ethanamine (50 mg, 0.21 mmols, Preparation 4) in ethanol (2 mL). It was heated in the microwave at 150ºC for 10h. It was concentrated in vacuo and purified by reverse phase chromatography. The expected compound was obtained (22 mg, 30%).
LRMS (m/z): 347 (M+1)⁺
1H NMR (400 MHz, DMSO-*d₆*) d ppm 1.36 (d, 3 H) 4.93 (s, 1 H) 5.05 - 5.25 (m, 1 H) 5.40 (s, 2 H) 5.70 (s, 2 H) 6.88 (s, 1 H) 7.30 (dd, 1 H) 7.45 - 7.71 (m, 4 H) 8.06 (d, 1 H) 8.12-8.43 (m, 2 H)

### EXAMPLE 14

### (S)-N-(1-(5-Methoxy-3-phenyl-3H-imidazo[4,5-b]pyridin-2-yl)propyl)-9H-purin-6-amine

Same procedure as described in example 1 was used from (S)-1-(5-methoxy-3-phenyl-3H-imidazo[4,5-b]pyridin-2-yl)propan-1-amine (100 mg, 0.35 mmols, Preparation 22). After 2 days the expected compound was obtained (97 mg, 68%).
LRMS (m/z): 401 (M+1)⁺
1H NMR (400 MHz, DMSO-*d*₆) d ppm 0.81 (t, 3 H) 1.74 - 2.12 (m, 2 H) 3.64 - 3.76 (s, 3 H) 5.20 - 5.54 (m, 1 H) 6.72 (d, 1 H) 7.32 - 7.75 (m, 4 H) 7.71 - 7.89 (m, 1 H) 7.88 - 8.03 (m, 1 H) 8.03 - 8.21 (m, 1 H) 12.92 (s, 1 H)

### EXAMPLE 15

### (S)-N-(1-(3-(3,5-Difluorophenyl)-5-methoxy-3H-imidazo[4,5-b]pyridin-2-yl)ethyl)-9H-purin-6-amine

Same procedure as described in example 1 was used from (S)-1-(3-(3,5-difluorophenyl)-5-methoxy-3H-imidazo[4,5-b]pyridin-2-yl)ethanamine (0.30 g, 0.99 mmols, Preparation 26). After 4 days the expected compound was obtained (82 mg, 20%).
LRMS (m/z): 423 (M+1)⁺
1H NMR (400 MHz, DMSO-*d*₆) d ppm 1.62 (d, 3 H) 3.75 (s, 3 H) 5.47 - 5.83 (m, 1 H) 6.53 - 6.89 (m, 1 H) 7.03 - 7.26 (m, 1 H) 7.32 - 7.57 (m, 2 H) 7.88 - 8.24 (m, 3 H) 12.89 (s, 1 H)

### EXAMPLE 16

### (S)-N-(1-(3-(3,5-Difluorophenyl)-5-methoxy-3H-imidazo[4,5-b]pyridin-2-yl)propyl)-9H-purin-6-amine

Same procedure as described in example 1 was used from (S)-1-(3-(3,5-difluorophenyl)-5-methoxy-3H-imidazo[4,5-b]pyridin-2-yl)propan-1-amine (100 mg, 0.31 mmols, Preparation 28). The expected compound was obtained (60 mg, 44%).
LRMS (m/z): 437 (M+1)⁺
1H NMR (400 MHz, DMSO-*d*₆) d ppm 0.90 (t, 3 H) 1.94 - 2.08 (m, 1 H) 2.05 - 2.23 (m, 1 H) 3.74 (s, 3 H) 5.29 - 5.76 (m, 1 H) 6.75 (d, 1 H) 7.04 - 7.31 (m, 1 H) 7.36 - 7.60 (m, 2 H) 7.90 - 8.28 (m, 3 H) 12.91 (s, 1 H)

### EXAMPLE 17

### 9-((5-Methyl-3-o-tolyl-3H-imidazo[4,5-b]pyridin-2-yl)methyl)-9H-purin-6-amine

Same procedure as described in Example 2 was used from 2-(chloromethyl)-5-methyl-3-o-tolyl-3H-imidazo[4,5-b]pyridine (0.38 g, 1.40 mmols, Preparation 30). The expected compound was obtained (20mg, 4%).
LRMS (m/z): 371 (M+1)⁺
1H NMR (400 MHz, DMSO-*d*₆) d ppm 1.87 (s, 3 H) 2.35 (s, 3 H) 5.40 (d, 1 H) 5.48 (d, 1 H) 6.85 - 7.08 (m, 1 H) 7.07 - 7.33 (m, 2 H) 7.37 - 7.69 (m, 4 H) 7.81 - 8.20 (m, 3 H)

### EXAMPLE 18

### 9-((5-Methoxy-3-phenyl-3H-imidazo[4,5-b]pyridin-2-yl)methyl)-9H-purin-6-amine

Same procedure as described in Example 2 was used from 2-(chloromethyl)-5-methoxy-3-phenyl-3H-imidazo[4,5-b]pyridine (0.25 g, 0.92 mmols, Preparation 31). After 2 days the expected compound was obtained (50 mg, 15%).
LRMS (m/z): 373 (M+1)⁺
1H NMR (400 MHz, DMSO-*d*₆) d ppm 3.80 (s, 3 H) 5.58 (s, 2 H) 6.62 - 6.86 (m, 1 H) 7.20 (s, 1 H) 7.44 - 7.79 (m, 3 H) 7.87 - 8.14 (m, 2 H) 8.24 - 8.58 (m, 2 H)

### EXAMPLE 19

### (S)-4-Amino-6-(1-(3-phenyl-3H-imidazo[4,5-b]pyridin-2-yl)ethylamino)pyrimidine-5-carbonitrile

DIEA (0.19 mL, 1.1 mmol) was added to a solution of (S)-1-(3-phenyl-3H-imidazo[4,5-b]pyridin-2-yl)ethanamine (86 mg, 0.36 mmols, Preparation 4) and 4-amino-6-chloropyrimidine-5-carbonitrile (55 mg, 0.36 mmols) in butanol (3.5 mL). It was stirred at 120ºC for 4h under inert atmosphere. It was concentrated in vacuo. It was purified by chromatography (Silica gel, Ethyl acetate). The expected compound was obtained (60 mg, 47%).
LRMS (m/z): 357 (M+1)⁺
1H NMR (400 MHz, DMSO-d₆) d ppm 1.52 (d, 3 H) 5.51 (m, 1 H) 7.07 - 7.28 (s, 2 H) 7.32 (dd, 1 H) 7.39 - 7.59 (m, 4 H) 7.74 (d, 1 H) 7.87 (s, 1 H) 8.03 - 8.18 (m, 1 H) 8.25 (dd, 1 H)

### EXAMPLE 20

### (S)-N6-(1-(5-Methoxy-3-phenyl-3H-imidazo[4,5-b]pyridin-2-yl)ethyl)-9H-purine-2,6-diamine

Same procedure as described in Example 10 was used from (S)-1-(5-methoxy-3-phenyl-3H-imidazo[4,5-b]pyridin-2-yl)ethanamine (0.10 g, 0.37 mmols, Preparation 14). After 7h the expected compound was obtained (23 mg, 15%).
LRMS (m/z): 402 (M+1)⁺
1H NMR (400 MHz, DMSO-*d*₆) d ppm 1.43 (d, 3 H) 3.75 (s, 3 H) 5.30 - 5.51 (m, 1 H) 5.58 (s, 2 H) 6.60 - 6.86 (m, 1 H) 7.07 - 7.40 (m, 1 H) 7.43 - 7.80 (m, 5 H) 7.86 - 8.12 (m, 1 H) 8.23 (s, 1 H)

### EXAMPLE 21

### (S)-4-Amino-6-(1-(5-methoxy-3-phenyl-3H-imidazo[4,5-b]pyridin-2-yl)ethylamino)pyrimidine-5-carbonitrile

Same procedure as described in Example 20 was used from (S)-1-(5-methoxy-3-phenyl-3H-imidazo[4,5-b]pyridin-2-yl)ethanamine (0.10 g, 0.37 mmols, Preparation 14). After 8h, it was purified by chromatography (Silica gel, dicholoromethane / methanol / ammonia 100:8:1) and the expected compound was obtained (92 mg, 64%).
LRMS (m/z): 387 (M+1)⁺
1H NMR (400 MHz, DMSO-*d*₆) d ppm 1.43 (d, 3 H) 3.75 (s, 3 H) 5.45 (m, 1 H) 6.80 (m, 1 H) 7.20 (s, 2 H) 7.43 - 7.70 (m, 5 H) 7.80 (m, 1 H) 7.92 (s, 1 H) 8.05 (m, 1 H)

### EXAMPLE 22

### (S)-N-(1-(5-Methoxy-3-phenyl-3H-imidazo[4,5-b]pyridin-2-yl)ethyl)pyrazolo[1,5-a]pyrimidin-7-amine

7-Chloropyrazolo[1,5-a]pyrimidine (40 mg, 0.26 mmols, Preparation 32) and DIEA (0.09 mL, 0.52 mmols) were added to a solution of (S)-1-(5-methoxy-3-phenyl-3H-imidazo[4,5-b]pyridin-2-yl)ethanamine (70 mg, 0.26 mmols, Preparation 14) in buthanol (10 mL). It was stirred at 120ºC for 7 h. It was concentrated in vacuo. Ethyl acetate was added and it was washed with water. The organic phase was dried, filtered and concentrated in vacuo. It was purified by chromatography (Silica gel, dichloromethane / methanol / ammonia 100:8:1). The expected compound was obtained (63 mg, 40%).
LRMS (m/z): 386 (M+1)⁺
1H NMR (400 MHz, DMSO-*d*₆) d ppm 1.62 (d, 3 H) 3.72 (s, 3 H) 5.10 - 5.34 (m, 1 H) 5.97 (d, 1 H) 6.42 (d, 1 H) 6.78 (d, 1 H) 7.30 - 7.71 (m, 4 H) 7.85 - 8.23 (m, 3 H)

### EXAMPLE 23

### 2-(1-(9H-Purin-6-ylamino)ethyl)-3-phenyl-3H-imidazo[4,5-b]pyridin-5-ol

Chlorotetramethylsilane (TMSCI, 0.07mL, 0.58 mmols) and sodium iodide (87mg, 0.58 mmols) were added to a solution of (S)-N-(1-(5-methoxy-3-phenyl-3H-imidazo[4,5-b]pyridin-2-yl)ethyl)-9H-purin-6-amine (75 mg, 0.19 mmols, Example 10) in acetonitrile (1.5mL). It was stirred at 85ºC overnight. More TMSCI (0.07 mL, 0.58 mmols) and sodium Iodide (87 mg, 0.58 mmols) were added and it was stirred at 95ºC for 24h. It was concentrated in vacuo and it was purified by chromatography (Silica gel, dichloromethane/methanol/ammonia (100:8:1). The expected compound was obtained (9mg, 13%).
LRMS (m/z): 373 (M+1)⁺
1H NMR (400 MHz, DMSO-*d*₆) d ppm 1.50 (d, 3 H) 5.40 (m, 1 H) 6.55 (m, 1 H) 7.30 - 7.70 (m, 5 H) 7.80 - 8.00 (m, 1 H) 8.10 (s, 1 H) 8.10-8.20 (m, 1 H) 10.70 (s, 1 H) 12.90 (s, 1 H)

### EXAMPLE 24

### (S)-N-(1-(5-Methyl-3-phenyl-3H-imidazo[4,5-b]pyridin-2-yl)ethyl)-9H-purin-6-amine

Same procedure as described in example 1 was used from (S)-1-(5-methyl-3-phenyl-3H-imidazo[4,5-b]pyridin-2-yl)ethanamine (75 mg, 0.30 mmols, Preparation 36). After 36h it was poured onto water/brine 1/1 and extracted with ethyl acetate. It was purified by chromatography (Silica gel, dichloromethane/methanol/ammonia 100:8:1) and the expected compound was obtained (46 mg, 42%).
LRMS (m/z): 371 (M+1)⁺
1H NMR (400 MHz, DMSO-d₆) d ppm 1.48 (d, 3 H) 2.33 - 2.49 (s, 3 H) 5.26 - 5.65 (m, 1 H) 6.97 - 7.26 (m, 1 H) 7.40 - 7.70 (m, 5H) 7.82 - 8.00 (m, 2 H) 8.00 - 8.20 (m, 2H) 12.80 - 13.00 (s, 1 H)

### EXAMPLE 25

### (S)-4-Amino-6-(1-(5-methyl-3-phenyl-3H-imidazo[4,5-b]pyridin-2-yl)ethylamino)pyrimidine-5-carbonitrile

DIEA (0.10 mL, 0.59 mmol) was added to a solution of (S)-1-(5-methyl-3-phenyl-3H-imidazo[4,5-b]pyridin-2-yl)ethanamine (75 mg, 0.30 mmols, Preparation 36) and 4-amino-6-chloropyrimidine-5-carbonitrile (0.18 mg, 0.59 mmols) in butanol (3 mL). It was stirred at 80ºC for 48h under inert atmosphere. It was concentrated in vacuo. Ethyl acetate was added and it was washed with water and brine. It was purified by chromatography (Silica gel, dichloromethane/methanol/ammonia 100:8:1). The expected compound was obtained (20 mg, 18%).
LRMS (m/z): 371 (M+1)⁺
1H NMR (400 MHz, DMSO-*d*₆) d ppm 1.49 (d, 3 H) 2.46 (s, 3 H) 5.29 - 5.59 (m, 1 H) 7.10 - 7.31 (m, 2 H) 7.40 - 7.62 (m, 5 H) 7.71 (d, 1 H) 7.86 (s, 1 H) 7.99 (d, 1 H)

### EXAMPLE 26

### (S)-N-(1-(5-Methyl-3-phenyl-3H-imidazo[4,5-b]pyridin-2-yl)ethyl)pyrazolo[1,5-a]pyrimidin-7-amine

Same procedure as described in example 20 was used from (S)-1-(5-methyl-3-phenyl-3H-imidazo[4,5-b]pyridin-2-yl)ethanamine (75 mg, 0.30 mmols, Preparation 36). In this case, only 2.00 equivalents of DIEA were used. After 8h it was poured onto water and it was extracted with ethyl acetate. It was triturated with hexane and diethyl ether and the expected compound was obtained (78 mg, 71 %).
LRMS (m/z): 370 (M+1)⁺
1H NMR (400 MHz, DMSO-*d*₆) d ppm 1.62 (d, 3 H) 2.44 - 2.45 (s, 3 H) 5.10 - 5.36 (m, 1 H) 5.78 (d, 1 H) 6.42 (d, 1 H) 7.21 (d,1 H) 7.32 - 7.65 (m, 5 H) 7.90 - 8.20 (m, 4 H)

### EXAMPLE 27

### 9-{[3-(2-Methylphenyl)-3H-imidazo[4,5-b]pyridin-2-yl]methyl}-9H-purin-6-amine

Obtained as a solid (71%) from 2-(chloromethyl)-3-o-tolyl-3H-imidazo[4,5-b]pyridine (Preparation 37) and 9H-purine-6-amine following the experimental procedure as described in Preparation 41.
LRMS (m/z): 357 (M+1)⁺.
1H NMR (400 MHz, dmso) δ 7.60 (m, 13H), 5.50 (m, 2H), 1.80 (m, 3H).

### EXAMPLE 28

### 6-{[(3-Phenyl-3H-imidazo[4,5-b]pyridin-2-yl)methyl]thio}-9H-purine

Obtained as a white solid (77%) from 2-(chloromethyl)-3-phenyl-3H-imidazo[4,5-b]pyridine (150 mg, 0.35 mmol, Preparation 5) and 9H-purine-6-thiol (64 mg, 0.42 mmol) following the experimental procedure as described in Example 4.
LRMS (m/z): 360 (M+1)⁺.
1H NMR (400 MHz, dmso) δ 8.5 (s, 1H), 8.40 (s, 1H), 8.30 (m, 1H), 8.1 (m, 1H), 7.60 (m, 5H), 7.30 (dd, 2H), 4.90 (s, 2H).

### EXAMPLE 29

### 6-({[3-(2-Methylphenyl)-3H-imidazo[4,5-b]pyridin-2-yl]methyl}thio)-9H-purine

Obtained as a white solid (73%) from 2-(chloromethyl)-3-o-tolyl-3H-imidazo[4,5-b]pyridine (100 mg, 0.27 mmol, Preparation 37) and 9H-purine-6-thiol (49 mg, 0.32 mmol) following the experimental procedure as described in Example 4.
LRMS (m/z): 374 (M+1)⁺.
1H NMR (400 MHz, dmso) δ 8.5 (s, 1H), 8.40 (s, 1H), 8.20 (d, 1H), 8.1 (d, 1H), 7.50 (d, 1H), 7.30 (m, 4H), 4.80 (m, 2H), 1.9 (s, 3H).

### EXAMPLE 30

### 9-((3-(Piperidin-4-yl)-3H-imidazo[4,5-b]pyridin-2-yl)methyl)-9H-purin-6-amine

Ethyl 4-(2-((6-amino-9H-purin-9-yl)methyl)-3H-imidazo[4,5-b]pyridin-3-yl)piperidine-1-carboxylate (310 mg, 0.63 mmol, Preparation 41) was suspended in 8N NaOH and heated overnight at 100 ºC. After cooling to room temperature, it was neutralized with 6N HCl. 350 mg of a solid were obtained, which were purified by reverse phase chromatography (C-18 silica gel from Waters, water/1:1 acetonitrile-methanol as eluents [0.1% v/v formic acid buffered] 0% to 100%) to give the title compound (30 mg, 11 %) as a solid.
LRMS (m/z): 350 (M+1)⁺.

### EXAMPLE 31

### 9-((3-(1-Methylpiperidin-4-yl)-3H-imidazo[4,5-b]pyridin-2-yl)methyl)-9H-purin-6-amine

9-((3-(Piperidin-4-yl)-3H-imidazo[4,5-b]pyridin-2-yl)methyl)-9H-purin-6-amine (100 mg, 0.29 mmol, Example 30) was dissolved in acetonitrile (2 mL). To this mixture, formaldehyde (37% in water, 16 µL, 0.57 mmol) and sodium cianoborhydride (58 mg, 0.34 mmol) were dropwise added. Then acetic acid (0.2 mL, 2 mmol) was dropwise added and the mixture was stirred overnight at room temperature. The solvents were removed under removed pressure and the crude was purified by reverse phase chromatography (C-18 silica gel from Waters, water/1:1 acetonitrile-methanol as eluents [0.1 % v/v formic acid buffered] 0% to 100%) to give the title compound (7 mg, 7%) as a solid.
LRMS (m/z): 364 (M+1)⁺.

### EXAMPLE 32

### 9-((6-Amino-3-phenyl-3H-imidazo[4,5-b]pyridin-2-yl)methyl)-9H-purin-6-amine

To a screw cap vial was added ethylene glycol (5 mL) followed by copper(I) oxide (0.4 mg, 2.60 µmol) and 9-((6-bromo-3-phenyl-3H-imidazo[4,5-b]pyridin-2-yl)methyl)-9H-purin-6-amine (0.11 g, 0.26 mmol, Preparation 45). The suspension was cooled to 0 °C and ammonia solution, 35% (0.25 mL, 13.00 mmol) was added. The reaction mixture was heated to ∼95 °C and stirred overnight. The reaction mixture was eluted over a SCX column and further eluted using MeOH. The product was flushed off of the

SCX column using ∼2M NH₃ in MeOH and evaporated to dryness under reduced pressure to afford a dark red oil. Purification by prep LCMS afforded 9-((6-amino-3-phenyl-3H-imidazo[4,5-b]pyridin-2-yl)methyl)-9H-purin-6-amine (34 mg, 0.10 mmol, 25 % yield) as an off-white solid.
LRMS (m/z): 358 (M+1)⁺.
1H NMR (400 MHz, dmso) δ 8.08 (s, 1H), 8.04 (s, 1H), 7.74 (s, 1H), 7.60 (m, 4H), 7.53 (m, 1H), 7.30 (bs, 2H), 7.21 (s, 1H), 5.55 (s, 2H), 5.10 (s, 2H).

### EXAMPLE 33

### 9-((6-Methyl-3-phenyl-3H-imidazo[4,5-b]pyridin-2-yl)methyl)-9H-purin-6-amine

To a solution of 9-((6-bromo-3-phenyl-3H-imidazo[4,5-b]pyridin-2-yl)methyl)-9H-purin-6-amine (50 mg, 0.12 mmol, Preparation 45) and methylboronic acid (8 mg, 0.13 mmol) in 1,4-dioxane:Water (4:1) (3 mL) was added K₂CO₃ (82 mg, 0.59 mmol). The reaction mixture was flushed with argon for several minutes. 1,1'-Bis(diphenylphosphino)ferrocene-palladium(II)dichloride (9 mg, 0.01 mmol) was added and the reaction mixture was heated in the microwave for 85 min at 140 °C. Ethyl acetate was added and the mixture was filtered using a phase separator. The organic layer was evaporated to dryness under reduced pressure to afford 135 mg of crude product. Purification by prep LCMS followed by evaporation using a Genevac afforded 9-((6-methyl-3-phenyl-3H-imidazo[4,5-b]pyridin-2-yl)methyl)-9H-purin-6-amine (13 mg, 0.04 mmol, 31 % yield) as a white solid.
LRMS (m/z): 357 (M+1)⁺.
1H NMR (400 MHz, dmso) δ 8.14 (s, 1H), 8.09 (s, 1H), 7.93 (s, 1H) 7.87 (s, 1H), 7.60 (m, 5H), 7.25 (s, 2H), 5.89 (s, 2H), 2.39 (s, 3H).

### EXAMPLE 34

### 9-((5-Amino-3-phenyl-3H-imidazo[4,5-b]pyridin-2-yl)methyl)-9H-purin-6-amine

tert-Butyl 2-((6-amino-9H-purin-9-yl)methyl)-3-phenyl-3Himidazo[4,5-b]pyridin-5-ylcarbamate (75 mg, 0.16 mmol, Preparation 51) was dissolved in trifluoroacetic acid/dichloromethane and stirred at room temperature for 30 min. The reaction mixture was diluted with dichloromethane (25 mL) and washed with sat. NaHCO₃. The organic layer was separated and evaporated to dryness (some solid also precipitated). The organic layer was evaporated, combined with solids and evaporated to dryness. The crude was purified by reverse phase chromatography (C-18 silica gel from Waters,

water/1:1 acetonitrile-methanol as eluents [0.1 % v/v formic acid buffered] 0% to 100%) to give the title compound (12 mg, 20%) as a solid.
LRMS (m/z): 358 (M+1)⁺.
¹H NMR (400 MHz, dmso) δ 8.01 (d, 2H), 7.63 (d, 1H), 7.55 (m, 5H), 7.20 (bs, 2H), 6.39 (d, 1H), 6.03 (bs, 2H), 5.44 (bs, 2H).

### EXAMPLE 35

### 7-((5-Amino-3-phenyl-3H-imidazo[4,5-b]pyridin-2-yl)methyl)-7H-purin-6-amine

Obtained as a by-product of the reaction described in Example 34 from the purification by reverse phase chromatography (C-18 silica gel from Waters, water/1:1 acetonitrile-methanol as eluents [0.1% v/v formic acid buffered] 0% to 100%) to give the title compound (6 mg, 10%) as a solid.
LRMS (m/z): 358 (M+1)⁺.
1H NMR (400 MHz, dmso) δ 8.01 (d, 2H), 7.63 (d, 1H), 7.55 (m, 5H), 7.20 (bs, 2H), 6.39 (d, 1H), 6.03 (bs, 2H), 5.44 (bs, 2H).

### EXAMPLE 36

### 2-((9H-Purin-6-ylthio)methyl)-3-phenyl-3H-imidazo[4,5-b]pyridin-5-amine

Obtained as a white solid (93%) from tert-butyl 2-((9H-purin-6-ylthio)methyl)-3-phenyl-3Himidazo[4,5-b]pyridin-5-ylcarbamate (115 mg, 0.24 mmol, Preparation 52) following the experimental procedure as described in Example 35.
LRMS (m/z): 375 (M+1)⁺.
1H NMR (400 MHz, dmso) δ 13.46 (bs, 1H), 8.55 (s, 1H), 8.43 (s, 1H), 7.66 (d, 1H), 7.47 (s, 4H), 7.45 (d, 1H), 6.40 (d, 1H), 6.03 (bs, 2H), 4.74 (s, 2H).

### EXAMPLE 37

### N-[(1S)-1-(6-Fluoro-3-phenyl-3H-imidazo[4,5-b]pyridin-2-yl)ethyl]-9H-purin-6-amine

To a solution of (S)-1-(6-fluoro-3-phenyl-3H-imidazo[4,5-b]pyridin-2-yl)ethanamine (109 mg, 0.43 mmol, Preparation 57) in 1-butanol (3 mL) was added 6-bromopurine (95 mg, 0.47 mmol) and diisopropylethylamine (DIEA, 0.2 mL, 0.94 mmol) and the reaction mixture was stirred at 85 °C overnight. Analysis showed almost complete conversion to desired product and still some starting material. Additional 6-bromopurine (15 mg, 0.08 mmol) and DIEA (0.1 mL, 0.29 mmol) were added and the reaction mixture was stirred at 85 °C for 4 h. The reaction mixture was evaporated to dryness under reduced pressure. Multiple purifications using preparative liquid chromatography-mass spectra (LCMS) were also insufficient to purifiy the product. Finally, precipitation from MeOH by standing in the freezer over two days followed by filtration and finally drying of the residue using the Genevac afforded (S)-N-(1-(6-fluoro-3-phenyl-3H-imidazo[4,5-b]pyridin-2-yl)ethyl)-9H-purin-6-amine (16 mg, 0.04 mmol, 10 % yield) as an off-white solid.
LRMS (m/z): 375 (M+1)⁺.
1H NMR (400 MHz, MeOD) δ 8.23 (s, 1H), 8.11 (bs, 2H), 7.90 (d, 1H), 7.60 (m, 5H), 5.56 (m, 1H), 4.21 (s, 1H), 1.65 (d, 3H).

### EXAMPLE 38

### N-[(1S)-1-(6-Fluoro-3-phenyl-3H-imidazo[4,5-b]pyridin-2-yl)propyl]-9H-purin-6-amine

Obtained as a white solid from (S)-1-(6-fluoro-3-phenyl-3H-imidazo[4,5-b]pyridin-2-yl)propan-1-amine (150 mg, 0.56 mmol, Preparation 59) following the experimental procedure as described in Example 38.
LRMS (m/z): 389 (M+1)⁺.
1H NMR (400 MHz, MeOD) δ 8.20 (s, 1H), 8.11 (bs, 2H), 7.85 (d, 1H), 7.55 (m, 5H), 5.47 (m, 1H), 2.15 (m, 2H), 1.36 (m, 2H), 0.99 (t, 3H).

### EXAMPLE 39

### N-{(1S)-1-[3-(2-Chlorophenyl)-3H-imidazo[4,5-b]pyridin-2-yl]ethyl}-9H-purin-6-amine

Obtained (51 mg, 24%) as a white solid from (1S)-1-(3-(2-chlorophenyl)-3H-imidazo[4,5-b]pyridin-2-yl)ethanamine (149 mg, 0.55 mmol, Preparation 63) and 6-bromo-9H-purine (108 mg, 0.54 mmol) following the experimental procedure as described in Example 38.
LRMS (m/z): 391 (M+1)⁺.
1H NMR (400 MHz, dmso) δ 12.86 (s, 1H), 8.23 (d, 1H), 8.11 (dd, 2H), 8.05 (s, 1H), 7.67 (d, 2H), 7.47 (s, 1H), 7.32 (dd, 2H), 5.44 (s, 1H), 1.61 (d, 3H).

### EXAMPLE 40

### N-{[3-(2-Chlorophenyl)-3H-imidazo[4,5-b]pyridin-2-yl]methyl}-9H-purin-6-amine

Obtained (68 mg, 30%) as a white solid from (3-(2-chlorophenyl)-3H-imidazo[4,5-b]pyridin-2-yl)methanamine (150 mg, 0.58 mmol, Preparation 65) and 6-bromo-9H-purine (115 mg, 0.58 mmol) following the experimental procedure as described in Example 38.
LRMS (m/z): 377 (M+1)⁺.
1H NMR (400 MHz, dmso) δ 12.70 (s, 1H), 8.23 (dd, 1H), 8.18 - 7.95 (m, 4H), 7.70 (dd, 2H), 7.59 - 7.44 (m, 2H), 7.31 (dd, 1H), 4.77 (s, 2H).

### EXAMPLE 41

### 9-((1-Phenyl-1H-imidazo[4,5-b]pyridin-2-yl)methyl)-9H-purin-6-amine

A mixture of 2-(chloromethyl)-1-phenyl-1H-imidazo[4,5-b]pyridine (39 mg, 0.16 mmol, Preparation 68), adenine (22 mg, 0.16 mmol), potassium carbonate (22 mg, 0.16 mmol) in N,N-dimethylformamide (dry, 2 mL) was stirred at room temperature for 3h. The reaction mixture was filtered and concentrated under reduced pressure to afford crude a dark brown oil (52 mg). Purification by prep LCMS followed by evaporation using a Genevac afforded 9-((1-phenyl-1H-imidazo[4,5-b]pyridin-2-yl)methyl)-9H-purin-6-amine (22 mg, 0.06 mmol, 39 % yield) as a white solid.
LRMS (m/z): 343 (M+1)⁺.
1H NMR (400 MHz, dmso) δ 8.29 (s, 1H), 8.10 - 8.07 (m, 3H), 7.60-7.70 (m, 5H), 7.46 (m, 1H), 7.34 (s, 2H), 5.64 (s, 2H).

### EXAMPLE 42

### 6-((1-Phenyl-1H-imidazo[4,5-b]pyridin-2-yl)methylthio)-9H-purine

A mixture of 2-(chloromethyl)-1-phenyl-1H-imidazo[4,5-b]pyridine (27 mg, 0.11 mmol, Preparation 68), 6-mercaptopurine monohydrate (19 mg, 0.11 mmol), potassium carbonate (17 mg, 0.12 mmol) in N,N-dimethylformamide (dry, 1 mL) was stirred at room temperature overnight. The reaction mixture was evaporated under reduced pressure to afford 85 mg of crude product. Purification by prep LCMS afforded 6-((1-phenyl-1H-imidazo[4,5-b]pyridin-2-yl)methylthio)-9H-purine (22 mg, 0.06 mmol, 55 % yield) as a white solid.
LRMS (m/z): 360 (M+1)⁺.
1H NMR (400 MHz, dmso) δ 13.5 (bs, 1H) 8.54 (s, 1H), 8.43 (s, 1H), 8.29 (d, 1H), 8.10 (d, 1H), 7.55-7.66 (m, 5H), 7.32 (d, 1H), 4.87 (s, 2H).

### EXAMPLE 43

### (S)-N-(1-(1-Phenyl-1H-imidazo[4,5-b]pyridin-2-yl)ethyl)-9H-purin-6-amine

Obtained (170 mg, 60%) as a white solid from (S)-1-(1-phenyl-1H-imidazo[4,5-b]pyridin-2-yl)ethanamine (190 mg, 0.80 mmol, Preparation 71) and 6-bromo-9H-purine (175 mg, 0.88 mmol) following the experimental procedure as described in Example 38.
LRMS (m/z): 357 (M+1)⁺.
1H NMR (400 MHz, dmso) δ 8.42 (dd, 1H), 8.10 (bs, 2H), 7.53-7.61 (m, 6H), 7.30 (m, 1H),5.65(bs, 1H), 1.73 (d, 3H).

### EXAMPLE 44

### (S)-N-(1-(3-(2,6-Difluorophenyl)-3H-imidazo[4,5-b]pyridin-2-yl)ethyl)-9H-purin-6-amine

Obtained (4 mg, 2%) from (S)-1-(3-(2,6-difluorophenyl)-3H-imidazo[4,5-b]pyridin-2-yl)ethanamine (130 mg, 0.38 mmol, Preparation 75) and 6-bromo-9H-purine (100 mg, 0.50 mmol) following the experimental procedure as described in Example 38.
LRMS (m/z): 393 (M+1)⁺.
1H NMR (400 MHz, CD₃OD) δ 8.19 (dd, 1H), 8.07 (dd, 1H), 7.96 (s, 1H), 7.88 (s, 1H), 7.37 (s, 1H), 7.32 (dt, 1H), 7.09 (s, 1H), 6.80 (t, 1H), 5.64 (s, 1H), 3.63 (dt, 1H), 1.73 (d, 1H), 1.27 (d,3H).

### EXAMPLE 45

### N-((1S)-1-(3-(2-Methoxyphenyl)-3H-imidazo[4,5-b]pyridin-2-yl)ethyl)-9H-purin-6-amine

Obtained (90 mg, 44%) from (S)-1-(3-(2-methoxyphenyl)-3H-imidazo[4,5-b]pyridin-2-yl)ethanamine (150 mg, 0.53 mmol, Preparation 79) and 6-bromo-9H-purine (112 mg, 0.56 mmol) following the experimental procedure as described in Example 38.
LRMS (m/z): 387 (M+1)⁺.
1H NMR (400 MHz, dmso) δ 12.88 (s, 1H), 8.20 (s, 1H), 8.08 (dd, 3H), 7.44 (d, 2H), 7.35 - 7.17 (m, 1H), 6.99 (s, 1H), 5.65 (s, 1H), 5.39 (s, 1H), 3.73 (s, 1H), 3.31 (s, 3H), 1.57 (d, 3H).

### EXAMPLE 46

### (S)-N-(1-(1-Phenyl-1H-imidazo[4,5-b]pyridin-2-yl)propyl)-9H-purin-6-amine

To a solution of (S)-1-(1-phenyl-1H-imidazo[4,5-b]pyridin-2-yl)propan-1-amine (127 mg, 0.51 mmol. Preparation 81) in 1-butanol (4 mL) was added 6-bromopurine (113 mg, 0.56 mmol) and DIEA (0.2 mL, 1.11 mmol) and the reaction mixture was stirred at 85 °C overnight. The reaction mixture was evaporated to dryness under reduced pressure. Purification by flash chromatography (SQ16, silica gel, CH₂Cl₂/MeOH, 0-10% MeOH) afforded 137 mg of still impure product. Purification by basic prep LCMS proved insufficient to purify the product. Purification by preparative Thin Layer Chormatography (TLC, CH₂Cl₂/(7 M NH₃ in MeOH), 9:1) afforded (S)-N-(1-(1-phenyl-1H-imidazo[4,5-b]pyridin-2-yl)propyl)-7Hpurin-6-amine (23 mg, 0.06 mmol, 12 % yield) as a white solid.
LRMS (m/z): 371 (M+1)⁺.
1H NMR (400 MHz, dmso) δ 12.75 (bs, 1H) 8.42 (dd, 1H), 8.17 (bs, 1H), 8.03 (bs, 1H), 8.01 (bs, 1H), 7.50-7.70 (m, 6H), 7.24 (m, 1H), 5.39 (bs, 1H), 2.03 (bs, 2H), 0.85 (t, 3H).

### EXAMPLE 47

### N-{(1S)-1-[3-(3-chlorophenyl)-3H-imidazo[4,5-b]pyridin-2-yl]ethyl}-9H-purin-6-amine

### EXAMPLE 48

### N-{(1S)-1-[3-(3-methoxyphenyl)-3H-imidazo[4,5-b]pyridin-2-yl]ethyl}-9H-purin-6-amine

### EXAMPLE 49

### N-[(1S)-1-(3-pyridin-2-yl-3H-imidazo[4,5-b]pyridin-2-yl)ethyl]-9H-purin-6-amine

### EXAMPLE 50

### N-[(1S)-1-(3-pyridin-3-yl-3H-imidazo[4,5-b]pyridin-2-yl)ethyl]-9H-purin-6-amine

### EXAMPLE 51

### N-[(1S)-1-(3-cyclohexyl-3H-imidazo[4,5-b]pyridin-2-yl)ethyl]-9H-purin-6-amine

### EXAMPLE 52

### N-[(1S)-1-(3-benzyl-3H-imidazo[4,5-b]pyridin-2-yl)ethyl]-9H-purin-6-amine

### EXAMPLE 53

### N-{(1S)-1-[3-(3-fluorophenyl)-3H-imidazo[4,5-b]pyridin-2-yl]ethyl}-9H-purin-6-amine

### EXAMPLE 54

### N-((1S)-1-{3-[3-(trifluoromethyl)phenyl]-3H-imidazo[4,5-b]pyridin-2-yl}ethyl)-9H-purin-6-amine

### EXAMPLE 55

### N-{(1S)-1-[3-(3-methylphenyl)-3H-imidazo[4,5-b]pyridin-2-yl]ethyl}-9H-purin-6-amine

### EXAMPLE 56

### 4-{2-[(1S)-1-(9H-purin-6-ylamino)ethyl]-3H-imidazo[4,5-b]pyridin-3-yl}pyridin-2(1 H)-one

### EXAMPLE 57

### N-{(1S)-1-[3-(1,3-thiazol-4-yl)-3H-imidazo[4,5-b]pyridin-2-yl]ethyl}-9H-purin-6-amine

### EXAMPLE 58

### 4-amino-6-({(1S)-1-[3-(3-chlorophenyl)-3H-imidazo[4,5-b]pyridin-2-yl]ethyl}amino)pyrimidine-5-carbonitrile

### EXAMPLE 59

### 4-amino-6-({(1S)-1-[3-(3-methoxyphenyl)-3H-imidazo[4,5-b]pyridin-2-yl]ethyl}amino)pyrimidine-5-carbonitrile

### EXAMPLE 60

### 4-amino-6-{[(1S)-1-(3-pyridin-2-yl-3H-imidazo[4,5-b]pyridin-2-yl)ethyl]amino}pyrimidine-5-carbonitrile

### EXAMPLE 61

### 4-amino-6-{[(1S)-1-(3-pyridin-3-yl-3H-imidazo[4,5-b]pyridin-2-yl)ethyl]amino}pyrimidine-5-carbonitrile

### EXAMPLE 62

### 4-amino-6-{[(1S)-1-(3-cyclohexyl-3H-imidazo[4,5-b]pyridin-2-yl)ethyl]aminolpyrimidine-5-carbonitrile

### EXAMPLE 63

### 4-amino-6-1[(1S)-1-(3-benzyl-3H-imidazo[4,5-b]pyridin-2-yl)ethyl]aminolpyrimidine-5-carbonitrile

### EXAMPLE 64

### 4-amino-6-({(1S)-1-[3-(3-fluorophenyl)-3H-imidazo[4,5-b]pyridin-2-yl]ethyl}amino)pyrimidine-5-carbonitrile

### EXAMPLE 65

### 4-amino-6-[((1S)-1-{3-[3-(trifluoromethyl)phenyl]-3H-imidazo[4,5-b]pyridin-2-yl}ethyl)amino]pyrimidine-5-carbonitrile

### EXAMPLE 66

### 4-amino-6-({(1S)-1-[3-(3-methylphenyl)-3H-imidazo[4,5-b]pyridin-2-yl]ethyl}amino)pyrimidine-5-carbonitrile

### EXAMPLE 67

### 4-amino-6-({(1S)-1-[3-(2-oxo-1,2-dihydropyridin-4-yl)-3H-imidazo[4,5-b]pyridin-2-yl]ethyl}amino)pyrimidine-5-carbonitrile

### EXAMPLE 68

### 4-amino-6-({(1S)-1-[3-(1,3-thiazol-4-yl)-3H-imidazo[4,5-b]pyridin-2-yl]ethyl}amino)pyrimidine-5-carbonitrile

### EXAMPLE 69

### N-{(1S)-1-[5-(methylamino)-3-phenyl-3H-imidazo[4,5-b]pyridin-2-yl]ethyl}-9H-purin-6-amine

### EXAMPLE 70

### 4-amino-6-({(1S)-1-[5-(methylamino)-3-phenyl-3H-imidazo[4,5-b]pyridin-2-yl]ethyl}amino)pyrimidine-5-carbonitrile

### EXAMPLE 71

### N-[(1S)-1-(5-ethoxy-3-phenyl-3H-imidazo[4,5-b]pyridin-2-yl)ethyl]-9H-purin-6-amine

### EXAMPLE 72

### 4-amino-6-{[(1S)-1-(5-ethoxy-3-phenyl-3H-imidazo[4,5-b]pyridin-2-yl)ethyl]amino}pyrimidine-5-carbonitrile

### EXAMPLE 73

### N-[(1S)-1-(6-fluoro-5-methoxy-3-phenyl-3H-imidazo[4,5-b]pyridin-2-yl)ethyl]-9H-purin-6-amine

### EXAMPLE 74

### 4-amino-6-{[(1S)-1-(6-fluoro-5-methoxy-3-phenyl-3H-imidazo[4,5-b]pyridin-2-yl)ethyl]amino}pyrimidine-5-carbonitrile

### EXAMPLE 75

### N-[(1S)-1-(5-methoxy-3-phenyl-3H-imidazo[4,5-b]pyridin-2-yl)ethyl]-N-methyl-9H-purin-6-amine

### EXAMPLE 76

### 4-amino-6-[[(1S)-1-(5-methoxy-3-phenyl-3H-imidazo[4,5-b]pyridin-2-yl)ethyl](methyl)amino]pyrimidine-5-carbonitrile

### EXAMPLE 77

### N-[(1S)-1-(7-methyl-3-phenyl-3H-imidazo[4,5-b]pyridin-2-yl)ethyl]-9H-purin-6-amine

### EXAMPLE 78

### 4-amino-6-{[(1S)-1-(7-methyl-3-phenyl-3H-imidazo[4,5-b]pyridin-2-yl)ethyl]amino}pyrimidine-5-carbonitrile

### REFERENCES

1.- Tehrani, A. K.; Borremans D.; De Kimpe N. Tetrahedron 1999, 55, 4133-4152
2.- Ohta, T.; Fukuda, T.; Ishibashi, F., Iwao, M. J. Org. Chem. 2009, 74, 8143-8153

### PHARMACOLOGICAL ACTIVITY

### PI3K α, β, δ and y Enzymatic Inhibition Assays

Compounds were screened for their ability to inhibit PI3Kα (PI3Ka), PI3Kβ (PI3Kb), PI3Kδ (PI3Kd) and PI3Kγ (PI3Kg) using a cell-free based P13K HTRF^{™} assay (Millipore, ref. #33-017) All reagents to perform the reactions were prepared according to the manufacturer protocol. All PI3K enzymes were recombinant and were purchased at Millipore.

All four assays were performed according to the following procedure:
1) Dilution curves of compounds were done in 100% DMSO and dispensed in a reservoir plate, typically from column 2 to 11. Column 1 and 12 were used for the negative controls (100% inhibition using a reference PI3K inhibitor for the four isoforms) and the positive controls (0% inhibition using dimethyl sulfoxide (DMSO) only).
2) Mix of Phosphoinositide 3-kinase (PI3K) + Phosphatidylinositol 4,5-bisphosphate (PIP2) was diluted in buffer (supplied with the kit) and plated in a medium binding black 96-well plate (Greiner ref. #675076) PI3Ka was diluted at 0.25 nM with PIP2 at 2 µM; PI3Kb was diluted at 0.50 nM with PIP2 at 5 µM; PI3Kd was diluted at 0.60 nM with PIP2 at 2 µM and PI3Kg was diluted at 0.30 nM with PIP2 at 10 µM. These concentrations were final in the assay.

3) A reservoir plate containing Adenosine TriPhosphate (ATP) diluted in the Millipore kit buffer was prepared for each isoform. The final concentration of ATP in the assay was 10 µM, 15 µM, 20 µM and 10 µM for PI3Ka, PI3Kb, PI3Kd and PI3Kg respectively.
4) Reactions were started by addition to the PI3K+PIP2 plates of the ATP and compounds simultaneously. Incubation was done for 8 minutes (for all four isoforms) then the reaction was stopped by addition of the Stop solution. The Detection solution was added. These solutions were prepared previously according to the kit specifications. The plates were then incubated overnight at RT before reading the signal in an Envision (PerkinElmer), with excitation at 340 nm and emissions at 620 and 665 nm.
5) Data obtained from the compound curves were normalized in respect to the negative and positive controls, and then fitted by a 4-parameter log curve in ActivityBase (IDBS) in order to determine their potency.

The results are shown in Table 1.

| **Example** | **IC₅₀ PI3Kd HTRF (nM)** |
|---|---|
| **9** | 85.9 |
| **10** | 31.6 |
| **12** | 188.3 |
| **14** | 14.4 |
| **15** | 10.9 |
| **19** | 132.2 |
| **21** | 22.2 |
| **25** | 52.1 |
| **26** | 693.0 |
| **37** | 165.2 |
| **39** | 215.1 |
| **43** | 1804.7 |

It can be seen from Table 1 that the compounds of formula (I) are potent inhibitors of Phosphoinositide 3-kinase delta (PI3kd). Preferred compounds of the invention possess an IC₅₀ value for the inhibition of PI3Kd (determined as defined above) of less than 10 µM (10,000 nM), preferably less than 1 µM (1,000 nM), even more preferably of less than 0.2 µM (200 nM), most preferably less than 0.05 µM (50 nM)

The invention is also directed to a compound of the invention as described herein for use in the treatment of the human or animal body by therapy. Compounds of the invention intended for pharmaceutical use may be administered as crystalline or amorphous products, or mixtures thereof. They may be obtained, for example, as solid plugs, powders, or films by methods such as precipitation, crystallization, freeze drying, spray drying, or evaporative drying. Microwave or radio frequency drying may be used for this purpose.

### Combinations

The imidazopyridine derivatives defined herein may also be combined with other active compounds in the treatment of a pathological condition or disease susceptible to amelioration by inhibition of PI3Ks.

The combinations of the invention can optionally comprise one or more additional active substances which are known to be useful in the treatment of respiratory diseases; allergic diseases; inflammatory or autoimmune-mediated diseases; function disorders and neurological disorders; cardiovascular diseases; viral infection; metabolism/endocrine function disorders; neurological disorders and pain; bone marrow and organ transplant rejection; myelo-dysplastic syndrome; myeloproliferative disorders (MPDs); cancer and hematologic malignancies, leukemia, lymphomas and solid tumors.

Particularly, the combinations of the invention can optionally comprise one or more additional active substances which are known to be useful in the treatment of neoplastic diseases (e.g. leukemia, lymphomas, solid tumors); transplant rejection, bone marrow transplant applications (e.g., graft- versus-host disease); autoimmune diseases (e.g. rheumatoid arthritis, multiple sclerosis, amyotrophic lateral sclerosis, Crohn's disease, ulcerative colitis, systemic lupus erythematosis, autoimmune hemolytic anemia, type I diabetes); respiratory inflammation diseases (e.g. asthma, chronic obstructive pulmonary disease, cystic fibrosis, idiopathic pulmonary fibrosis, sarcoidosis); allergic diseases (e.g. allergic rhinitis); skin inflammatory diseases (e.g., atopic dermatitis, contact dermatitis, eczema or psoriasis); premalignant and malignant skin conditions (e.g. basal cell carcinoma (BCC), squamous cell carcinoma (SCC) or actinic keratosis (AK)); neurological disorders and pain (such as pain associated with rheumatoid arthritis or osteoarthritis, back pain, general inflammatory pain, inflammatory neuropathic pain, trigeminal neuralgia or central pain)

Preferably, the combinations of the invention can optionally comprise one or more additional active substances which are known to be useful in the treatment of neoplastic diseases leukemia, lymphomas and solid tumors, rheumatoid arthritis, multiple sclerosis, amyotrophic lateral sclerosis, Crohn's disease, ulcerative colitis, systemic lupus erythematosis, autoimmune hemolytic anemia, type I diabetes, asthma, chronic obstructive pulmonary disease, cystic fibrosis, idiopathic pulmonary fibrosis, sarcoidosis, allergic rhinitis, atopic dermatitis, contact dermatitis, eczema, psoriasis, basal cell carcinoma, squamous cell carcinoma and actinic keratosis.

The combinations of the invention comprise (i) a compound of the invention as defined above; and (ii) another compound selected from the group consisting of an Adenoside A_{2A} agonist, an agent for treating cardiovascular disorders, an agent for treating diabetes, and an agent for treating liver disease, an anti-allergic agent, an anti-cholinergic agent, an anti-inflammatory agent, an anti-infective agent, a β2-adrenergic agonist, a Chemoattractant receptor homologous molecule expressed on TH₂ cells (CRTH2) inhibitor, a chemotherapeutic agent, a corticosteroid, an IKKβ/IKBKB (IkB kinase beta or IKK2) inhibitor, an immunosuppressant, a Janus kinase (JAK) inhibitor, a topically acting p38 Mitogen-Activated Protein Kinase (p38 MAPK) inhibitor, a Phosphosdiesterase (PDE) IV inhibitor, and a Spleen tyrosine kinase (Syk) inhibitor, for simultaneous, separate or sequential use in the treatment of the human or animal body.

In a particular embodiment, the combinations of the invention can optionally comprise one or more additional active substances selected from
a) Dyhydrofolate reductase inhibitors, such as Methotrexate or CH-1504;
b) Dihydroorotate dehydrogenase (DHODH) inhibitors such as leflunomide, teriflunomide, or the compounds described in the International Patent Application Nos. WO2008/077639 and WO2009/021696;
c) Immunomodulators such as Glatiramer acetate (Copaxone), Laquinimod or Imiquimod;
d) Inhibitors of DNA synthesis and repair, such as Mitoxantrone or Cladribine;
e) Immunosuppressants, such as Imuran (azathioprine) or Purinethol (6-mercaptopurine or 6-MP);
f) Anti-alpha 4 integrin antibodies, such as Natalizumab (Tysabri) ;
g) Alpha 4 integrin antagonists such as R-1295 , TBC-4746, CDP-323, ELND-002, Firategrast or TMC-2003;
*h)* Corticoids and glucocorticoids such as prednisone or methylprednisolone, fluticasone, mometasone, budesonide, ciclesonide or beta-metasone;
*i)* Fumaric acid esters, such as *BG-12;*
j) Anti-tumor necrosis factor-alpha (Anti-TNF-alpha) monoclonal antibodies such as Infliximab, Adalimumab or Certolizumab pegol;
k) Soluble Tumor necrosis factor-alpha (TNF-alpha) Antagonists such as Ethanercept;
l) Anti-CD20 (lymphocyte protein) monoclonal antibodies such as Rituximab, Ocrelizumab Ofatumumab or TRU-015;
m) Anti-CD52 (lymphocyte protein) monoclonal antibodies such as alemtuzumab;
n) Anti-CD25 (lymphocyte protein) such as daclizumab;
o) Anti-CD88 (lymphocyte protein), such as eculizumab or pexilizumab;
p) Anti-Interleukin 6 Receptor (IL-6R), such as tocilizumab;
q) Anti-Interleukin 12 Receptor (IL-12R) / Interleukin 23 Receptor (IL-23R), such as ustekinumab;
r) Calcineurin inhibitors such as cyclosporine A or tacrolimus;
s) Inosine-monophosphate dehydrogenase (IMPDH) inhibitors, such as mycophenolate mophetyl, ribavirin, mizoribine or mycophenolic acid;
t) Cannabinoid receptor agonists such as Sativex;
u) Chemokine CCR1 antagonists such as MLN-3897 or PS-031291; v) Chemokine CCR2 antagonists such as INCB-8696;
w) Necrosis factor-kappaB (NF-kappaB or NFKB) Activation Inhibitors such as Sulfasalazine, Iguratimod or MLN-0415;
x) Adenosine A_{2A} agonists, such as ATL-313, ATL-146e, CGS-21680, Regadenoson or UK-432,097;
y) Sphingosine-1 (S1 P) phosphate receptor agonists such as fingolimod, BAF-312, or ACT128800;
z) Sphingosine-1 (S1 P) liase inhibitors such as LX2931;
aa) Spleen tyrosine kinase (Syk) inhibitors, such as R-112;
bb) Protein Kinase Inhibitors (PKC) inhibitors, such as NVP-AEB071; cc) Anti-cholinergic agents such as tiotropium or aclidinium;
dd) Beta adrenergic agonists such as formoterol, indacaterol or LAS100977;
ee) MABA (molecules with dual activity: beta-adrenergic agonists and muscarinic receptor antagonists)
ff) Histamine 1 (H1) receptor antagonists, such as azelastine or ebastine;
gg) Cysteinyl leukotriene (CysLT) receptor antagonists, such as montelukast;
hh) Mast cell stabilizers, such as nedocromil or chromoglycate;
ii) 5-lipoxygenase-activating protein (FLAP) inhibitors, such as MK886 or BAY X 1005;
jj) 5-lipoxygenase (5-LO) inhibitors, such as WY-50295T;
kk) Chemoattractant receptor homologous molecule expressed on TH₂ cells (CRTH2) inhibitors, such as OC-459, AZD-1981, ACT-129968, QAV-680;
ll) Vitamin D derivatives like calcipotriol (Daivonex) ;
mm) Anti-inflammatory agents, such as non-steroidal anti-inflammatory drugs (NSAIDs) or selective cyclooxygenase-2 (COX-2) inhibitors such as aceclofenac, diclofenac, ibuprofen, naproxen, apricoxib, celecoxib, cimicoxib, deracoxib, etoricoxib, lumiracoxib, parecoxib sodium, rofecoxib, selenocoxib-1 or valdecoxib;
nn) Anti-allergic agents;
oo) Anti-viral agents;
pp) Phosphodiestearase (PDE) III inhibitors;
qq) Phosphosdiesterase (PDE) IV inhibitors such as roflumilast or GRC-4039;
rr) Dual Phosphodiestearase (PDE) III/IV inhibitors;
ss) Xanthine derivatives, such as theophylline or theobromine;
tt) p38 Mitogen-Activated Protein Kinase (p38 MAPK) Inhibitors such as ARRY-797;
uu) Mitogen-activated extracellular signal regulated kinase kinase (MEK) inhibitor, such as ARRY-142886 or ARRY-438162;
vv) Janus kinase (JAK) inhibitors, such as tofacitinib (previously known as tasocitinib or CP-690,550) from Pfizer and INCB-18424, from Incyte;
ww) Interferons comprising Interferon beta 1 a such as Avonex from Biogen Idec, CinnoVex from CinnaGen and Rebif from EMD Serono, and Interferon beta 1 b such as Betaferon from Schering and Betaseron from Berlex;
xx) Interferon alpha such as Sumiferon MP;
yy) Epidermal Growth Factor Receptor (EGFR) inhibitors such as erlotinib, Trastuzumab, Herceptin, Avastin, Platins (cisplatin, carboplatin) or Temazolamide;
zz) Antineoplastic agents such as Docetaxel, Estramustine, Anthracyc lines, (doxorubicin (Adriamycin), epirubicin (Ellence), and liposomal doxorubicin (Doxil)), Taxanes (docetaxel (Taxotere), paclitaxel (Taxol), and protein-bound paclitaxel (Abraxane)), Cyclophosphamide (Cytoxan), Capecitabine (Xeloda), 5 fluorouracil (5 FU), Gemcitabine (Gemzar) or Vinorelbine (Navelbine);

Specific examples of suitable corticoids and glucocorticoids that can be combined with the PI3K inhibitors of the present invention are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RPR-106541, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, hydrocortisone acetate, hydrocortisone sodium succinate, prednisolone sodium phosphate and hydrocortisone probutate.

Specific examples of suitable Syk kinase inhibitors that can be combined with the PI3K inhibitors of the present invention are fosfamatinib (from Rigel), R-348 (from Rigel), R-343 (from Rigel), R-112 (from Rigel), piceatannol, 2-(2-Aminoethylamino)-4-[3-(trifluoromethyl)phenylamino] pyrimidine-5-carboxamide, R-091 (from Rigel), 6-[5-Fluoro-2-(3,4,5-trimethoxyphenylamino)pyrimidin-4-ylamino]-2,2-dimethyl-3,4-dihydro-2H-pyrido[3,2-b][1,4]oxazin-3-one benzenesulfonate (R-406 from Rigel), 1-(2,4,6-Trihydroxyphenyl)-2-(4-methoxyphenyl)ethan-1-one, N-[4-[6-(Cyclobutylamino)-9H-purin-2-ylamino]phenyl]-N-methylacetamide (QAB-205 from Novartis), 2-[7-(3,4-Dimethoxyphenyl)imidazo[1,2-c]pyrimidin-5-ylamino]pyridine-3-carboxamide dihydrochloride (BAY-61-3606 from Bayer) and AVE-0950 (from Sanofi-Aventis).

Specific examples of suitable M3 antagonists (anticholinergics) that can be combined with the PI3K inhibitors of the present invention are tiotropium salts, oxitropium salts, flutropium salts, ipratropium salts, glycopyrronium salts, trospium salts, zamifenacin, revatropate, espatropate, darotropium bromide, Cl-923, NPC-14695, BEA-2108, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1 (R)-cyclopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1(R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-yl]-1-methyl-1-[2-oxo-2-(3-thienyl)ethyl]pyrrolidinium iodide, N-[1-(3-Hydroxybenzyl)-1-methylpiperidinium-3(S)-yl]-N-[N-[4-(isopropoxycarbonyl)phenyl]carbamoyl]-L-tyrosinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 3(R)-[4,4-Bis(4-fluorophenyl)-2-oxoimidazolidin-1-yl]-1-methyl-1-(2-phenylethyl)pyrrolidinium iodide, trans-4-[2-[Hydroxy-2,2-(dithien-2-yl)acetoxy]-1-methyl-1-(2-phenoxyethyl)piperidinium bromide from Novartis (412682), 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Specific examples of suitable beta adrenergic agonists (β2-agonists) that can be combined with the PI3K inhibitors of the present invention are are terbutaline sulphate, eformoterol fumarate, formoterol fumarate, bambuterol, ibuterol, isoprenaline hydrochloride, dopexamine, metaprotenerol, tulobuterol, procaterol hydrochloride, sibenadet hydrochloride, mabuterol hydrochloride, albuterol sulphate, salbutamol sulphate, salmefamol, salmeterol xinafoate, carmoterol hydrochloride, (R)-albuterol hydrochloride, Levalbuterol hydrochloride; Levosalbutamol hydrochloride; (-)-Salbutamol hydrochloride, formoterol, (R,R)-Formoterol tartrate; Arformoterol tartrate, sulfonterol, Bedoradrine sulphate, Indacaterol, Trantinterol hydrochloride, Milveterol hydrochloride, Olodaterol, fenoterol hydrobromide, rimoterol hydrobromide, riproterol hydrochloride, Vilanterol broxaterol, pirbuterol hydrochloride, bitolterol mesylate, clenbuterol hydrochloride, AZD-3199, GSK-159802; GSK-597901, GSK-678007, GSK-961081; 4-[2-[3-(1H-Benzimidazol-1-yl)-1,1-dimethylpropylamino]-1-hydroxyethyl]-2-(4-methoxybenzylamino)phenol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-domethoxyphenyl)-2-methyl-2-propylamino]ethanol, 1-[2H-5-hydroxy-3-oxo-4H-1,4-benzoxazin-8-yl]-2-[3-(4-n-butyloxyhenyl)-2-methyl-2-propylamino]ethanol, KUL-1248, HOKU-81, SM-110444, RP-58802B, LAS 100977 and compounds described in PCT patent applications Nos. WO 2007/124898, WO 2006/122788A1, WO 2008/046598, WO 2008095720, WO 2009/068177 and WO 2010/072354.

Specific examples of suitable anti-allergic agents that can be combined with the PI3K inhibitors of the present invention are anti-histamines (e.g. Methapyrilene, Mequitazine, Azelastine hydrochloride, Acrivastine, Emedastine difumarate, Emedastine fumarate, Loratadine, Cyproheptadine hydrochloride, Diphenhydramine hydrochloride, Doxepin hydrochloride, Promethazine hydrochloride, Levocabastine hydrochloride, Desloratadine, Cinnarizine, Setastine hydrochloride, Mizolastine, Ebastine, Cetirizine hydrochloride, Epinastine hydrochloride, Olopatadine hydrochloride, Bepotastine besilate,Triprolidine hydrochloride, Rupatadine fumarate, Fexofenadine hydrochloride, Levocetirizine dihydrochloride, Ketotifen, Azatadine maleate, Dimethindene maleate, Clemastine fumarate, Alcaftadine, Bilastine, Vapitadine hydrochloride, AZD-1744, GSK-1004723D, GSK-835726 or SUN-1334H.

Specific examples of suitable Phosphosdiesterase IV (PDE IV) inhibitors that can be combined with the PI3K inhibitors of the present invention are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, tetomilast, filaminast, (R)-(+)-4-[2-(3-Cyclopentyloxy-4-methoxyphenyl)-2-phenylethyl]pyridine (CDP-840), N-(3,5-Dichloro-4-pyridinyl)-2-[1-(4-fluorobenzyl)-5-hydroxy-1H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide (D-4418), 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsulfonyl]-4-(3-methoxyphenylamino)-8-methylquinoline-3-carboxamide hydrochloride (GSK-256066), 4-[6,7-Diethoxy-2,3-bis(hydroxymethyl)naphthalen-1-yl]-1-(2-methoxyethyl)pyridin-2(1H)-one (T-440), (-)-trans-2-[3'-[3-(N-Cyclopropylcarbamoyl)-4-oxo-1,4-dihydro-1,8-naphthyridin-1-yl]-3-fluorobiphenyl-4-yl]cyclopropanecarboxylic acid, MK-0873, CDC-801, UK-500001, BLX-914, 2-carbomethoxy-4-cyano-4-(3-cyclopropylmethoxy-4-difluroromethoxyphenyl)cyclohexanl-one, cis [4-cyano-4-(3-cyclopropylmethoxy-4-difluoromethoxyphenyl)cyclohexan-1-ol, 5(S)-[3-(Cyclopentyloxy)-4-methoxyphenyl]-3(S)-(3-methylbenzyl)piperidin-2-one (IPL-455903), ONO-6126 (Eur Respir J 2003, 22(Suppl. 45): Abst 2557) and the compounds claimed in the PCT patent applications number WO 03/097613, WO 2004/058729, WO 2005/049581, WO 2005/123693, WO 2005/123692, and WO 2010/069504.

Specific examples of suitable immunosupressants that can be combined with the PI3K inhibitors of the present invention are picremolimus, tacrolimus, cyclosporine A, leflunomide, teriflunomide, vidofludimus, laquinimod, methotrexate, 5-fluorouracil (5-FU), anti-TNF agents and compounds described in PCT patent applications Nos. WO 2008/077639, WO 2009/021696, WO 2009/153043, and WO2010083975 (in particular amino(iso)nicotinic acid derivatives selected from the group consisting of 2-(3'-ethoxy-3-(trifluoromethoxy)biphenyl-4-ylamino)nicotinic acid, 2-(3,5-difluoro-3'-methoxybiphenyl-4-ylamino)nicotinic acid and 2-(3,5-difluoro-2-methylbiphenyl-4-ylamino)nicotinic acid; and azabiphenylaminobenzoic acid derivatives selected from the group consisting of 5-cyclopropyl-2-(2-(2,6-difluorophenyl)pyrimidin-5-ylamino)benzoic acid, 5-cyclopropyl-2-((2-(2-(trifluoromethyl)phenyl)pyrimidin-5-yl)amino)benzoic acid and 5-methyl-2-((6-(2,3-difluorophenyl)pyridin-3-yl)amino)benzoic acid)

Specific examples of suitable anti-infectives that can be combined with the PI3K inhibitors of the present invention are aclarubicin, actinomycin D, amrubicin, annamycin, adhamycin, bleomycin, daunorubicin, doxorubicin, elsamitrucin, epirubicin, galarubicin, idarubicin, mitomycin C, mupiricin, nemorubicin, neocarzinostatin, peplomycin, pirarubicin, rebeccamycin, retapamulin, stimalamer, streptozocin, valrubicin, zinostatin, amphotericin B, bifonazole, caspofungin, clotrimazole, echinocandin B, econazole, fluconazole, flucytosine, itraconazole, ketoconazole, miconazole, posaconazole, ravuconazole, terbinafine, tioconazole, voriconazole and combinations thereof.

Particularly preferred combination products according to the invention comprise a compound of formula (I) and a therapeutically effective amount of one or more additional therapeutic agents selected from the group consisting of mometasone furoate, ciclesonide, budesonide, fluticasone propionate, fluticasone furoate, betamethasone valerate, clobetasol propionate, tiotropium salts, glycopyrronium salts, 3-[2-Hydroxy-2,2-bis(2-thienyl)acetoxy]-1-(3-phenoxypropyl)-1-azoniabicyclo[2.2.2]octane salts (in particular aclidinium salts, preferably aclidinium bromide), 1-(2-Phenylethyl)-3-(9H-xanthen-9-ylcarbonyloxy)-1-azoniabicyclo[2.2.2]octane salts, formoterol, salmeterol, indacaterol, carmoterol, LAS 100977, compounds described in PCT patent applications Nos. WO 2008/077639, WO 2009/021696, WO 2009/153043, and WO 2010/083975 (in particular amino(iso)nicotinic acid derivatives selected from the group consisting of 2-(3'-ethoxy-3-(trifluoromethoxy)biphenyl-4-ylamino)nicotinic acid, 2-(3,5-difluoro-3'-methoxybiphenyl-4-ylamino)nicotinic acid and 2-(3,5-difluoro-2-methylbiphenyl-4-ylamino)nicotinic acid; and azabiphenylaminobenzoic acid derivatives selected from the group consisting of 5-cyclopropyl-2-(2-(2,6-difluorophenyl)pyrimidin-5-ylamino)benzoic acid, 5-cyclopropyl-2-((2-(2-(trifluoromethyl)phenyl)pyrimidin-5-yl)amino)benzoic acid and 5-methyl-2-((6-(2,3-difluorophenyl)pyridin-3-yl)amino)benzoic acid), methapyrilene, cetirizine, loratadine, ebastine, desloratadine, fexofenadine, azelastine, levocabastine, olopatadine, Montelukast, picremolimus, tacrolimus, mupiricin, retapamulin, clotrimazole, ketoconazole and terbinafine.

The compounds of formula (I) and the combinations of the invention may be used in the treatment of respiratory diseases; allergic diseases; inflammatory or autoimmune-mediated diseases; function disorders and neurological disorders; cardiovascular diseases; viral infection; metabolism/endocrine function disorders; neurological disorders and pain; bone marrow and organ transplant rejection; myelo-dysplastic syndrome; myeloproliferative disorders (MPDs such as polycythemia vera, essential thrombocythemia or mielofibrosis); cancer and hematologic malignancies, leukemia, lymphomas and solid tumors, wherein the use of a PI3K inhibitor is expected to have a beneficial effect, for example leukemia, lymphomas and solid tumors, rheumatoid arthritis, multiple sclerosis, amyotrophic lateral sclerosis, Crohn's disease, ulcerative colitis, systemic lupus erythematosis, autoimmune hemolytic anemia, type I diabetes, asthma, chronic obstructive pulmonary disease, cystic fibrosis, idiopathic pulmonary fibrosis, sarcoidosis, allergic rhinitis, atopic dermatitis, contact dermatitis, eczema, psoriasis, basal cell carcinoma, squamous cell carcinoma and actinic keratosis.

The active compounds in the combination product may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be administered in the morning and the other (s) later in the day. Or in another scenario, one or two actives could be administered twice daily and the other (s) once daily, either at the same time as one of the twice-a-day dosing occurred, or separately. Preferably at least two, and more preferably all, of the actives would be administered together at the same time. Preferably, at least two, and more preferably all actives would be administered as an admixture.

The invention is also directed to a combination product of the compounds of the invention together with one or more other therapeutic agents for use in the treatment of a pathological condition or disease susceptible to amelioration by inhibiton of Phosphoinositide 3-Kinases (PI3Ks), in particular wherein the pathological condition or disease is selected from respiratory diseases; allergic diseases; inflammatory or autoimmune-mediated diseases; function disorders and neurological disorders; cardiovascular diseases; viral infection; metabolism/endocrine function disorders; neurological disorders and pain; bone marrow and organ transplant rejection; myelo-dysplastic syndrome; myeloproliferative disorders (MPDs such as polycythemia vera, essential thrombocythemia or mielofibrosis); cancer and hematologic malignancies, leukemia, lymphomas and solid tumors; more in particular wherein the pathological condition or disease is selected from leukemia, lymphomas and solid tumors, rheumatoid arthritis, multiple sclerosis, amyotrophic lateral sclerosis, Crohn's disease, ulcerative colitis, systemic lupus erythematosis, autoimmune hemolytic anemia, type I diabetes, asthma, chronic obstructive pulmonary disease, cystic fibrosis, idiopathic pulmonary fibrosis, sarcoidosis, allergic rhinitis, atopic dermatitis, contact dermatitis, eczema, psoriasis, basal cell carcinoma, squamous cell carcinoma and actinic keratosis.

The invention also encompasses the use of a combination of the compounds of the invention together with one or more other therapeutic agents for the manufacture of a formulation or medicament for treating these diseases.

The invention also provides a method of treatment of a pathological condition or disease susceptible to amelioration by inhibiton of Phosphoinositide 3-Kinases (PI3Ks), in particular wherein the pathological condition or disease is selected from respiratory diseases; allergic diseases; inflammatory or autoimmune-mediated diseases; function disorders and neurological disorders; cardiovascular diseases; viral infection; metabolism/endocrine function disorders; neurological disorders and pain; bone marrow and organ transplant rejection; myelo-dysplastic syndrome; myeloproliferative disorders (MPDs such as polycythemia vera, essential thrombocythemia or mielofibrosis); cancer and hematologic malignancies, leukemia, lymphomas and solid tumors; more in particular wherein the pathological condition or disease is selected from leukemia, lymphomas and solid tumors, rheumatoid arthritis, multiple sclerosis, amyotrophic lateral sclerosis, Crohn's disease, ulcerative colitis, systemic lupus erythematosis, autoimmune hemolytic anemia, type I diabetes, asthma, chronic obstructive pulmonary disease, cystic fibrosis, idiopathic pulmonary fibrosis, sarcoidosis, allergic rhinitis, atopic dermatitis, contact dermatitis, eczema, psoriasis, basal cell carcinoma, squamous cell carcinoma and actinic keratosis; comprising administering a therapeutically effective amount of a combination of the compounds of the invention together with one or more other therapeutic agents.

The active compounds in the combinations of the invention may be administered by any suitable route, depending on the nature of the disorder to be treated, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The active compounds in the combination, i.e. the imidazopyridine derivatives of the invention, and the other optional active compounds may be administered together in the same pharmaceutical composition or in different compositions intended for separate, simultaneous, concomitant or sequential administration by the same or a different route.

One execution of the present invention consists of a kit of parts comprising a imidazopyridine derivative of the invention together with instructions for simultaneous, concurrent, separate or sequential use in combination with another active compound useful in the treatment of respiratory diseases; allergic diseases; inflammatory or autoimmune-mediated diseases; function disorders and neurological disorders; cardiovascular diseases; viral infection; metabolism/endocrine function disorders; neurological disorders and pain; bone marrow and organ transplant rejection; myelo-dysplastic syndrome; myeloproliferative disorders (MPDs such as polycythemia vera, essential thrombocythemia or mielofibrosis); cancer and hematologic malignancies, leukemia, lymphomas and solid tumors; more in particular wherein the pathological condition or disease is selected from leukemia, lymphomas and solid tumors, rheumatoid arthritis, multiple sclerosis, amyotrophic lateral sclerosis, Crohn's disease, ulcerative colitis, systemic lupus erythematosis, autoimmune hemolytic anemia, type I diabetes, asthma, chronic obstructive pulmonary disease, cystic fibrosis, idiopathic pulmonary fibrosis, sarcoidosis, allergic rhinitis, atopic dermatitis, contact dermatitis, eczema, psoriasis, basal cell carcinoma, squamous cell carcinoma and actinic keratosis.

Another execution of the present invention consists of a package comprising a imidazopyridine derivative of the invention and another active compound useful in the treatment of respiratory diseases; allergic diseases; inflammatory or autoimmune-mediated diseases; function disorders and neurological disorders; cardiovascular diseases; viral infection; metabolism/endocrine function disorders; neurological disorders and pain; bone marrow and organ transplant rejection; myelo-dysplastic syndrome; myeloproliferative disorders (MPDs such as polycythemia vera, essential thrombocythemia or mielofibrosis); cancer and hematologic malignancies, leukemia, lymphomas and solid tumors; more in particular wherein the pathological condition or disease is selected from leukemia, lymphomas and solid tumors, rheumatoid arthritis, multiple sclerosis, amyotrophic lateral sclerosis, Crohn's disease, ulcerative colitis, systemic lupus erythematosis, autoimmune hemolytic anemia, type I diabetes, asthma, chronic obstructive pulmonary disease, cystic fibrosis, idiopathic pulmonary fibrosis, sarcoidosis, allergic rhinitis, atopic dermatitis, contact dermatitis, eczema, psoriasis, basal cell carcinoma, squamous cell carcinoma and actinic keratosis.

### Pharmaceutical Compositions

Pharmaceutical compositions according to the present invention comprise the compounds of the invention in association with a pharmaceutically acceptable diluent or carrier.

As used herein, the term pharmaceutical composition refers to a mixture of one or more of the compounds described herein, or physiologically/pharmaceutically acceptable salts, solvates, N-oxides, stereoisomers, deuterated derivatives thereof or prodrugs thereof, with other chemical components, such as physiologically/pharmaceutically acceptable carriers and excipients. The purpose of a pharmaceutical composition is to facilitate administration of a compound to an organism.

As used herein, a physiologically/pharmaceutically acceptable diluent or carrier refers to a carrier or diluent that does not cause significant irritation to an organism and does not abrogate the biological activity and properties of the administered compound.

The invention further provides pharmaceutical compositions comprising the compounds of the invention in association with a pharmaceutically acceptable diluent or carrier together with one or more other therapeutic agents for use in the treatment of a pathological condition or disease susceptible to amelioration by inhibiton of Phosphoinositide 3-Kinases (PI3Ks), such as the ones previously described.

The invention is also directed to pharmaceutical compositions of the invention for use in the treatment of a pathological condition or disease susceptible to amelioration by inhibiton of Phosphoinositide 3-Kinases (PI3Ks), in particular wherein the pathological condition or disease is selected from respiratory diseases; allergic diseases; inflammatory or autoimmune-mediated diseases; function disorders and neurological disorders; cardiovascular diseases; viral infection; metabolism/endocrine function disorders; neurological disorders and pain; bone marrow and organ transplant rejection; myelo-dysplastic syndrome; myeloproliferative disorders (MPDs such as polycythemia vera, essential thrombocythemia or mielofibrosis); cancer and hematologic malignancies, leukemia, lymphomas and solid tumors; more in particular wherein the pathological condition or disease is selected from leukemia, lymphomas and solid tumors, rheumatoid arthritis, multiple sclerosis, amyotrophic lateral sclerosis, Crohn's disease, ulcerative colitis, systemic lupus erythematosis, autoimmune hemolytic anemia, type I diabetes, asthma, chronic obstructive pulmonary disease, cystic fibrosis, idiopathic pulmonary fibrosis, sarcoidosis, allergic rhinitis, atopic dermatitis, contact dermatitis, eczema, psoriasis, basal cell carcinoma, squamous cell carcinoma and actinic keratosis. The invention also encompasses the use of a pharmaceutical composition of the invention for the manufacture of a medicament for treating these diseases.

The invention also provides a method of treatment of a pathological condition or disease susceptible to amelioration by inhibiton of Phosphoinositide 3-Kinases (PI3Ks), in particular wherein the pathological condition or disease is selected from respiratory diseases; allergic diseases; inflammatory or autoimmune-mediated diseases; function disorders and neurological disorders; cardiovascular diseases; viral infection; metabolism/endocrine function disorders; neurological disorders and pain; bone marrow and organ transplant rejection; myelo-dysplastic syndrome; myeloproliferative disorders (MPDs such as polycythemia vera, essential thrombocythemia or mielofibrosis); cancer and hematologic malignancies, leukemia, lymphomas and solid tumors; more in particular wherein the pathological condition or disease is selected from leukemia, lymphomas and solid tumors, rheumatoid arthritis, multiple sclerosis, amyotrophic lateral sclerosis, Crohn's disease, ulcerative colitis, systemic lupus erythematosis, autoimmune hemolytic anemia, type I diabetes, asthma, chronic obstructive pulmonary disease, cystic fibrosis, idiopathic pulmonary fibrosis, sarcoidosis, allergic rhinitis, atopic dermatitis, contact dermatitis, eczema, psoriasis, basal cell carcinoma, squamous cell carcinoma and actinic keratosis; comprising administering a therapeutically effective amount of a pharmaceutical composition of the invention.

The present invention also provides pharmaceutical compositions which comprise, as an active ingredient, at least a compound of formula (I) or a pharmaceutically acceptable salt thereof in association with a pharmaceutically acceptable excipient such as a carrier or diluent. The active ingredient may comprise 0.001% to 99% by weight, preferably 0.01% to 90% by weight, of the composition depending upon the nature of the formulation and whether further dilution is to be made prior to application. Preferably the compositions are made up in a form suitable for oral, inhalation, topical, nasal, rectal, percutaneous or injectable administration.

Pharmaceutical compositions suitable for the delivery of compounds of the invention and methods for their preparation will be readily apparent to those skilled in the art. Such compositions and methods for their preparation can be found, for example, in Remington: The Science and Practice of Pharmacy, 21 st Edition, Lippincott Williams & Wilkins, Philadelphia, Pa., 2001.

The pharmaceutically acceptable excipients which are admixed with the active compound or salts of such compound, to form the compositions of this invention are well-known per se and the actual excipients used depend inter alia on the intended method of administering the compositions. Examples, without limitation, of excipients include calcium carbonate, calcium phosphate, various sugars and types of starch, cellulose derivatives, gelatin, vegetable oils and polyethylene glycols.

Additional suitable carriers for formulations of the compounds of the present invention can be found in Remington: The Science and Practice of Pharmacy, 21 st Edition, Lippincott Williams & Wilkins, Philadelphia, Pa., 2001.

### i) Oral Administration

The compounds of the invention may be administered orally (peroral administration; *per os* (latin)). Oral administration involve swallowing, so that the compound is absorbed from the gut and delivered to the liver via the portal circulation (hepatic first pass metabolism) and finally enters the gastrointestinal (GI) tract.

Compositions for oral administration may take the form of tablets, retard tablets, sublingual tablets, capsules, inhalation aerosols, inhalation solutions, dry powder inhalation, or liquid preparations, such as mixtures, solutions, elixirs, syrups or suspensions, all containing the compound of the invention; such preparations may be made by methods well-known in the art. The active ingredient may also be presented as a bolus, electuary or paste.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For tablet dosage forms, depending on dose, the drug may make up from 1 wt% to 80 wt% of the dosage form, more typically from 5 wt% to 60 wt% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl- substituted hydroxypropyl cellulose, starch, pregelatinized starch and sodium alginate. Generally, the disintegrant will comprise from 1 wt% to 25 wt%, preferably from 5 wt% to 20 wt% of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinized starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate. Tablets may also optionally include surface active agents, such as sodium lauryl sulfate and polysorbate 80, and glidants such as silicon dioxide and talc. When present, surface active agents are typically in amounts of from 0.2 wt% to 5 wt% of the tablet, and glidants typically from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally are present in amounts from 0.25 wt% to 10 wt%, preferably from 0.5 wt% to 3 wt% of the tablet. Other conventional ingredients include anti-oxidants, colorants, flavoring agents, preservatives and taste-masking agents.

Exemplary tablets contain up to about 80 wt% drug, from about 10 wt% to about 90 wt% binder, from about 0 wt% to about 85 wt% diluent, from about 2 wt% to about 10 wt% disintegrant, and from about 0.25 wt% to about 10 wt% lubricant. Tablet blends may be compressed directly or by roller to form tablets. Tablet blends or portions of blends may alternatively be wet-, dry-, or melt-granulated, melt congealed, or extruded before tabletting. The final formulation may include one or more layers and may be coated or uncoated; or encapsulated.

The formulation of tablets is discussed in detail in "Pharmaceutical Dosage Forms: Tablets, Vol. 1 ", by H. Lieberman and L. Lachman, Marcel Dekker, N.Y., 1980.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Solid formulations for oral administration may be formulated to be immediate and/or modified release. Modified release formulations include delayed-, sustained-, pulsed-, controlled-, targeted and programmed release.

Suitable modified release formulations are described in U.S. Patent No. 6,106,864. Details of other suitable release technologies such as high energy dispersions and osmotic and coated particles can be found in Verma et al, Pharmaceutical Technology On-line, 25(2), 1-14 (2001). The use of chewing gum to achieve controlled release is described in WO 00/35298. The disclosures of these references are incorporated herein by reference in their entireties.

Liquid formulations include suspensions, solutions, syrups and elixirs. Such formulations may be used as fillers in soft or hard capsules and typically include a carrier, for example, water, ethanol, polyethylene glycol, propylene glycol, methylcellulose, or a suitable oil, and one or more emulsifying agents and/or suspending agents. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent. Liquid formulations may also be prepared by the reconstitution of a solid, for example, from a sachet.

### ii) Oral mucosal administration

The compounds of the invention can also be administered via the oral mucosal. Within the oral mucosal cavity, delivery of drugs is classified into three categories: (a) sublingual delivery, which is systemic delivery of drugs through the mucosal membranes lining the floor of the mouth, (b) buccal delivery, which is drug administration through the mucosal membranes lining the cheeks (buccal mucosa), and (c) local delivery, which is drug delivery into the oral cavity.

Pharmaceutical products to be administered via the oral mucosal can be designed using mucoadhesive, quick dissolve tablets and solid lozenge formulations, which are formulated with one or more mucoadhesive (bioadhesive) polymers (such as hydroxy propyl cellulose, polyvinyl pyrrolidone, sodium carboxymethyl cellulose, hydroxy propyl methyl cellulose, hydroxy ethyl cellulose, polyvinyl alcohol, polyisobutylene or polyisoprene); and oral mucosal permeation enhancers (such as butanol, butyric acid, propranolol, sodium lauryl sulphate and others)

### iii) Inhaled administration

The compounds of the invention can also be administered by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurized container, pump, spray, atomizer (preferably an atomizer using electrohydrodynamics to produce a fine mist), or nebulizer, with or without the use of a suitable propellant, such as 1 ,1 ,1 ,2-tetrafluoroethane or 1 ,1 ,1 ,2,3,3,3-heptafluoropropane. For intranasal use, the powder may include a bioadhesive agent, for example, chitosan or cyclodextrin.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 0.001-50 mg, more preferably 0.01-5 mg of active ingredient or the equivalent amount of a pharmaceutically acceptable salt thereof. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (Ex. EP0069715) or disks (Ex. GB 2041763; EP 0424790; DE 4239402 and EP 0674533), rotatable cylinders (Ex. EP 0166294; GB 2165159 and WO 92/09322) and rotatable frustums (Ex. WO 92/00771), all having cavities which have to be filled with powder from the container. Other multi dose devices have measuring slides (Ex. US 5201308 and WO 97/00703) or measuring plungers with a local or circumferential recess to displace a certain volume of powder from the container to a delivery chamber or an air conduit (Ex. EP 0505321, WO 92/04068 and WO 92/04928), or measuring slides such as the Genuair® (formerly known as Novolizer SD2FL), which is described the following patent applications Nos: WO97/000703, WO03/000325 and WO2006/008027.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (Ex. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with. Such atomiser is the Respimat® which is described, for example, in PCT Patent Applications Nos. WO 91/14468 and WO 97/12687, reference here is being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetra-fluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant.

The aerosol composition may be excipient free or may optionally contain additional formulation excipients well known in the art such as surfactants (eg oleic acid or lecithin) and cosolvens (eg ethanol). Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10 µm preferably 2-5 µm. Particles having a size above 20 µm are generally too large when inhaled to reach the small airways. To achieve these particle sizes the particles of the active ingredient as produced may be size reduced by conventional means eg by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate.

Pressurized aerosol compositions will generally be filled into canisters fitted with a valve, especially a metering valve. Canisters may optionally be coated with a plastics material e. g. a fluorocarbon polymer as described in W096/32150. Canisters will be fitted into an actuator adapted for buccal delivery.

### iv) Nasal mucosal administration

The compounds of the invention may also be administered via the nasal mucosal. Typical compositions for nasal mucosa administration are typically applied by a metering, atomizing spray pump and are in the form of a solution or suspension in an inert vehicle such as water optionally in combination with conventional excipients such as buffers, anti-microbials, tonicity modifying agents and viscosity modifying agents.

### v) Parenteral Administration

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water.

The preparation of parenteral formulations under sterile conditions, for example, by lyophilization, may readily be accomplished using standard pharmaceutical techniques well known to those skilled in the art. The solubility of compounds of the invention used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

### vi) Topical Administration

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated; see, for example, J Pharm Sci, 88 (10), 955-958 by Finnin and Morgan (October 1999). Other means of topical administration include delivery by electroporation, iontophoresis, phonophoresis, sonophoresis and microneedle or needle-free injection.

Formulations for topical administration may be formulated to be immediate and/or modified release. Modified release formulations include delayed-, sustained-, pulsed-, controlled-, targeted and programmed release.

### vii) Rectal/Intravaginal Administration

Compounds of the invention may be administered rectally or vaginally, for example, in the form of a suppository, pessary, or enema. Cocoa butter is a traditional suppository base, but various alternatives may be used as appropriate. Formulations for rectal/vaginal administration may be formulated to be immediate and/or modified release. Modified release formulations include delayed-, sustained-, pulsed-, controlled-, targeted and programmed release.

### viii) Ocular Administration

Compounds of the invention may also be administered directly to the eye or ear, typically in the form of drops of a micronized suspension or solution in isotonic, pH-adjusted, sterile saline. Other formulations suitable for ocular and aural administration include ointments, biodegradable {e.g. absorbable gel sponges, collagen) and nonbiodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

### ix) Other Technologies

Compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

The amount of the active compound administered will be dependent on the subject being treated, the severity of the disorder or condition, the rate of administration, the disposition of the compound and the discretion of the prescribing physician. However, an effective dosage is typically in the range of 0.01-3000 mg, more preferably 0.5-1000 mg of active ingredient or the equivalent amount of a pharmaceutically acceptable salt thereof per day. Daily dosage may be administered in one or more treatments, preferably from 1 to 4 treatments, per day.

Preferably, the the pharmaceutical compositions of the invention are made up in a form suitable for oral, inhalation or topical administration, being particularly preferred oral or inhalation administration.

The pharmaceutical formulations may conveniently be presented in unit dosage form and may be prepared by any of the methods well known in the art of pharmacy. Preferably the composition is in unit dosage form, for example a tablet, capsule or metered aerosol dose, so that the patient may administer a single dose.

The amount of each active which is required to achieve a therapeutic effect will, of course, vary with the particular active, the route of administration, the subject under treatment, and the particular disorder or disease being treated.

The following preparations forms are cited as formulation examples:

### Formulation Examples

### Formulation Example 1 (Oral suspension)

| **Ingredient** | **Amount** |
|---|---|
| Active Compound | 3 mg |
| Citric acid | 0,5 g |
| Sodium chloride | 2,0 g |
| Methyl paraben | 0,1 g |
| Granulated sugar | 25 g |
| Sorbitol (70% solution) | 11 g |
| Veegum K | 1,0 g |
| Flavoring | 0,02 g |
| Dye | 0,5 mg |
| Distilled water | q.s. to 100 mL |

### Formulation Example 2 (Hard gelatine capsule for oral administration)

| **Ingredient** | **Amount** |
|---|---|
| Active Compound | 1 mg |
| Lactose | 150 mg |
| Magnesium stearate | 3 mg |

### Formulation Example 3 (Gelatin cartridge for inhalation)

| **Ingredient** | **Amount** |
|---|---|
| Active Compound (micronized) | 0,2 mg |
| Lactose | 25 mg |

### Formulation Example 4 (Formulation for inhalation with a DPI)

| **Ingredient** | **Amount** |
|---|---|
| Active Compound (micronized) | 15 mg |
| Lactose | 3000 mg |

### Formulation Example 5 (Formulation for a MDI)

| **Ingredient** | **Amount** |
|---|---|
| Active Compound (micronized) | 10g |
| 1,1,1,2,3,3,3-heptafluoro-n-propane | q.s. to 200 mL |

In all the formulation examples, active compound is (S)-N-(1-(5-Methoxy-3-phenyl-3H-imidazo[4,5-b]pyridin-2-yl)propyl)-9H-purin-6-amine.

Modifications, which do not affect, alter, change or modify the essential aspects of the compounds, combinations or pharmaceutical compositions described, are included within the scope of the present invention.

## Claims

1. A compound of formula (I), or a pharmaceutically acceptable salt, or solvate, or N-oxide, or stereoisomer or deuterated derivative thereof: 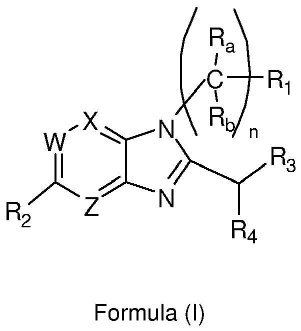 wherein,
X represents a nitrogen atom, W represents a -CR₆ group and Z represents a -CR₇ group; or X represents a -CR₅ group, W represents a nitrogen atom and Z represents a -CR₇ group; or X represents a -CR₅ group, W represents a -CR₆ group and Z represents nitrogen atom;
Rₐ and R_{b} each independently represent a hydrogen atom, a C₁-C₄ haloalkyl group, a C₁-C₄ hydroxyalkyl group or a linear or branched C₁-C₄ alkyl group;
n represents 0, 1, 2 or 3;
R₁ represents a linear or branched C₁-C₄ alkyl group, a C₃-C₁₀ cycloalkyl group, a C₃-C₁₀ cycloalkenyl group, a monocyclic or bicyclic C₆-C₁₄ aryl group, a 5- to 14-membered monocyclic or bicyclic heteroaryl group containing at least one heteroatom selected from O, S and N, a 5- to 14- membered monocyclic or bicyclic heterocyclyl group containing at least one heteroatom selected from O, S and N, or a 2-pyridone group,
wherein the cycloalkyl, cycloalkenyl, aryl, heteroaryl, and heterocyclyl groups are unsubstituted or substituted by one or more substituents selected from a halogen atom, a hydroxy group, a cyano group, a linear or branched C₁-C₄ alkyl group, a C₁-C₄ haloalkyl group, a C₁-C₄ hydroxyalkyl group, a C₃-C₄ cycloalkyl group, a -(CH₂)₁-₃CN group, a -(CH₂)₀₋₃OR₈ group, a -(CH₂)₀₋₃NR₈R₉ group, a -C(O)-(CH₂)₁₋₃-CN group, a -C(O)-(CH₂)₀₋₃-R₈ group, a -C(O)-(CH₂)₀₋₃-NR₈R₉ group, a -S(O)₂(CH₂)₀₋₃R₈ groupa -S(O)₂(CH₂)₀₋₃NR₈R₉ group;
R₂ represents a hydrogen atom, a halogen atom, a hydroxy group, a cyano group, a C₁-C₄ alkoxy group, a C₁-C₄ haloalkyl group, a C₁-C₄ hydroxyalkyl group, a C₃-C₇ cycloalkyl group, a -NR'R" group, or a linear or branched C₁-C₆ alkyl group, which alkyl group is unsubstituted or substituted by one or more substituents selected from a C₁-C₄ alkoxy group, a cyano group or a C₃-C₇ cycloalkyl group;
R₃ represents a hydrogen atom, a C₁-C₄ alkoxy group, a C₁-C₄ haloalkyl group, a C₁-C₄ hydroxyalkyl group, a C₃-C₇ cycloalkyl group, a -(CH₂)₁₋₄NR'R" group, or a linear or branched C₁-C₄ alkyl group, which alkyl group is unsubstituted or substituted by one or more substituents selected from a C₁-C₄ alkoxy group, a cyano group, a C₃-C₄ cycloalkyl group, a -C(O)-(CH₂)₀₋₃-R' group or a -C(O)-(CH₂)₀₋₃-NR'R" group;
R₅, R₆ and R₇ each independently represent a hydrogen atom, a halogen atom, a hydroxy group, a cyano group, a C₁-C₄ alkoxy group, a C₁-C₄ haloalkyl group, a C₁-C₄ hydroxyalkyl group, a C₃-C₇ cycloalkyl group, a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group;
R₈ and R₉ each independently represent a hydrogen atom, a C₁-C₄ haloalkyl group, a C₁-C₄ hydroxyalkyl group or a linear or branched C₁-C₄ alkyl group, which alkyl group is unsubstituted or substituted by one or more substituents selected from a C₁-C₄ alkoxy group, a cyano group or a C₃-C₄ cycloalkyl group;
R' and R" each independently represent a hydrogen atom, a hydroxy group, a C₁-C₄ alkoxy group or a linear or branched C₁-C₄ alkyl group.
R₄ represents a group selected from:
i) a group of formula (IIa) or (IIa') 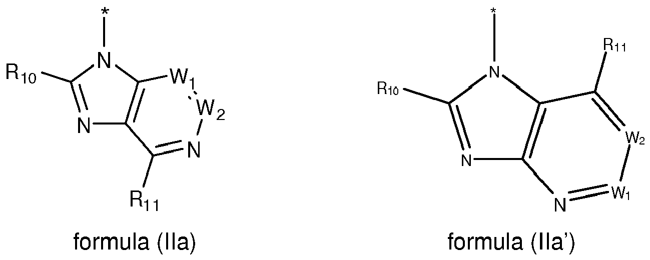
ii) a group of formula (IIb) 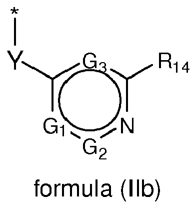 and
iii) a group of formula (IIc) 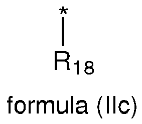 wherein
Y represents a linker selected from a -NR'- group, -O- or -S-; wherein R' is as defined above;
(*) represents where R₄ is bonded to the carbon atom attached to R₃ and to the imidazole group;
W₁ represents a -CR₁₂ group and W₂ represents a nitrogen atom, or W₁ represents a nitrogen atom and W₂ represents a -CR₁₃ group;
G₁ represents a -CR₁₅ group and G₂ represents a nitrogen atom, or G₁ represents a nitrogen atom and G₂ represents a -CR₁₆ group, or G₁ represents a -CR₁₅ group and G₂ represents a -CR₁₆ group;
G₃ represents a nitrogen atom or a -CR₁₇ group;
R₁₀, R₁₁, R₁₂ R₁₃, R₁₄, R₁₅, R₁₆ and R₁₇ each independently represent a hydrogen atom, a C₁-C₄ alkoxy group, a C₁-C₄ haloalkyl group, a C₁-C₄ hydroxyalkyl group, a C₃-C₄ cycloalkyl group, a -(CH₂)₀₋₃CN group,
a -C(O)-(CH₂)₁-₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group; wherein R' and R" are as defined above;
R₁₈ represents a group selected from 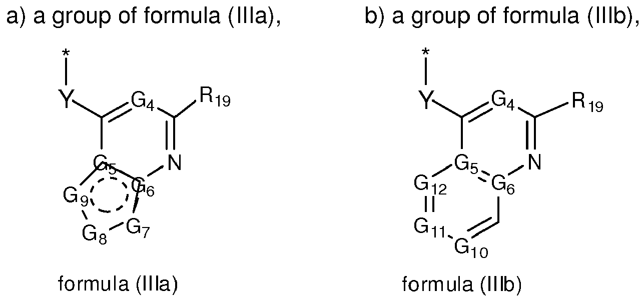 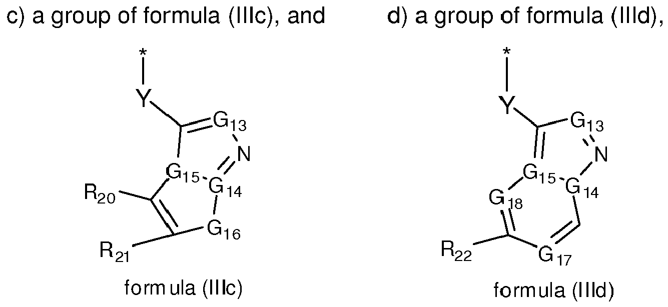 wherein
G₄ represents a nitrogen atom or a -CR₂₃ group;
G₅ and G₆ each independently represents a nitrogen atom or a carbon atom, wherein when one of G₅ and G₆ represents a nitrogen atom the remaining represents a carbon atom;
G₇ represents a -NH group or a -CH group;
G₈ represents a nitrogen atom or a -CR₂₄ group;
G₉ represents a nitrogen atom or a -CR₂₅ group;
G₁₀ represents a nitrogen atom or a -CR₂₆ group;
G₁₁ represents a nitrogen atom or a -CR₂₇ group;
G₁₂ represents a nitrogen atom or a -CR₂₈ group;
G₁₃ represents a nitrogen atom or a -CR₂₉ group;
G₁₄ and G₁₅ each independently represents a nitrogen atom or a carbon atom, wherein when one of G₁₄ and G₁₅ represents a nitrogen atom the remaining represents a carbon atom;
G₁₆ represents a -NH group or a -CH group;
G₁₇ represents a nitrogen atom or a -CR₃₀ group;
G₁₈ represents a nitrogen atom or a -CR₃₁ group;
R₁₉, R₂₀, R₂₁, R₂₂, R₂₃, R₂₄, R₂₅, R₂₆, R₂₇, R₂₈, R₂₉, R₃₀ and R₃₁ each independently represent a hydrogen atom, a C₁-C₄ alkoxy group, a C₁-C₄ haloalkyl group, a C₁-C₄ hydroxyalkyl group, a C₃-C₄ cycloalkyl group,
a -(CH₂)₀₋₃CN group, a -C(O)-(CH₂)₁-₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group; wherein R' and R" are as defined above; and wherein Y is as defined above;
or in the case that Y represents a -NR'- group, R₃ together with the -NR'- group and the carbon atom to which both R₃ and the -NR'- group are bonded form a 4- to 7-membered, saturated N-containing heterocyclyl group, which heterocyclyl group is unsubstituted or substituted by one or more substituents selected from a halogen atom, a hydroxyl group, a cyano group, a -CHF₂ group or a -CF₃ group.

2. A compound according to claim 1, wherein
Rₐ and R_{b} each independently represent a hydrogen atom or a linear or branched C₁-C₃ alkyl group;
n represents 0, 1 or 2;
R₁ represents a C₃-C₇ cycloalkyl group, a phenyl group, a 5- to 10- membered monocyclic or bicyclic heteroaryl group containing containing one, two or three heteroatoms selected from O, S and N, a pyrrolidinyl group, a piperidinyl group, a piperazinyl group, a tetrahydropyranyl group, a 2-pyridone group or a morpholinyl group,
wherein the cycloalkyl, phenyl, heteroaryl, pyrrolidinyl, piperidinyl, piperazinyl, tetrahydropyranyl, 2-pyridone or morpholinyl groups are unsubstituted or substituted by one or more substituents selected from a halogen atom, a hydroxy group, a linear or branched C₁-C₄ alkyl group, a C₁-C₄ haloalkyl group, a C₁-C₄ hydroxyalkyl group, a C₃-C₄ cycloalkyl group, a -(CH₂)₀₋₃OR₈ group, a - (CH₂)₀₋₃NR₈R₉ group, a -C(O)-(CH₂)₀₋₃-R₈ group or a -C(O)-(CH₂)₀₋₃-NR₈R₉ group; wherein R₈ and R₉ each independently represent a hydrogen atom or a C₁-C₄ alkyl group;
R₂ represents a hydrogen atom, a halogen atom, a hydroxy group, a C₁-C₃ alkoxy group, a C₁-C₃ haloalkyl group, a C₃-C₄ cycloalkyl group, a -NH₂ group,
a -N(CH₃)H group, a -N(CH₃)₂ group, or a linear or branched C₁-C₄ alkyl group, which alkyl group is unsubstituted or substituted by a C₁-C₂ alkoxy group;
R₃ represents a hydrogen atom, a C₁-C₃ alkoxy group, a C₁-C₃ haloalkyl group, a C₃-C₄ cycloalkyl group, or a linear or branched C₁-C₃ alkyl group;
R₅, R₆ and R₇ each independently represent a hydrogen atom, a halogen atom, a hydroxy group, a C₁-C₃ alkoxy group, a C₁-C₃ haloalkyl group, a C₃-C₄ cycloalkyl group, a -NH₂ group, a -N(CH₃)H group, a -N(CH₃)₂ group, or a linear or branched C₁-C₄ alkyl group, which alkyl group is unsubstituted or substituted by a C₁-C₂ alkoxy group;
R₄ represents a group of formula (IIa-2), (IIa'-1), (IIb-2), (IIIa-1) or (IIIa-14) 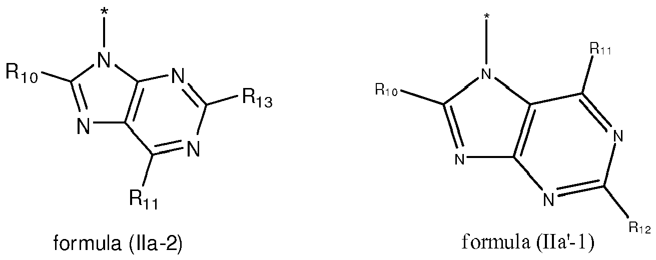 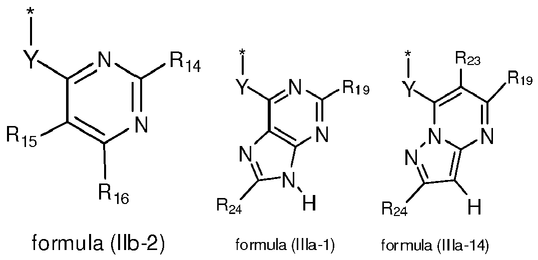 wherein:
$ R₁₀, R₁₁, and R₁₃ each independently represent a hydrogen atom, a -(CH₂)₀₋₃CN group, a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group; wherein R' and R" each independently represent a hydrogen atom, a hydroxy group, a C₁-C₄ alkoxy group or a linear or branched C₁-C₄ alkyl group;
$ R₁₄, R₁₅ and R₁₆ each independently represent a hydrogen atom, a -(CH₂)₀₋₃CN group, a -C(O)-(CH₂)₀-₃-NR'R" or a -(CH₂)₀₋₃NR'R" group; wherein R' and R" each independently represent a hydrogen atom, a hydroxy group, or a linear or branched C₁-C₄ alkyl group;
$ R₁₉, R₂₃ and R₂₄ each independently represent a hydrogen atom, a -(CH₂)₀₋₃CN group, a -C(O)-(CH₂)₁₋₃-CN group, a -C(O)-(CH₂)₀₋₃-R' group, a -C(O)-(CH₂)₀-₃-NR'R", a -(CH₂)₀-₃NR'R" group, or a linear or branched C₁-C₄ alkyl group; wherein R' and R" each independently represent a hydrogen atom, a hydroxy group, a C₁-C₄ alkoxy group or a linear or branched C₁-C₄ alkyl group; and
$ Y represents a -NR'- group, -O- or -S- wherein R' represents hydrogen or a linear or branched C₁-C₄ alkyl group; or in the case that Y represents a -NR'-group, R₃ together with the -NR'- group and the carbon atom to which both R₃ and the -NR'- group are bonded form a 4- to 7- membered, saturated N-containing heterocyclyl group.

3. A compound according to claim 1, wherein R₁ represents a C₁-C₃ alkyl group, a C₃-C₇ cycloalkyl group, a phenyl group, a naphthyl group, a 5- to 10- membered monocyclic or bicyclic heteroaryl group containing containing one, two or three heteroatoms selected from O, S and N, or a 5- to 10- membered monocyclic or bicyclic heterocyclyl group containing containing one, two or three heteroatoms selected from O, S and N,
wherein the cycloalkyl, phenyl, naphthyl, heteroaryl and heterocyclyl groups are unsubstituted or substituted by one or more substituents selected from a halogen atom, a hydroxy group, a cyano group, a linear or branched C₁-C₄ alkyl group, a C₁-C₄ haloalkyl group, a C₁-C₄ hydroxyalkyl group, a C₃-C₄ cycloalkyl group, a -(CH₂)₀₋₃OR₈ group, a -(CH₂)₀₋₃NR₈R₉ group, a -C(O)-(CH₂)₀₋₃R₈ group or a -C(O)-(CH₂)₀₋₃-NR₈R₉ group; wherein R₇ and R₈ are as defined in claim 1.

4. A compound according to claim 3, wherein R₁ represents a C₃-C₇ cycloalkyl group, a phenyl group, a 5- to 10- membered monocyclic or bicyclic heteroaryl group containing containing one, two or three heteroatoms selected from O, S and N, a pyrrolidinyl group, a piperidinyl group, a piperazinyl group, a tetrahydropyranyl group or a morpholinyl group,
wherein the cycloalkyl, phenyl, heteroaryl, pyrrolidinyl, piperidinyl, piperazinyl, tetrahydropyranyl or morpholinyl groups are unsubstituted or substituted by one or more substituents selected from a halogen atom, a hydroxy group, a linear or branched C₁-C₄ alkyl group, a C₁-C₄ haloalkyl group, a C₁-C₄ hydroxyalkyl group, a C₃-C₄ cycloalkyl group, a -(CH₂)₀₋₃OR₈ group, a -(CH₂)₀-₃NR₇R₈ group, a -C(O)-(CH₂)₀₋₃-R₈ group or a -C(O)-(CH₂)₀₋₃-NR₇R₈ group; wherein R₇ and R₈ each independently represent a hydrogen atom or a C₁-C₄ alkyl group;
and
wherein R₁ preferably represents a phenyl group or a pyridinyl group, which phenyl or pyridinyl is unsubstituted or substituted by one, two or three substituents selected from a halogen atom, a hydroxy group, a linear or branched C₁-C₃ alkyl group, a C₁-C₃ haloalkyl group or a -(CH₂)₀₋₃OCH₃ group; and wherein preferably said phenyl and pyridinyl groups are directly bonded to the imidazopyridine group.

5. A compound according to any one of claims 1, 3 or 4, wherein R₂ represents a hydrogen atom, a halogen atom, a hydroxy group, a cyano group, a C₁-C₄ alkoxy group, a C₁-C₄ haloalkyl group, a C₁-C₄ hydroxyalkyl group, a C₃-C₄ cycloalkyl group, a -(CH₂)₀₋₃NR'R" group, or a linear or branched C₁-C₄ alkyl group, which alkyl group is unsubstituted or substituted by a C₁-C₃ alkoxy group; wherein R' and R" each independently represent a hydrogen atom, a hydroxy group, or a linear or branched C₁-C₃ alkyl group;
and
wherein R₂ preferably represents a hydrogen atom, a halogen atom, a hydroxy group, a C₁-C₃ alkoxy group, a C₁-C₃ haloalkyl group, a C₃-C₄ cycloalkyl group, a -NH₂ group, a -N(CH₃)H group, a -N(CH₃)₂ group, or a linear or branched C₁-C₄ alkyl group, which alkyl group is unsubstituted or substituted by a C₁-C₂ alkoxy group.

6. A compound according to any one of claims 1 or 3 to 5, wherein R₃ represents a hydrogen atom, a C₁-C₄ alkoxy group, a C₁-C₄ haloalkyl group, a C₁-C₄ hydroxyalkyl group, a C₃-C₄ cycloalkyl group, a -(CH₂)₁₋₄NR'R" group, or a linear or branched C₁-C₄ alkyl group, which alkyl group is unsubstituted or substituted by a C₁-C₃ alkoxy group, a -C(O)-(CH₂)₀₋₃-R' group or a -C(O)-(CH₂)₀₋₃-NR'R" group; wherein R' and R" each independently represent a hydrogen atom, a hydroxy group, or a linear or branched C₁-C₃ alkyl group;
and
wherein R₃ preferably represents a hydrogen atom, a C₁-C₃ alkoxy group, a C₁-C₃ haloalkyl group, a C₃-C₄ cycloalkyl group, or a linear or branched C₁-C₃ alkyl group.

7. A compound according to any one of claims 1 or 3 to 6, wherein R₅, R₆ and R₇ each independently represent a hydrogen atom, a halogen atom, a hydroxy group, a cyano group, a C₁-C₄ alkoxy group, a C₁-C₄ haloalkyl group, a C₁-C₄ hydroxyalkyl group, a C₃-C₄ cycloalkyl group, a -(CH₂)₀₋₃NR'R" group, or a linear or branched C₁-C₄ alkyl group, which alkyl group is unsubstituted or substituted by a C₁-C₃ alkoxy group; wherein R' and R" each independently represent a hydrogen atom, a hydroxy group, or a linear or branched C₁-C₃ alkyl group;
and
wherein R₅, R₆ and R₇ each independently preferably represent a hydrogen atom, a halogen atom, a hydroxy group, a C₁-C₃ alkoxy group, a C₁-C₃ haloalkyl group, a C₃-C₄ cycloalkyl group, a -NH₂ group, a -N(CH₃)H group, a -N(CH₃)₂ group, or a linear or branched C₁-C₄ alkyl group, which alkyl group is unsubstituted or substituted by a C₁-C₂ alkoxy group.

8. A compound according to any one of claims 1 or 3 to 7, wherein R₄ represents a group selected from
i) a group of formula (IIa-1), 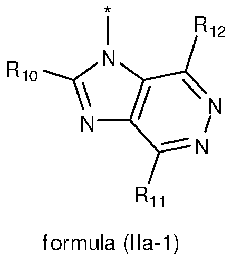
ii) a group of formula (IIa-2), 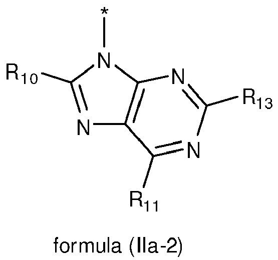
and iii) a group of formula (IIa'-1), 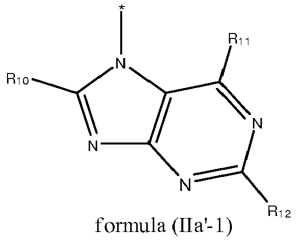 wherein
R₁₀, R₁₁, R₁₂ and R₁₃ each independently represent a hydrogen atom, a C₁-C₄ alkoxy group, a C₁-C₄ haloalkyl group, a C₁-C₄ hydroxyalkyl group, a C₃-C₄ cycloalkyl group, a -(CH₂)₀₋₃CN group, a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group; wherein R' and R" each independently represent a hydrogen atom, a hydroxy group, a C₁-C₄ alkoxy group or a linear or branched C₁-C₄ alkyl group; and wherein Y is as defined in claim 1.

9. A compound according to any one of claims 1 or 3 to 7, wherein R₄ represents a group selected from 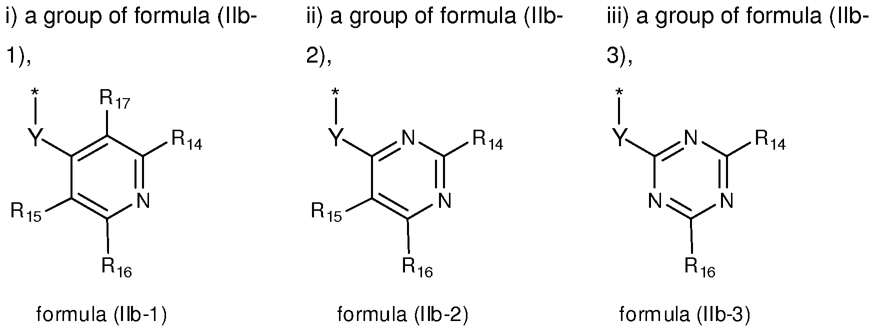 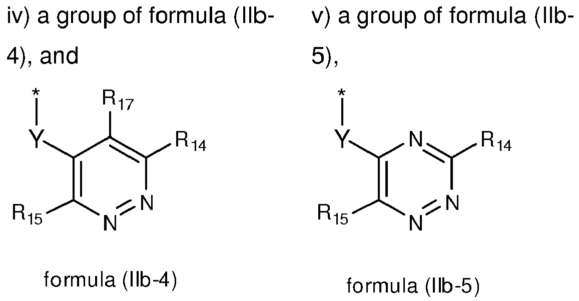 wherein
R₁₄, R₁₅, R₁₆ and R₁₇ each independently represent a hydrogen atom, a C₁-C₄ alkoxy group, a C₁-C₄ haloalkyl group, a C₁-C₄ hydroxyalkyl group, a C₃-C₄ cycloalkyl group, a -(CH₂)₀₋₃CN group, a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group; wherein R' and R" each independently represent a hydrogen atom, a hydroxy group, a C₁-C₄ alkoxy group or a linear or branched C₁-C₄ alkyl group; and wherein Y is as defined in claim 1;
or in the case that Y represents a -NR'- group, R₃ together with the -NR'- group and the carbon atom to which both R₃ and the -NR'- group are bonded form a 4- to 7-membered, saturated N-containing heterocyclyl group, which heterocyclyl group is unsubstituted or substituted by one or more substituents selected from a halogen atom, a hydroxyl group, a cyano group, a -CHF₂ group or a -CF₃ group.

10. A compound according to any one of claims 1 or 3 to 7, wherein R₅ represents a group selected from 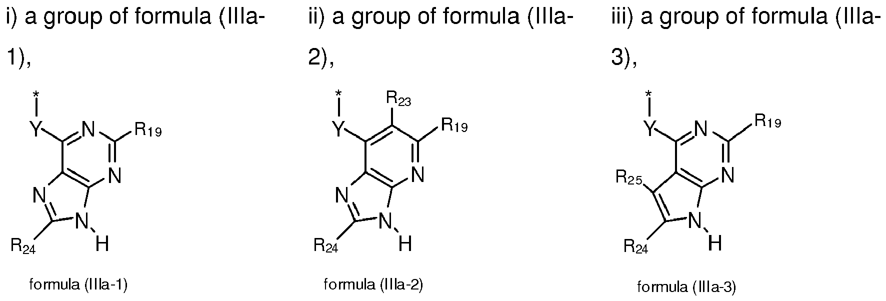 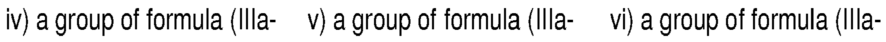 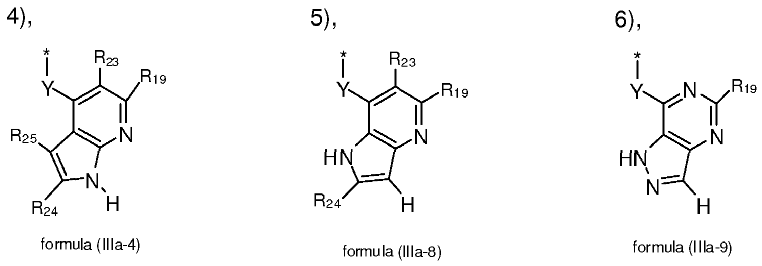 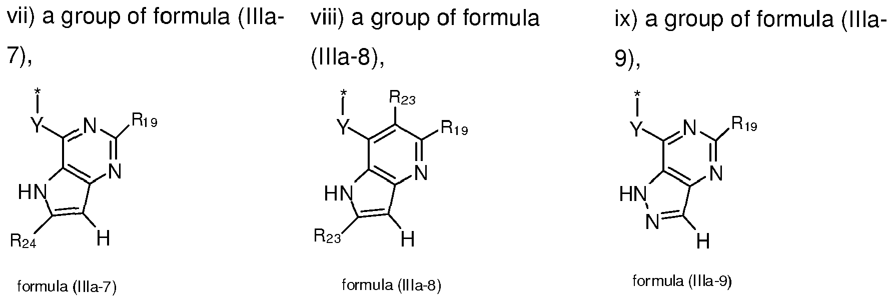 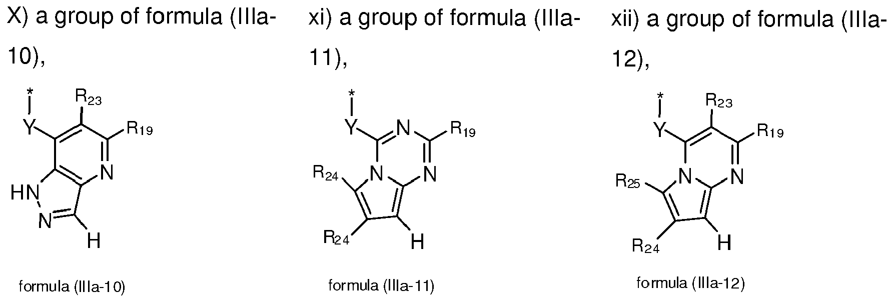 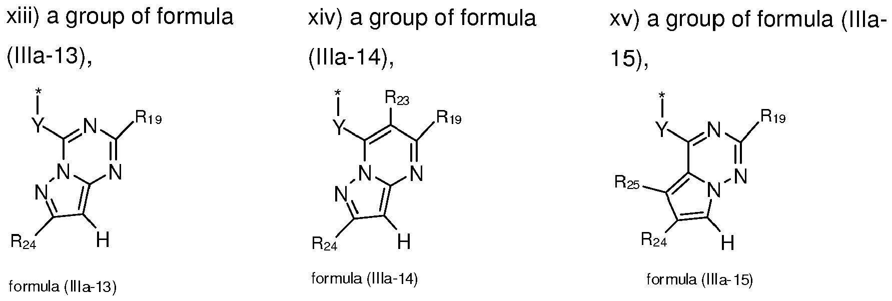 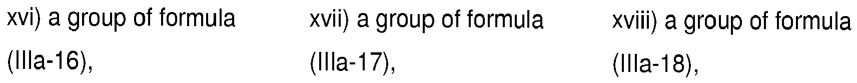 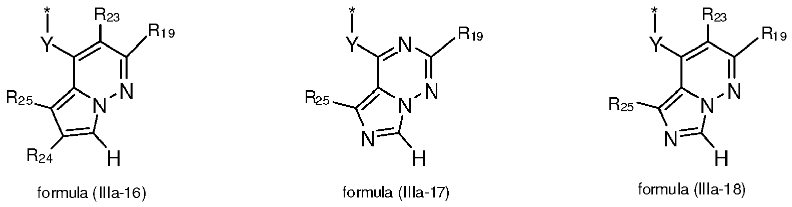 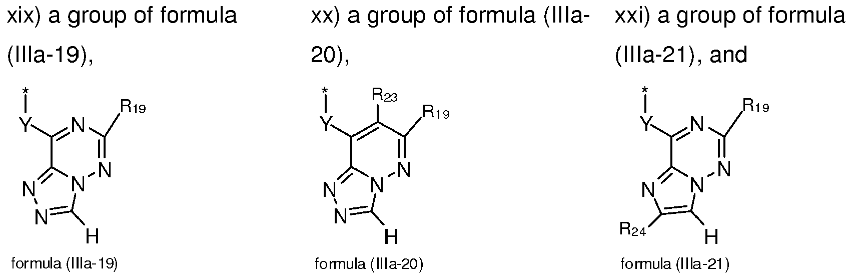 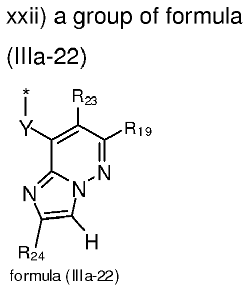 wherein
R₁₉, R₂₂, R₂₃, R₂₄, and R₂₅ each independently represent a hydrogen atom, a C₁-C₄ alkoxy group, a C₁-C₄ haloalkyl group, a C₁-C₄ hydroxyalkyl group, a C₃-C₄ cycloalkyl group, a -(CH₂)₀₋₃CN group, a -C(O)-(CH₂)₁-₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group; wherein R' and R" each independently represent a hydrogen atom, a hydroxy group, a C₁-C₄ alkoxy group or a linear or branched C₁-C₄ alkyl group; and wherein Y is as defined in claim 1;
or in the case that Y represents a -NR'- group, R₃ together with the -NR'- group and the carbon atom to which both R₃ and the -NR'- group are bonded form a 4- to 7-membered, saturated N-containing heterocyclyl group, which heterocyclyl group is unsubstituted or substituted by one or more substituents selected from a halogen atom, a hydroxyl group, a cyano group, a -CHF₂ group or a -CF₃ group.

11. A compound according to any one of claims 1 or 3 to 7, wherein R₄ represents a group selected from 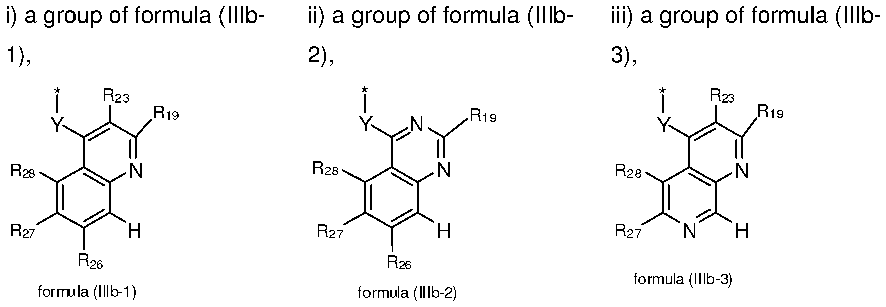 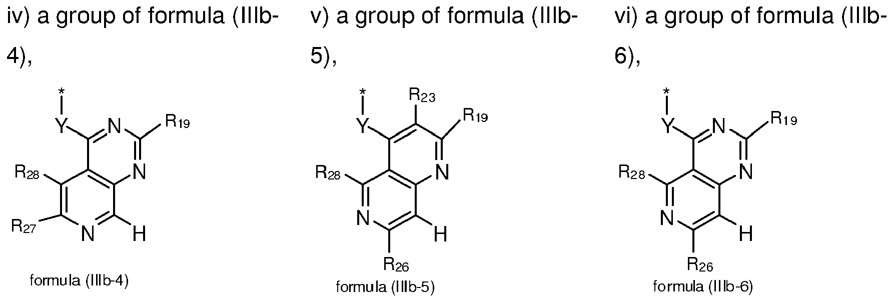 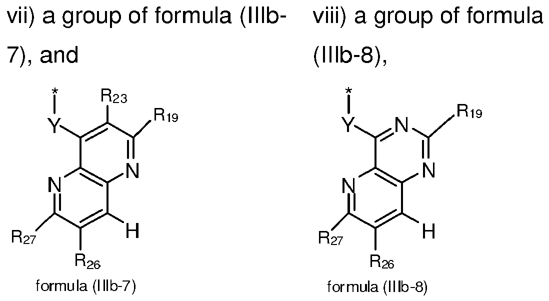 wherein
R₁₉, R₂₃, R₂₆, R₂₇, and R₂₈ each independently represent a hydrogen atom, a C₁-C₄ alkoxy group, a C₁-C₄ haloalkyl group, a C₁-C₄ hydroxyalkyl group, a C₃-C₄ cycloalkyl group, a -(CH₂)₀₋₃CN group, a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group; wherein R' and R" each independently represent a hydrogen atom, a hydroxy group, a C₁-C₄ alkoxy group or a linear or branched C₁-C₄ alkyl group; and wherein Y is as defined in claim 1;
or in the case that Y represents a -NR'- group, R₃ together with the -NR'- group and the carbon atom to which both R₃ and the -NR'- group are bonded form a 4- to 7-membered, saturated N-containing heterocyclyl group, which heterocyclyl group is unsubstituted or substituted by one or more substituents selected from a halogen atom, a hydroxyl group, a cyano group, a -CHF₂ group or a -CF₃ group.

12. A compound according to any one of claims 1 or 3 to 7, wherein R₄ represents a group selected from 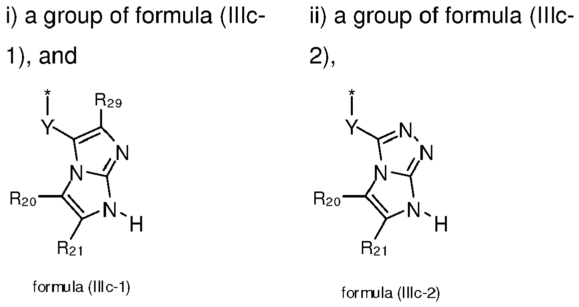 wherein
R₂₀, R₂₁, and R₂₉ each independently represent a hydrogen atom, a C₁-C₄ alkoxy group, a C₁-C₄ haloalkyl group, a C₁-C₄ hydroxyalkyl group, a C₃-C₄ cycloalkyl group, a -(CH₂)₀₋₃CN group, a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group; wherein R' and R" each independently represent a hydrogen atom, a hydroxy group, a C₁-C₄ alkoxy group or a linear or branched C₁-C₄ alkyl group; and wherein Y is as defined in claim 1;
or in the case that Y represents a -NR'- group, R₃ together with the -NR'- group and the carbon atom to which both R₃ and the -NR'- group are bonded form a 4- to 7-membered, saturated N-containing heterocyclyl group, which heterocyclyl group is unsubstituted or substituted by one or more substituents selected from a halogen atom, a hydroxyl group, a cyano group, a -CHF₂ group or a -CF₃ group.

13. A compound according to any one of claims 1 or 3 to 7, wherein R₄ represents a group selected from 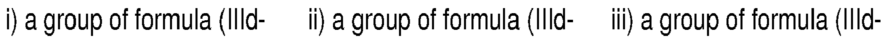 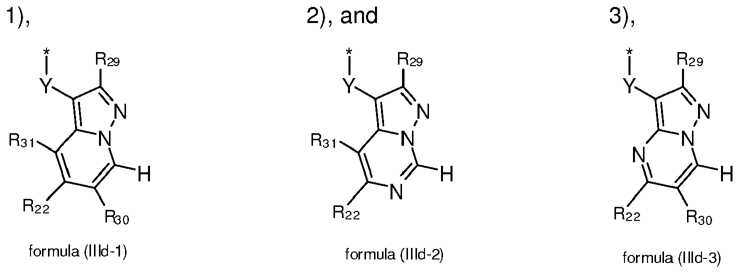 wherein
R₂₂, R₂₉, R₃₀, and R₃₁ each independently represent a hydrogen atom, a C₁-C₄ alkoxy group, a C₁-C₄ haloalkyl group, a C₁-C₄ hydroxyalkyl group, a C₃-C₄ cycloalkyl group, a -(CH₂)₀₋₃CN group, a -C(O)-(CH₂)₁₋₃-CN group, a -C(O)-(CH₂)₀₋₃-R' group, a -C(O)-(CH₂)₀₋₃-NR'R", a -(CH₂)₀₋₃NR'R" group, or a linear or branched C₁-C₄ alkyl group, which alkyl group is unsubstituted or substituted by one or more substituents selected from a C₁-C₄ alkoxy group, a cyano group or a C₃-C₄ cycloalkyl group; wherein R' and R" each independently represent a hydrogen atom, a hydroxy group, a C₁-C₄ alkoxy group or a linear or branched C₁-C₄ alkyl group; and wherein Y is as defined in claim 1;
or in the case that Y represents a -NR'- group, R₃ together with the -NR'- group and the carbon atom to which both R₃ and the -NR'- group are bonded form a 4- to 7-membered, saturated N-containing heterocyclyl group, which heterocyclyl group is unsubstituted or substituted by one or more substituents selected from a halogen atom, a hydroxyl group, a cyano group, a -CHF₂ group or a -CF₃ group.

14. A compound of according to claim 1, of fomula (Ia): 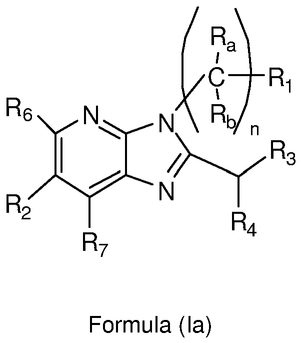 wherein R₁, R₂, R₃, R₄, R₆, R₇, Rₐ, R_{b} and n are as defined in in any one of the preceding claims.

15. A compound according to claim 1, of fomula (Ib): 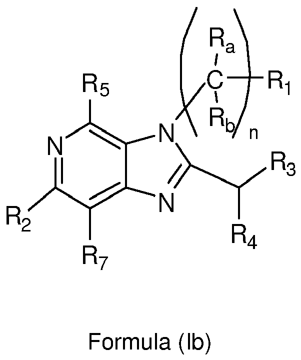 wherein R₁, R₂, R₃, R₄, R₅, R₇, Rₐ, R_{b} and n are as defined in any one of claims 1 to 13.

16. A compound according to claim 1, of fomula (Ic): 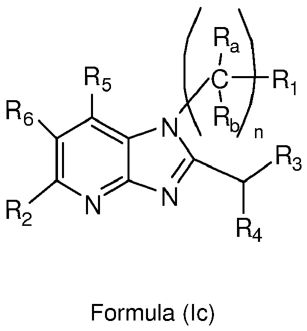 wherein R₁, R₂, R₃, R₄, R₅, R₆, Rₐ, R_{b} and nare as defined in any one of claims 1 to 13.

17. A compound according to claim 1, wherein:
X represents a nitrogen atom, W represents a -CR₆ group and Z represents a -CR₇ group; or X represents a -CR₅ group, W represents a -CR₆ group and Z represents nitrogen atom;
Rₐ and R_{b} each independently represent a hydrogen atom or a methyl group;
n represents 0 or 1;
R₁ represents a C₃-C₇ cycloalkyl group, a phenyl group, a pyridinyl group, a thiazolyl group, a piperidinyl group or a 2-pyridone group;
wherein the cycloalkyl, phenyl, pyridinyl, thiazolyl, piperidinyl and 2-pyridone groups are unsubstituted or substituted by one or more substituents selected from a halogen atom, a hydroxy group, a C₁-C₃ haloalkyl group, a linear or branched C₁-C₃ alkyl group, a -NR₈R₉ group or a -OR₉ group; wherein R₈ and R₉ each independently represent a hydrogen atom or a linear or branched C₁-C₃ alkyl group;
R₂ represents a hydrogen atom, a halogen atom, a -NR'R" group or a linear or branched C₁-C₃ alkyl group;
R₃ represents a hydrogen atom or a linear or branched C₁-C₃ alkyl group;
R₅, R₆ and R₇ each independently represent a hydrogen atom, a hydroxy group, a C₁-C₃ alkoxy group, a -(CH₂)₀₋₃NR'R" group, or a linear or branched C₁-C₃ alkyl group;
R₄ represents a group selected from:
i) a group of formula (IIa), which group is a purinyl group unsubstituted or substituted by a -NR'R" group;
ii) a group of formula (IIb), which group is selected from a -NH-pyrimidinyl group, a -N(CH₃)-pyrimidinyl group or a -S-pyrimidinyl group; wherein the pyrimidinyl group is unsubstituted or substituted by one, two or three substituents selected from a -(CH₂)₀₋₃CN group, a -C(O)-(CH₂)₀₋₃-NR'R" group or a -(CH₂)₀₋₃NR'R" group;
iii) a group of formula (IIc), which group is selected from a -NH-purinyl group, a -S-purinyl group, a -N(CH₃)-purinyl group, or
a -NH-pyrazolo[1,5-a]pyrimidinyl group; wherein the purinyl and pyrazolo[1,5-a]pyrimidinyl groups are unsubstituted or substituted by a -(CH₂)₀₋₃NR'R" group; or
R₃ and R₄ together with the carbon atom to which they are attached form (a) a pyrrolidinyl-pyrimidinyl group in which the pyrimidinyl group is unsubstituted or substituted by one, two or three substituents selected from a -(CH₂)₀₋₃CN group, a -C(O)-(CH₂)₀₋₃-NR'R" group or a -(CH₂)₀₋₃NR'R" group, or (b) a pyrrolidinyl-purinyl group wherein the purinyl group is unsubstituted or substituted by a -(CH₂)₀₋₃NR'R" group;
R' and R" each independently represent a hydrogen atom, a C₁-C₃ alkoxy group or a linear or branched C₁-C₃ alkyl group.

18. A compound according to claim 1, which is one of:
(S)-N-(1-(3-Phenyl-3H-imidazo[4,5-b]pyridin-2-yl)ethyl)-9H-purin-6-amine;
9-((3-Phenyl-3H-imidazo[4,5-b]pyridin-2-yl)methyl)-9H-purin-6-amine;
9-(1-(3-Phenyl-3H-imidazo[4,5-b]pyridin-2-yl)ethyl)-9H-purin-6-amine;
6-(1-(3-Phenyl-3H-imidazo[4,5-b]pyridin-2-yl)ethylthio)-9H-purine;
(S)-N-(1-(3-Phenyl-3H-imidazo[4,5-b]pyridin-2-yl)propyl)-9H-purin-6-amine;
(S)-N4-(1-(3-Phenyl-3H-imidazo[4,5-b]pyridin-2-yl)ethyl)pyrimidine-4,6-diamine;
(S)-6-(2-(3-Phenyl-3H-imidazo[4,5-b]pyridin-2-yl)pyrrolidin-1-yl)-9H-purine;
(S)-6-(2-(3-Phenyl-3H-imidazo[4,5-b]pyridin-2-yl)pyrrolidin-1-yl)pyrimidin-4-amine;
(S)-N6-(1-(3-Phenyl-3H-imidazo[4,5-b]pyridin-2-yl)ethyl)-9H-purine-2,6-diamine;
(S)-N-(1-(5-Methoxy-3-phenyl-3H-imidazo[4,5-b]pyridin-2-yl)ethyl)-9H-purin-6-amine;
(S)-N-(1-(3-(3,5-Difluorophenyl)-3H-imidazo[4,5-b]pyridin-2-yl)propyl)-9H-purin-6-amine;
(S)-N-(1-(3-(3,5-Difluorophenyl)-3H-imidazo[4,5-b]pyridin-2-yl)ethyl)-9H-purin-6-amine;
(S)-N4-(1-(3-Phenyl-3H-imidazo[4,5-b]pyridin-2-yl)ethyl)pyrimidine-2,4,6-triamine;
(S)-N-(1-(5-Methoxy-3-phenyl-3H-imidazo[4,5-b]pyridin-2-yl)propyl)-9H-purin-6-amine;
(S)-N-(1-(3-(3,5-Difluorophenyl)-5-methoxy-3H-imidazo[4,5-b]pyridin-2-yl)ethyl)-9H-purin-6-amine;
(S)-N-(1-(3-(3,5-Difluorophenyl)-5-methoxy-3H-imidazo[4,5-b]pyridin-2-yl)propyl)-9H-purin-6-amine;
9-((5-Methyl-3-o-tolyl-3H-imidazo[4,5-b]pyridin-2-yl)methyl)-9H-purin-6-amine;
9-((5-Methoxy-3-phenyl-3H-imidazo[4,5-b]pyridin-2-yl)methyl)-9H-purin-6-amine;
(S)-4-Amino-6-(1-(3-phenyl-3H-imidazo[4,5-b]pyridin-2-yl)ethylamino)pyrimidine-5-carbonitrile;
(S)-N6-(1-(5-Methoxy-3-phenyl-3H-imidazo[4,5-b]pyridin-2-yl)ethyl)-9H-purine-2,6-diamine;
(S)-4-Amino-6-(1-(5-methoxy-3-phenyl-3H-imidazo[4,5-b]pyridin-2-yl)ethylamino)pyrimidine-5-carbonitrile;
(S)-N-(1-(5-Methoxy-3-phenyl-3H-imidazo[4,5-b]pyridin-2-yl)ethyl)pyrazolo[1,5-a]pyrimidin-7-amine;
2-(1-(9H-Purin-6-ylamino)ethyl)-3-phenyl-3H-imidazo[4,5-b]pyridin-5-ol;
(S)-N-(1-(5-Methyl-3-phenyl-3H-imidazo[4,5-b]pyridin-2-yl)ethyl)-9H-purin-6-amine;
(S)-4-Amino-6-(1-(5-methyl-3-phenyl-3H-imidazo[4,5-b]pyridin-2-yl)ethylamino)pyrimidine-5-carbonitrile;
(S)-N-(1-(5-Methyl-3-phenyl-3H-imidazo[4,5-b]pyridin-2-yl)ethyl)pyrazolo[1,5-a]pyrimidin-7-amine;
9-{[3-(2-Methylphenyl)-3H-imidazo[4,5-b]pyridin-2-yl]methyl}-9H-purin-6-amine;
6-{[(3-Phenyl-3H-imidazo[4,5-b]pyridin-2-yl)methyl]thio}-9H-purine;
6-({[3-(2-Methylphenyl)-3H-imidazo[4,5-b]pyridin-2-yl]methyl}thio)-9H-purine;
9-((3-(Piperidin-4-yl)-3H-imidazo[4,5-b]pyridin-2-yl)methyl)-9H-purin-6-amine;
9-((3-(1-Methylpiperidin-4-yl)-3H-imidazo[4,5-b]pyridin-2-yl)methyl)-9H-purin-6-amine;
9-((6-Amino-3-phenyl-3H-imidazo[4,5-b]pyridin-2-yl)methyl)-9H-purin-6-amine;
9-((6-Methyl-3-phenyl-3H-imidazo[4,5-b]pyridin-2-yl)methyl)-9H-purin-6-amine;
9-((5-Amino-3-phenyl-3H-imidazo[4,5-b]pyridin-2-yl)methyl)-9H-purin-6-amine;
7-((5-Amino-3-phenyl-3H-imidazo[4,5-b]pyridin-2-yl)methyl)-7H-purin-6-amine;
2-((9H-Purin-6-ylthio)methyl)-3-phenyl-3H-imidazo[4,5-b]pyridin-5-amine;
N-[(1S)-1-(6-Fluoro-3-phenyl-3H-imidazo[4,5-b]pyridin-2-yl)ethyl]-9H-purin-6-amine;
N-[(1S)-1-(6-Fluoro-3-phenyl-3H-imidazo[4,5-b]pyridin-2-yl)propyl]-9H-purin-6-amine;
N-{(1S)-1-[3-(2-Chlorophenyl)-3H-imidazo[4,5-b]pyridin-2-yl]ethyl}-9H-purin-6-amine;
N-1[3-(2-Chlorophenyl)-3H-imidazo[4,5-b]pyridin-2-yl]methyll-9H-purin-6-amine;
9-((1-Phenyl-1H-imidazo[4,5-b]pyridin-2-yl)methyl)-9H-purin-6-amine;
6-((1-Phenyl-1H-imidazo[4,5-b]pyridin-2-yl)methylthio)-9H-purine;
(S)-N-(1-(1-Phenyl-1H-imidazo[4,5-b]pyridin-2-yl)ethyl)-9H-purin-6-amine;
(S)-N-(1-(3-(2,6-Difluorophenyl)-3H-imidazo[4,5-b]pyridin-2-yl)ethyl)-9H-purin-6-amine;
N-((1S)-1-(3-(2-Methoxyphenyl)-3H-imidazo[4,5-b]pyridin-2-yl)ethyl)-9H-purin-6-amine;
(S)-N-(1-(1-Phenyl-1H-imidazo[4,5-b]pyridin-2-yl)propyl)-9H-purin-6-amine;
N-{(1S)-1-[3-(3-chlorophenyl)-3H-imidazo[4,5-b]pyridin-2-yl]ethyl}-9H-purin-6-amine;
N-{(1S)-1-[3-(3-methoxyphenyl)-3H-imidazo[4,5-b]pyridin-2-yl]ethyl}-9H-purin-6-amine;
N-[(1S)-1-(3-pyridin-2-yl-3H-imidazo[4,5-b]pyridin-2-yl)ethyl]-9H-purin-6-amine;
N-[(1S)-1-(3-pyridin-3-yl-3H-imidazo[4,5-b]pyridin-2-yl)ethyl]-9H-purin-6-amine;
N-[(1S)-1-(3-cyclohexyl-3H-imidazo[4,5-b]pyridin-2-yl)ethyl]-9H-purin-6-amine;
N-[(1S)-1-(3-benzyl-3H-imidazo[4,5-b]pyridin-2-yl)ethyl]-9H-purin-6-amine;
N-{(1S)-1-[3-(3-fluorophenyl)-3H-imidazo[4,5-b]pyridin-2-yl]ethyl}-9H-purin-6-amine;
N-((1S)-1-{3-[3-(trifluoromethyl)phenyl]-3H-imidazo[4,5-b]pyridin-2-yl}ethyl)-9H-purin-6-amine;
N-{(1S)-1-[3-(3-methylphenyl)-3H-imidazo[4,5-b]pyridin-2-yl]ethyl}-9H-purin-6-amine;
4-{2-[(1S)-1-(9H-purin-6-ylamino)ethyl]-3H-imidazo[4,5-b]pyridin-3-yl}pyridin-2(1H)-one;
N-{(1S)-1-[3-(1,3-thiazol-4-yl)-3H-imidazo[4,5-b]pyridin-2-yl]ethyl}-9H-purin-6-amine;
4-amino-6-({(1S)-1-[3-(3-chlorophenyl)-3H-imidazo[4,5-b]pyridin-2-yl]ethyl}amino)pyrimidine-5-carbonitrile;
4-amino-6-({(1S)-1-[3-(3-methoxyphenyl)-3H-imidazo[4,5-b]pyridin-2-yl]ethyl}amino)pyrimidine-5-carbonitrile;
4-amino-6-{[(1S)-1-(3-pyridin-2-yl-3H-imidazo[4,5-b]pyridin-2-yl)ethyl]amino]pyrimidine-5-carbonitrile;
4-amino-6-{[(1S)-1-(3-pyridin-3-yl-3H-imidazo[4,5-b]pyridin-2-yl)ethyl]amino]pyrimidine-5-carbonitrile;
4-amino-6-{[(1S)-1-(3-cyclohexyl-3H-imidazo[4,5-b]pyridin-2-yl)ethyl]amino}pyrimidine-5-carbonitrile;
4-amino-6-{[(1S)-1-(3-benzyl-3H-imidazo[4,5-b]pyridin-2-yl)ethyl]amino]pyrimidine-5-carbonitrile;
4-amino-6-({(1S)-1-[3-(3-fluorophenyl)-3H-imidazo[4,5-b]pyridin-2-yl]ethyl}amino)pyrimidine-5-carbonitrile;
4-amino-6-[((1S)-1-{3-[3-(trifluoromethyl)phenyl]-3H-imidazo[4,5-b]pyridin-2-yl}ethyl)amino]pyrimidine-5-carbonitrile;
4-amino-6-({(1S)-1-[3-(3-methylphenyl)-3H-imidazo[4,5-b]pyridin-2-yl]ethyl}amino)pyrimidine-5-carbonitrile;
4-amino-6-({(1S)-1-[3-(2-oxo-1,2-dihydropyridin-4-yl)-3H-imidazo[4,5-b]pyridin-2-yl]ethyl}amino)pyrimidine-5-carbonitrile;
4-amino-6-({(1S)-1-[3-(1,3-thiazol-4-yl)-3H-imidazo[4,5-b]pyridin-2-yl]ethyl}amino)pyrimidine-5-carbonitrile;
N-{(1S)-1-[5-(methylamino)-3-phenyl-3H-imidazo[4,5-b]pyridin-2-yl]ethyl}-9H-purin-6-amine;
4-amino-6-({(1S)-1-[5-(methylamino)-3-phenyl-3H-imidazo[4,5-b]pyridin-2-yl]ethyl}amino)pyrimidine-5-carbonitrile;
N-[(1S)-1-(5-ethoxy-3-phenyl-3H-imidazo[4,5-b]pyridin-2-yl)ethyl]-9H-purin-6-amine;
4-amino-6-{[(1S)-1-(5-ethoxy-3-phenyl-3H-imidazo[4,5-b]pyridin-2-yl)ethyl]amino}pyrimidine-5-carbonitrile;
N-[(1S)-1-(6-fluoro-5-methoxy-3-phenyl-3H-imidazo[4,5-b]pyridin-2-yl)ethyl]-9H-purin-6-amine;
4-amino-6-{[(1S)-1-(6-fluoro-5-methoxy-3-phenyl-3H-imidazo[4,5-b]pyridin-2-yl)ethyl]amino}pyrimidine-5-carbonitrile;
N-[(1S)-1-(5-methoxy-3-phenyl-3H-imidazo[4,5-b]pyridin-2-yl)ethyl]-N-methyl-9H-purin-6-amine;
4-amino-6-[[(1S)-1-(5-methoxy-3-phenyl-3H-imidazo[4,5-b]pyridin-2-yl)ethyl](methyl)amino]pyrimidine-5-carbonitrile;
N-[(1S)-1-(7-methyl-3-phenyl-3H-imidazo[4,5-b]pyridin-2-yl)ethyl]-9H-purin-6-amine;
4-amino-6-{[(1S)-1-(7-methyl-3-phenyl-3H-imidazo[4,5-b]pyridin-2-yl)ethyl]amino}pyrimidine-5-carbonitrile;
or a pharmaceutically acceptable salt, or solvate, or N-oxide, or stereoisomer or deuterated derivative thereof.

19. A compound according to any one of claims 1 to 18, for use in the treatment of a pathological condition or disease susceptible to amelioration by inhibition of Phosphoinositide 3-Kinase (PI3K).

20. A compound according to claim 19, wherein the pathological condition or disease is selected from respiratory diseases; allergic diseases; inflammatory or autoimmune-mediated; function disorders and neurological disorders; cardiovascular diseases; viral infection; metabolism/endocrine function disorders; neurological disorders and pain; bone marrow and organ transplant rejection; myelo-dysplastic syndrome; myeloproliferative disorders (MPDs); cancer and hematologic malignancies, leukemia, lymphomas and solid tumors.

21. A compound according to claims 19 or 20, wherein the pathological condition or disease is selected from leukemia, lymphomas and solid tumors, rheumatoid arthritis, multiple sclerosis, amyotrophic lateral sclerosis, Crohn's disease, ulcerative colitis, systemic lupus erythematosis, autoimmune hemolytic anemia, type I diabetes, asthma, chronic obstructive pulmonary disease, cystic fibrosis, idiopathic pulmonary fibrosis, sarcoidosis, allergic rhinitis, atopic dermatitis, contact dermatitis, eczema, psoriasis, basal cell carcinoma, squamous cell carcinoma and actinic keratosis.

22. A pharmaceutical composition comprising a compound as defined in any one of claims 1 to 17 in association with a pharmaceutically acceptable diluent or carrier.

23. Use of a compound as defined in any one of claims 1 to 18, for the manufacture of a medicament for the treatment of a pathological condition or disease as defined in any one of claims 19 to 21.

24. A method for treating a subject afflicted with a pathological condition or disease as defined in any one of claims 19 to 21, which comprises administering to said subject a therapeutically effective amount of a compound as defined in any one of claims 1 to 17, or a pharmaceutical composition as defined in claim 22.

25. A combination product comprising (i) a compound as defined in any one of claims 1 to 18; and (ii) another compound selected from the group consisting of an Adenoside A_{2A} agonist, an agent for treating cardiovascular disorders, an agent for treating diabetes, and an agent for treating liver disease, an anti-allergic agent, an anti-cholinergic agent, an anti-inflammatory agent, an anti-infective agent, a β2-adrenergic agonist, a Chemoattractant receptor homologous molecule expressed on TH₂ cells (CRTH2) inhibitor, a chemotherapeutic agent, a corticosteroid, an IKKβ/IKBKB (IkB kinase beta or IKK2) inhibitor, an immunosuppressant, a Janus kinase (JAK) inhibitor, a topically acting p38 Mitogen-Activated Protein Kinase (p38 MAPK) inhibitor, a Phosphosdiesterase (PDE) IV inhibitor, and a Spleen tyrosine kinase (Syk) inhibitor, for simultaneous, separate or sequential use in the treatment of the human or animal body.
